# EUROPEAN PATENT SPECIFICATION

(11) **EP 4 486 913 B1**
(45) Date of publication and mention of the grant of the patent: **16.07.2025**
(21) Application number: 23848754.0
(22) Date of filing: 28.12.2023
(51) Int. Cl.: C12Q 1/6806, C12Q 1/6823, C12Q 1/6841

(54) **METHODS, COMPOSITIONS, AND KITS FOR MULTIPLE BARCODING AND/OR HIGH-DENSITY SPATIAL BARCODING**
VERFAHREN, ZUSAMMENSETZUNGEN UND KITS ZUR MEHRFACH-BARCODIERUNG UND/ODER HOCHDICHTEN RÄUMLICHEN BARCODIERUNG
PROCÉDÉS, COMPOSITIONS ET KITS POUR LE CODAGE À BARRES MULTIPLE ET/OU LE CODAGE À BARRES SPATIAL À HAUTE DENSITÉ

(30) Priority: 30.12.2022 US 202263436324 P; 22.03.2023 US 202363453876 P
(43) Date of publication of application: 08.01.2025
(60) Divisional application: 25182060.1
(73) Proprietor: 10x Genomics, Inc., Pleasanton, CA 94588-3260 (US)
(72) Inventor: KIM, Hanyoup, Pleasanton, CA 94588-3260 (US); MIKHAIEL, Nabil, Pleasanton, CA 94588-3260 (US); TENTORI, Augusto, Manuel, Pleasanton, CA 94588-3260 (US); PATTERSON, David, Michael, Pleasanton, CA 94588-3260 (US); YIN, Yifeng, Pleasanton, CA 94588-3260 (US); TUNCER, Sultan, Doganay, Pleasanton, CA 94588-3260 (US); FREY, Meghan L., F., Pleasanton, CA 94588-3260 (US); LANCE, Shea, Thompson, Pleasanton, CA 94588-3260 (US)
(74) Representative: Vossius & Partner Patentanwälte Rechtsanwälte mbB
(86) International application number: PCT/US2023/086255
(87) International publication number: WO 2024/145491

(56) References cited:
- WO-A1-2020/123309
- WO-A1-2021/133849

## Description

### TECHNICAL FIELD OF THE INVENTION

The present invention relates to methods for multiple barcoding and/or high-density barcoding of target nucleic acids in a biological sample and/or to determine the location of the target nucleic acid in the biological sample..

### BACKGROUND

Cells within a tissue of a subject have differences in cell morphology and/or function due to varied analyte levels (e.g., gene and/or protein expression) within the different cells. The specific position of a cell within a tissue (e.g., the cell's position relative to neighboring cells or the cell's position relative to the tissue microenvironment) can affect, e.g., the cell's morphology, differentiation, fate, viability, proliferation, behavior, signaling and cross-talk with other cells in the tissue.

Spatial heterogeneity has been previously studied using techniques that provide data for a handful of analytes in the context of an intact tissue or a portion of a tissue, or provides substantial analyte data for dissociated tissue (i.e., single cells), but fails to provide information regarding the position of a single cell in a parent biological sample (e.g., tissue sample).

Spatial analysis of analytes within a biological sample requires determining the sequence of the analyte sequence or a complement thereof and the sequence of the spatial barcode or a complement thereof to identify the location of the analyte in the biological sample. The biological sample can be placed on a substrate to improve specificity and efficiency when being analyzed for identification or characterization of an analyte, such as DNA, RNA, or other genetic material, within the biological sample. Described herein are methods utilizing more than one spatial barcode (e.g., high-density barcoding) to identify the location of analytes in a biological sample.

### SUMMARY

The invention is set out in the appended set of claims. The technical disclosure set out below may in some respects go beyond the scope of the claims. Elements of the disclosure which do not fall within the scope of the claims are provided for information.

The present disclosure features methods, compositions, devices, and systems for determining the location and/or abundance of an analyte in a biological sample. Determining the spatial location and/or abundance of analytes (e.g., protein, DNA, or RNA) within a biological sample leads to better understanding of spatial heterogeneity in various contexts, such as disease models. Described herein are methods for capturing probes and/or barcodes to a capture domain of a capture probe in an array. In some instances, the techniques disclosed herein facilitate downstream processing, such as sequencing of the probes and/or barcodes bound to a capture domain.

Thus provided herein are methods of spatially barcoding a target nucleic acid in a biological sample, the method including: (a) providing an array including a first plurality of capture probes, where a first capture probe of the first plurality of capture probes includes: (i) a first spatial barcode and (ii) a first capture domain, and where the first capture probe is attached to a substrate by its 3' end, and a second plurality of capture probes, where a second capture probe of the second plurality of capture probes includes: (iii) a second spatial barcode and (iv) a second capture domain, and where the second capture probe is attached to the substrate by its 3' end; (b) hybridizing a first probe oligonucleotide and a second probe oligonucleotide to the target nucleic acid, where the first probe oligonucleotide and the second probe oligonucleotide each include a sequence that is substantially complementary to a first sequence and a second sequence of the target nucleic acid, respectively, and where the first probe oligonucleotide includes a first capture probe binding domain and the second probe oligonucleotide includes a second capture probe binding domain; (c) coupling the first probe oligonucleotide and the second probe oligonucleotide, thereby generating a ligation product; (d) releasing the first capture probe and/or the second capture probe from the substrate; and (e) hybridizing (i) the first capture domain of the first capture probe to the first capture probe binding domain of the ligation product, and optionally, (ii) the second capture domain of the second capture probe to the second capture probe binding domain of the ligation product, thereby spatially barcoding the target nucleic acid in the biological sample.

In some embodiments, the first probe oligonucleotide and the second probe oligonucleotide hybridize to adjacent sequences on the target nucleic acid. In some embodiments, the first probe oligonucleotide and the second probe oligonucleotide hybridize to non-adjacent sequences on the target nucleic acid. In some embodiments, a gap-filling reaction extends the first probe oligonucleotide or second probe oligonucleotide.

In some embodiments, the coupling of the first probe oligonucleotide and the second probe oligonucleotide includes ligating the first probe oligonucleotide and the second probe oligonucleotide via a ligase.

In some embodiments, the method includes releasing the ligation product from the target nucleic acid, e.g., by using a nuclease. In some embodiments, the nuclease includes an RNase, optionally, where the RNase is selected from RNase A, RNase C, RNase H, or RNase I.

In some embodiments, the first capture probe binding domain includes a first sequence complementary to at least a portion of the first capture domain of the first capture probe, and the second capture probe binding domain includes a second sequence complementary to at least a portion of the second capture domain of the second capture probe. In some embodiments, the first sequence complementary to at least a portion of the first capture domain of the first capture probe includes a poly(A) sequence. In some embodiments, the second sequence complementary to at least a portion of the second capture domain of the second capture probe includes a fixed sequence.

In some embodiments, the first capture probe hybridized to the first capture probe binding domain of the ligation product is extended with a polymerase, thereby generating a complement of the ligation product. In some embodiments, the complement of the ligation product is ligated to the second capture probe. In some embodiments, the ligation includes use of a ligase.

In some embodiments, the method includes determining (i) all or a part of the sequence of the ligation product, or a complement thereof, and (ii) the sequence of the first spatial barcode, or a complement thereof and, optionally, the sequence of the second spatial barcode, or a complement thereof. In some embodiments, the determining includes sequencing. In some embodiments, the sequences of (i) and (ii) are determined by sequencing the ligation product and/or the complement of the ligation product.

In some embodiments, the method includes using the combination of the determined sequence of the first spatial barcode, or a complement thereof, and, optionally, the determined sequence of the second spatial barcode, or a complement thereof, to determine a location of the target nucleic acid in the biological sample.

In some embodiments, the first capture probe, and optionally, the second capture probe include a quality control (QC) sequence.

In some embodiments, releasing the first capture probe and/or the second capture probe includes cleaving the first capture probe, and optionally, the second capture probe from the substrate.

In some embodiments, the first capture probe includes a first cleavable linker and the second capture probe includes a second cleavable linker. In some embodiments, the first cleavable linker and the second cleavable linker include a restriction enzyme site. In some embodiments, the first cleavable linker and the second cleavable linker include a photocleavable linker. In some embodiments, the first cleavable linker and the second cleavable linker are different. In some embodiments, the first cleavable linker and the second cleavable linker are the same. In some embodiments, the first cleavable linker and the second cleavable linker are cleaved at the same time.

In some embodiments, the first cleavable linker is cleaved prior to the second cleavable linker being cleaved, and where the first capture domain hybridizes to the first capture probe binding domain of the ligation product at a first time point and the second capture domain hybridizes to the second capture probe binding domain of the ligation product at a second time point.

In some embodiments, the method includes i) cleaving the first cleavable linker; ii) permeabilizing the biological sample; iii) hybridizing the first capture domain of the first capture probe to the first capture probe binding domain of the ligation product, thereby generating a first capture probe/ligation product complex; and iv) hybridizing the first capture probe/ligation product complex to the second capture probe on the array via the second capture probe binding domain.

In some embodiments, the target nucleic acid is DNA. In some embodiments, the target nucleic acid is RNA. In some embodiments, the RNA is mRNA.

In some embodiments, the array includes a plurality of wells, where a well of the plurality of wells includes the first capture probe and the second capture probe.

In some embodiments, the biological sample is disposed on the array. In some embodiments, the biological sample is disposed on a second substrate. In some embodiments, the method includes aligning the second substrate with the array e.g., on the first substrate, such that at least a portion of the biological sample is aligned with at least a portion of the array.

In some embodiments, the first capture domain is 5' to the first spatial barcode and the second capture domain is 5' to the second spatial barcode.

Also provided herein are methods for spatially barcoding a target nucleic acid in a biological sample, the method including: (a) providing an array including a first plurality of capture probes, where a first capture probe of the first plurality of capture probes includes: (i) a first capture domain, (ii) a first spatial barcode, and (iii) a ligation domain substantially complementary to a portion of a first splint oligonucleotide of a plurality of first splint oligonucleotides, and optionally, a second plurality of capture probes, where a second capture probe of the second plurality of capture probes includes: (iv) a second ligation domain substantially complementary to a portion of the first splint oligonucleotide, (v) a second spatial barcode, and (vi) a third ligation domain substantially complementary to a portion of a second splint oligonucleotide of a plurality of second splint oligonucleotides; (b) hybridizing a first probe oligonucleotide and a second probe oligonucleotide to the target nucleic acid, where the first probe oligonucleotide and the second probe oligonucleotide each include a sequence that is substantially complementary to a first sequence and a second sequence of the target nucleic acid, respectively, and where the first probe oligonucleotide includes a first capture probe binding domain; (c) coupling the first probe oligonucleotide and the second probe oligonucleotide, thereby generating a ligation product; (d) releasing the first capture probe, and optionally, the second capture probe from the array; and (e) hybridizing the first capture domain of the first capture probe to the first capture probe binding domain of the ligation product, thereby spatially barcoding the target nucleic acid.

In some embodiments, the method includes contacting the biological sample with the first plurality of splint oligonucleotides, where the first splint oligonucleotide includes: (i) a sequence complementary to the ligation domain and (ii) a sequence complementary to the second ligation domain.

In some embodiments, the method includes hybridizing the first splint oligonucleotide to (i) the ligation domain and (ii) the second ligation domain.

In some embodiments, the method includes releasing a third capture probe of a plurality of third capture probes from the array, where the third capture probe of the plurality of third capture probes includes: (i) a fourth ligation domain substantially complementary to a portion of the second splint oligonucleotide, (ii) a third spatial barcode, and (iii) a fifth ligation domain substantially complementary to a portion of a third splint oligonucleotide of a third plurality of splint oligonucleotides.

In some embodiments, the method includes contacting the biological sample with the second plurality of splint oligonucleotides, where the second splint oligonucleotide includes: (i) a sequence substantially complementary to the third ligation domain and (ii) a sequence substantially complementary to the fourth ligation domain.

In some embodiments, the method includes hybridizing the second splint oligonucleotide to (i) the third ligation domain and (ii) the fourth ligation domain.

In some embodiments, the method includes releasing a fourth capture probe of a plurality of fourth capture probes from the array, where the fourth capture probe of the plurality of fourth capture probes includes: (i) a sixth ligation domain substantially complementary to a portion of the third splint oligonucleotide, (ii) a fourth spatial barcode, and (iii) a ligation domain substantially complementary to a portion of a fourth splint oligonucleotide of a fourth plurality of splint oligonucleotides.

In some embodiments, the method includes contacting the biological sample with the third plurality of splint oligonucleotides, where the third splint oligonucleotide includes: (i) a sequence complementary to the fifth ligation domain and (ii) a sequence complementary to the sixth ligation domain.

In some embodiments, the first plurality of capture probes, and optionally, the second plurality of capture probes, the third plurality of capture probes, and the fourth plurality of capture probes are released simultaneously. In some embodiments, the first plurality of capture probes, and optionally, the second plurality of capture probes, the third plurality of capture probes, and the fourth plurality of capture probes are released sequentially.

In some embodiments, the first plurality of splint oligonucleotides, the second plurality of splint oligonucleotides, and the third plurality of splint oligonucleotides are contacted with the biological sample simultaneously. In some embodiments, the first plurality of splint oligonucleotides, the second plurality of splint oligonucleotides, and the third plurality of splint oligonucleotides are contacted with the biological sample sequentially.

In some embodiments, the first plurality of capture probes, the second plurality of capture probes, the third plurality of capture probes, and the fourth plurality of capture probes are released via denaturation.

In some embodiments, the first plurality of capture probes, the second plurality of capture probes, the third plurality of capture probes, and the fourth plurality of capture probes include a cleavage domain. In some embodiments, the cleavage domain is a photocleavable cleavage domain. In some embodiments, the cleavage domain is a restriction enzyme site.

In some embodiments, the cleavage domain for the first plurality of capture probes, the second plurality of capture probes, the third plurality of capture probes, and the fourth plurality of capture probes is the same. In some embodiments, the cleavage domain for the first plurality of capture probes, the second plurality of capture probes, the third plurality of capture probes, and the fourth plurality of capture probes is different for each plurality.

In some embodiments, the first plurality of splint oligonucleotides, the second plurality of splint oligonucleotides, and the third plurality of splint oligonucleotides include the same sequence. In some embodiments, the first plurality of splint oligonucleotides, the second plurality of splint oligonucleotides, and the third plurality of splint oligonucleotides each include a different sequence.

In some embodiments, the first probe oligonucleotide and the second probe oligonucleotide hybridize to adjacent sequence on the target nucleic acid. In some embodiments, the first probe oligonucleotide and the second probe oligonucleotide hybridize to non-adjacent sequences on the target nucleic acid. In some embodiments, a gap-filling reaction extends the first probe or the second probe.

In some embodiments, the coupling of the first probe oligonucleotide and the second probe oligonucleotide includes ligating the first probe oligonucleotide and the second probe oligonucleotide via a ligase, and where the first capture probe is ligated to the second capture probe, the second capture probe is ligated to the third capture probe, and the third capture probe is ligated to the fourth capture probe via a ligase, and where the first capture probe is extended, thereby generating a complement of the ligation product.

In some embodiments, the method includes releasing the target nucleic acid from the ligation product using a nuclease. In some embodiments, the nuclease includes an RNase, optionally where the RNase is selected from RNase A, RNase C, RNase H, or RNase I.

In some embodiments, the first capture probe binding domain includes a poly(A) sequence.

In some embodiments, the target nucleic acid is DNA. In some embodiments, the target nucleic acid is RNA. In some embodiments, the RNA is mRNA.

In some embodiments, the array includes a plurality of wells, where a well of the plurality of wells includes the first capture probe, and optionally, the second capture probe, the third capture probe, and/or the fourth capture probe.

In some embodiments, the biological sample is disposed on the array. In some embodiments, the biological sample is disposed on a substrate. In some embodiments, the method includes aligning the substrate with the array, such that at least a portion of the biological sample is aligned with at least a portion of the array.

In some embodiments, the method includes determining (i) all or a part of the sequence of the ligation product, or a complement thereof, and (ii) the sequences of the first spatial barcode and the second spatial barcode of the first capture probe and the second capture probe, respectively, or a complement thereof. In some embodiments, the determining includes sequencing.

Also provided herein are methods of determining a location of a target nucleic acid in a biological sample, the method including: (a) providing an array including a first plurality of capture probes, where a first capture probe of the first plurality of capture probes includes: (i) a first spatial barcode and (ii) a first capture domain, and where the first capture probe is attached to a substrate by its 3' end, and a second plurality of capture probes, where a second capture probe of the second plurality of capture probes includes: (iii) a second spatial barcode and (iv) a second capture domain, and where the second capture probe is attached to the substrate by its 3' end; (b) hybridizing a first probe oligonucleotide and a second probe oligonucleotide to the target nucleic acid, where the first probe oligonucleotide and the second probe oligonucleotide each include a sequence that is substantially complementary to a first sequence and a second sequence of the target nucleic acid, respectively, and where the first probe oligonucleotide includes a first capture probe binding domain and the second probe oligonucleotide includes a second capture probe binding domain; (c) coupling the first probe oligonucleotide and the second probe oligonucleotide, thereby generating a ligation product; (d) releasing the first capture probe and/or the second capture probe from the array; (e) hybridizing the first capture probe to the first capture probe binding domain of the ligation product and hybridizing the second capture probe to the second capture probe binding domain of the ligation product, thereby generating a spatially barcoded product; and (f) determining (i) all or a portion of the sequence of the ligation product of the spatially barcoded product, or a complement thereof, (ii) the sequence of the first spatial barcode, or a complement thereof, and optionally, (iii) the sequence of the second spatial barcode, or a complement thereof, and using the determined sequences of (i), (ii), and optionally, (iii) to determine the location of the target nucleic acid in the biological sample.

Also provided herein are methods of determining a location of a target nucleic acid in a biological sample, the method including: (a) providing an array including a first plurality of capture probes, where a first capture probe of the first plurality of capture probes includes: (i) a first capture domain, (ii) a first spatial barcode, and (iii) a ligation domain substantially complementary to a portion of a first splint oligonucleotide of a plurality of first splint oligonucleotides, and optionally, a second plurality of capture probes, where a second capture probe of the second plurality of capture probes includes: (i) a second ligation domain substantially complementary to a portion of the first splint oligonucleotide, (ii) a second spatial barcode, and (iii) a third ligation domain substantially complementary to a portion of a second splint oligonucleotide of a plurality of second splint oligonucleotides; (b) hybridizing a first probe oligonucleotide and a second probe oligonucleotide to the target nucleic acid, where the first probe oligonucleotide and the second probe oligonucleotide each include a sequence that is substantially complementary to a first sequence and a second sequence of the target nucleic acid, respectively, and where the first probe oligonucleotide includes a first capture probe binding domain; (c) coupling the first probe oligonucleotide and the second probe oligonucleotide, thereby generating a ligation product; (d) releasing the first capture probe, and optionally, the second capture probe from the array; (e) hybridizing the first capture domain of the first capture probe to the capture probe binding domain of the ligation product and extending the first capture probe, thereby generating a complement of the ligation product; (f) optionally, hybridizing a first splint oligonucleotide of a plurality of first splint oligonucleotides to the ligation domain of the first capture probe and to the second ligation domain of the second capture probe; and (g) determining (i) all or a portion of the sequence of the ligation product, or a complement thereof, and (ii) the sequence of the first spatial barcode, or a complement thereof, and using the determined sequences of (i) and (ii), to determine the location of the target nucleic acid in the biological sample.

**In** some embodiments, the method includes imaging the biological sample. **In** some embodiments, the imaging includes one or more of light field, bright field, dark field, phase contrast, fluorescence microscopy, reflection, interference, and confocal microscopy.

In some embodiments, the method includes staining the biological sample. **In** some embodiments, the staining includes hematoxylin and/or eosin staining. **In** some embodiments, the staining includes use of a detectable label selected from the group consisting of a radioisotope, a fluorophore, a chemiluminescent compound, a bioluminescent compound, or a combination thereof.

In some embodiments, the biological sample is a tissue sample. In some embodiments, the tissue sample is a fixed tissue sample or a fixed tissue section. In some embodiments, the fixed tissue section is a formalin-fixed paraffin-embedded (FFPE) tissue section, a paraformaldehyde-fixed tissue section, an acetone-fixed tissue section, a methanol-fixed tissue section, or an ethanol-fixed tissue section. In some embodiments, the biological sample is a fresh-frozen sample In some embodiments, the biological sample is a tissue section. In some embodiments, the tissue section is a fresh-frozen tissue section. In some embodiments, the FFPE tissue section is deparaffinized and decrosslinked prior to or after mounting on the substrate or second substrate.

In some embodiments, the method includes determining one or more regions of interest in the biological sample; and applying a mask to the substrate such that the mask does not align with the one or more regions of interest, where the mask is applied between the first and second plurality of capture probes and an activation source.

In some embodiments, determining one or more regions of interest and applying the mask occur prior to releasing the first capture probe and/or second capture probe from the substrate. In some embodiments, the method includes when the first and/or second capture probe are aligned with the one or more regions of interest, releasing the first and/or second capture probe from the substrate including applying an activation source, thereby cleaving the first and/or second capture probe aligned with the one or more regions of interest.

In some embodiments, the one or more regions of interest include at least one, two, three, four, five, six, seven, eight, nine, ten, or more regions of interest.

In some embodiments, the mask includes a composition that reduces or prevents light from permeating through the composition. In some embodiments, the activation source is a light source. In some embodiments, the mask reduces or prevents cleavage of a capture probe at one or more regions outside of, or not overlapping with, the one or more regions of interest. In some embodiments, the method includes removing the mask and analyzing a second analyte in the biological sample in a region outside of the one or more regions of interest.

Also provided herein are methods of spatially barcoding a target nucleic acid in a biological sample, the method including: (a) providing a substrate including an array including a first plurality of capture probes, where a first capture probe of the first plurality of capture probes includes: (i) a first spatial barcode and (ii) a first capture domain, and where the first capture probe is attached to the substrate by its 3' end, and a second plurality of capture probes, where a second capture probe of the second plurality of capture probes includes: (iii) a second spatial barcode and (iv) a second capture domain, and where the second capture probe is attached to the substrate by its 3' end; (b) hybridizing a first probe oligonucleotide and a second probe oligonucleotide to the target nucleic acid, where the first probe oligonucleotide and the second probe oligonucleotide each include a sequence that is substantially complementary to a first sequence and a second sequence of the target nucleic acid, respectively, and where the first probe oligonucleotide includes a first capture probe binding domain and the second probe oligonucleotide includes a second capture probe binding domain; (c) coupling the first probe oligonucleotide and the second probe oligonucleotide, thereby generating a ligation product; (d) determining a region of interest in the biological sample; (e) applying a mask to the substrate, such that the mask does not align with the region of interest; (f) releasing the first capture probe and/or the second capture probe from the substrate, where the first capture probe and/or the second capture probe are aligned with the region of interest before release; and (g) hybridizing (i) the first capture domain of the first capture probe to the first capture probe binding domain of the ligation product, and optionally, (ii) the second capture domain of the second capture probe to the second capture probe binding domain of the ligation product, thereby spatially barcoding the target nucleic acid in the biological sample.

Also provided herein are methods for spatially barcoding a target nucleic acid in a biological sample, the method including: a) providing an array including a first plurality of capture probes, where a first capture probe of the first plurality of capture probes includes: (i) a first capture domain, (ii) a first spatial barcode, and (iii) a ligation domain substantially complementary to a portion of a first splint oligonucleotide of a plurality of first splint oligonucleotides, and optionally, a second plurality of capture probes, where a second capture probe of the second plurality of capture probes includes: (iv) a second ligation domain substantially complementary to a portion of the first splint oligonucleotide, (v) a second spatial barcode, and (vi) a third ligation domain substantially complementary to a portion of a second splint oligonucleotide of a plurality of second splint oligonucleotides; b) hybridizing a first probe oligonucleotide and a second probe oligonucleotide to the target nucleic acid, where the first probe oligonucleotide and the second probe oligonucleotide each include a sequence that is substantially complementary to a first sequence and a second sequence of the target nucleic acid, respectively, and where the first probe oligonucleotide includes a first capture probe binding domain; c) coupling the first probe oligonucleotide and the second probe oligonucleotide, thereby generating a ligation product; d) determining a region of interest in the biological sample; e) selectively releasing the first capture probe, and optionally, the second capture probe from the array into the region of interest; and f) hybridizing the first capture domain of the first capture probe to the first capture probe binding domain of the ligation product, thereby spatially barcoding the target nucleic acid.

In some embodiments, the method includes contacting the biological sample with the first plurality of splint oligonucleotides, where the first splint oligonucleotide includes: (i) a sequence complementary to the ligation domain and (ii) a sequence complementary to the second ligation domain.

In some embodiments, the method includes hybridizing the first splint oligonucleotide to (i) the ligation domain and (ii) the second ligation domain.

In some embodiments, the method includes determining (i) all or part of the sequence of the ligation product hybridized to the first capture domain, or a complement thereof, and (ii) the sequence of the first spatial barcode, or a complement thereof, and, optionally, (iii) the sequence of the second spatial barcode, or a complement thereof, and using the determined sequences of (i) and (ii) and optionally (iii) to determine a location of the target nucleic acid in the biological sample.

In some embodiments, the biological sample is disposed on a substrate including the array. In some embodiments, the array is included on a substrate, and where the biological sample is disposed on a second substrate. In some embodiments, the method includes aligning the second substrate with the substrate including the array, such that at least a portion of the region of interest in the biological sample is aligned with at least a portion of the array.

In some embodiments, selectively releasing the first capture probe, and optionally, the second capture probe includes aligning the biological sample and the array such that the first capture probe, and optionally, the second capture probe are aligned with the region of interest in the biological sample, and applying an activation source to an area of the array including the first capture probe, and optionally, the second capture probe; thereby cleaving the first capture probe, and optionally, the second capture probe aligned with the one or more regions of interest.

Also provided herein are kits including: (a) an array including a first plurality of capture probes, where a first capture probe of the first plurality of capture probes includes: (i) a first spatial barcode and (ii) a first capture domain, and where the first capture probe is attached to a substrate by its 3' end, and a second plurality of capture probes, where a second capture probe of the second plurality of capture probes includes: (iii) a second spatial barcode and (iv) a second capture domain, and where the second capture probe is attached to the substrate by its 3' end; and (b) a plurality of probe pair oligonucleotides, where a probe pair oligonucleotide of the plurality of probe pair oligonucleotides includes a first probe oligonucleotide and a second probe oligonucleotide, where the first probe oligonucleotide and the second probe oligonucleotide each include a sequence that is substantially complementary to a first sequence and a second sequence of a target nucleic acid, respectively, and where the first probe oligonucleotide includes a first capture probe binding domain and the second probe oligonucleotide includes a second capture probe binding domain.

In some embodiments, the kit includes one or more of an RNase, a DNase, a protease, a lipase, and combinations thereof. In some embodiments, the protease is one or more of Proteinase K, pepsin, and collagenase. In some embodiments,

In some embodiments, the kit includes a ligase. In some embodiments, the ligase is one or more of a T4 RNA ligase (Rnl2), a PBCV-1 DNA ligase, a *Chlorella* virus DNA ligase, a single-stranded DNA ligase, or a T4 DNA ligase.

In some embodiments, the kit includes instructions for performing any of the methods described herein.

Also provided herein are kits including: (a) an array including a first plurality of capture probes, where a first capture probe of the first plurality of capture probes includes: (i) a first capture domain, (ii) a first spatial barcode, and (iii) a ligation domain substantially complementary to a portion of a first splint oligonucleotide of a plurality of first splint oligonucleotides, and optionally, a second plurality of capture probes, where a second capture probe of the second plurality of capture probes includes: (i) a second ligation domain substantially complementary to a portion of the first splint oligonucleotide, (ii) a second spatial barcode, and (iii) a third ligation domain substantially complementary to a portion of a second splint oligonucleotide of a plurality of second splint oligonucleotides; (b) a plurality of probe pair oligonucleotides including a first probe oligonucleotide and a second probe oligonucleotide, where the first probe oligonucleotide and the second probe oligonucleotide each include a sequence that is substantially complementary to a first sequence and a second sequence of a target nucleic acid, respectively, and where the first probe oligonucleotide includes a first capture probe binding domain; (c) a plurality of first splint oligonucleotides, where a first splint oligonucleotide of the plurality of first splint oligonucleotides includes a sequence substantially complementary to the ligation domain of the first capture probe and a sequence substantially complementary to the second ligation domain of the second capture probe; and (d) a ligase.

In some embodiments, the kit includes one or more of an RNase, a DNase, a protease, a lipase, and combinations thereof. In some embodiments, the protease is one or more of Proteinase K, pepsin, and collagenase.

In some embodiments, the ligase is one or more of a T4 RNA ligase (Rnl2), a PBCV-1 DNA ligase, a *Chlorella* virus DNA ligase, a single-stranded DNA ligase, or a T4 DNA ligase.

In some embodiments, the includes a plurality of second splint oligonucleotides, where a second splint oligonucleotide of the plurality of second splint oligonucleotides includes a sequence complementary to the third ligation domain of the second capture probe and a sequence complementary to a fourth ligation domain of a third capture probe of a plurality of third capture probes.

In some embodiments, the kit includes a plurality of third splint oligonucleotides, where a third splint oligonucleotide of the plurality of third splint oligonucleotides includes a sequence complementary to a fifth ligation domain of the third capture probe and a sequence complementary to a sixth ligation domain of a fourth capture probe of a plurality of fourth capture probes.

In some embodiments, the kit includes instructions for performing any of the methods described herein.

Also provided herein are compositions including: (a) an array including a first plurality of capture probes, where a first capture probe of the first plurality of capture probes includes: (i) a first spatial barcode and (ii) a first capture domain, and where the first capture probe is attached to a substrate by its 3' end, and a second plurality of capture probes, where a second capture probe of the second plurality of capture probes includes: (iii) a second spatial barcode and (iv) a second capture domain, and where the second capture probe is attached to the substrate by its 3' end; and (b) a plurality of probe pair oligonucleotides, where a probe pair oligonucleotide of the plurality of probe pair oligonucleotides includes a first probe oligonucleotide and a second probe oligonucleotide, where the first probe oligonucleotide and the second probe oligonucleotide each include a sequence that is substantially complementary to a first sequence and a second sequence of a target nucleic acid, respectively, and where the first probe oligonucleotide includes a first capture probe binding domain and the second probe oligonucleotide includes a second capture probe binding domain.

In some embodiments, the first probe oligonucleotide and the second probe oligonucleotide are hybridized to the target nucleic acid. In some embodiments, the first probe oligonucleotide and the second probe oligonucleotide are ligated, thereby generating a ligation product.

In some embodiments, the target nucleic acid is removed from the ligation product.

In some embodiments, the first capture probe, and optionally, the second capture probe are released from the array.

In some embodiments, the first capture probe, and optionally, the second capture probe hybridize to the first capture probe binding domain and the second capture probe binding domain, respectively.

In some embodiments, the target nucleic acid is removed from the ligation product.

In some embodiments, the first capture probe is extended, thereby generating a complement of the ligation product.

In some embodiments, the complement of the ligation product is ligated to the second capture probe.

In some embodiments, the composition includes one or more of an RNase, a DNase, a protease, a lipase, and combinations thereof. In some embodiments, the protease is one or more of Proteinase K, pepsin, and collagenase.

In some embodiments, the composition includes a ligase and a polymerase. In some embodiments, the ligase is one or more of a T4 RNA ligase (Rnl2), a PBCV-1 DNA ligase, a *Chlorella* virus DNA ligase, a single-stranded DNA ligase, or a T4 DNA ligase.

Also provided herein are compositions including: (a) an array including a first plurality of capture probes, where a first capture probe of the first plurality of capture probes includes: (i) a first capture domain, (ii) a first spatial barcode, and (iii) a ligation domain substantially complementary to a portion of a first splint oligonucleotide of a plurality of first splint oligonucleotides, and optionally, a second plurality of capture probes, where a second capture probe includes: (iv) a second ligation domain substantially complementary to a portion of the first splint oligonucleotide, (v) a second spatial barcode, and (vi) a third ligation domain substantially complementary to a portion of a second splint oligonucleotide of a plurality of second splint oligonucleotides; and (b) a plurality of probe pair oligonucleotides including a first probe oligonucleotide and a second probe oligonucleotide, where the first probe oligonucleotide and the second probe oligonucleotide each include a sequence that is substantially complementary to a first sequence and a second sequence of a target nucleic acid, respectively, and where the first probe oligonucleotide includes a first capture probe binding domain.

In some embodiments, the first probe oligonucleotide and the second probe oligonucleotide are hybridized to the target nucleic acid. In some embodiments, the first probe oligonucleotide and the second probe oligonucleotide are ligated, thereby generating a ligation product.

In some embodiments, the target nucleic acid is removed from the ligation product.

In some embodiments, the composition includes a plurality of first splint oligonucleotides, where a first splint oligonucleotide of the plurality of first splint oligonucleotides includes a sequence substantially complementary to the ligation domain of the first capture probe and a sequence substantially complementary to the second ligation domain of the second capture probe. In some embodiments, the first splint oligonucleotide is hybridized to the ligation domain of the first capture probe and to the second ligation domain of the second capture probe.

In some embodiments, the composition includes a plurality of second splint oligonucleotides, where a second splint oligonucleotide of the plurality of second splint oligonucleotides includes a sequence substantially complementary to a third ligation domain of the second capture probe and a sequence substantially complementary to a fourth ligation domain of a third capture probe of a plurality of third capture probes. In some embodiments, the second splint oligonucleotide is hybridized to the third ligation domain of the second capture probe and to the fourth ligation domain of the third capture probe.

In some embodiments, the composition includes a plurality of third splint oligonucleotides, where a third splint oligonucleotide of a plurality of third splint oligonucleotides includes a sequence substantially complementary to a fifth ligation domain of the third capture probe and a sequence substantially complementary to a sixth ligation domain of a fourth capture probe of a plurality of fourth capture probes. In some embodiments, the third splint oligonucleotide is hybridized to the fifth ligation domain of the third capture probe and to the sixth ligation domain of the fourth capture probe.

In some embodiments, the first capture probe is ligated to the second capture probe, and optionally, the second capture probe is ligated to the third capture probe, and the third capture probe is ligated to the fourth capture probe.

In some embodiments, the first capture probe is extended, thereby generating a complement of the ligation product.

In some embodiments, the composition includes one or more of an RNase, a DNase, a protease, a lipase, and combinations thereof. In some embodiments, the protease is one or more of Proteinase K, pepsin, and collagenase.

In some embodiments, the composition includes a ligase and a polymerase.

In some embodiments, the ligase is one or more of a T4 RNA ligase (Rnl2), a PBCV-1 DNA ligase, a *Chlorella* virus DNA ligase, a single-stranded DNA ligase, or a T4 DNA ligase.

Where values are described in terms of ranges, it should be understood that the description includes the disclosure of all possible sub-ranges within such ranges, as well as specific numerical values that fall within such ranges irrespective of whether a specific numerical value or specific sub-range is expressly stated.

The term "about" or "approximately" as used herein means within an acceptable error range for the particular value as determined by one of ordinary skill in the art, which will depend in part on how the value is measured or determined, i.e., the limitations of the measurement system. For example, "about" can mean within an acceptable standard deviation, per the practice in the art. Alternatively, "about" can mean a range of up to ±20%, preferably up to ±10%, more preferably up to ±5%, and more preferably still up to ±1% of a given value. Alternatively, particularly with respect to biological systems or processes, the term can mean within an order of magnitude, preferably within 2-fold, of a value. Where particular values are described in the application and claims, unless otherwise stated, the term "about" is implicit and in this context means within an acceptable error range for the particular value.

The term "substantially complementary" used herein means that a first sequence is at least 50%, 55%, 60%, 65%, 70%, 75%, 80%, 85%, 90%, 95%, 97%, 98% or 99% identical to the complement of a second sequence over a region of 2, 3, 4, 5, 6, 7, 8, 9, 10, 11, 12, 13, 14, 15, 16, 17, 18, 19, 20-40, 40-60, 60-100, or more nucleotides, or that the two sequences hybridize under stringent hybridization conditions. Substantially complementary also means that a sequence in one strand is not completely and/or perfectly complementary to a sequence in an opposing strand, but that sufficient bonding occurs between bases on the two strands to form a stable hybrid complex in set of hybridization conditions (e.g., salt concentration and temperature). Such conditions can be predicted by using the sequences and standard mathematical calculations known to those skilled in the art.

The term "each," when used in reference to a collection of items, is intended to identify an individual item in the collection but does not necessarily refer to every item in the collection, unless expressly stated otherwise, or unless the context of the usage clearly indicates otherwise.

The term "about" or "approximately" as used herein means within an acceptable error range for the particular value as determined by one of ordinary skill in the art, which will depend in part on how the value is measured or determined, i.e., the limitations of the measurement system. For example, "about" can mean within an acceptable standard deviation, per the practice in the art. Alternatively, "about" can mean a range of up to ±20%, preferably up to ±10%, more preferably up to ±5%, and more preferably still up to ±1% of a given value. Alternatively, particularly with respect to biological systems or processes, the term can mean within an order of magnitude, preferably within 2-fold, of a value. Where particular values are described in the application and claims, unless otherwise stated, the term "about" is implicit and in this context means within an acceptable error range for the particular value.

The term "substantially complementary" used herein means that a first sequence is at least 50%, 55%, 60%, 65%, 70%, 75%, 80%, 85%, 90%, 95%, 97%, 98% or 99% identical to the complement of a second sequence over a region of 2, 3, 4, 5, 6, 7, 8, 9, 10, 11, 12, 13, 14, 15, 16, 17, 18, 19, 20-40, 40-60, 60-100, or more nucleotides, or that the two sequences hybridize under stringent hybridization conditions. Substantially complementary also means that a sequence in one strand is not completely and/or perfectly complementary to a sequence in an opposing strand, but that sufficient bonding occurs between bases on the two strands to form a stable hybrid complex in set of hybridization conditions (e.g., salt concentration and temperature). Such conditions can be predicted by using the sequences and standard mathematical calculations known to those skilled in the art.

### DESCRIPTION OF DRAWINGS

The following drawings illustrate certain embodiments of the features and advantages of this disclosure. Like reference symbols in the drawings indicate like elements.
**FIG. 1A** shows an exemplary sandwiching process where a first substrate (e.g., a slide), including a biological sample, and a second substrate (e.g., array slide) are brought into proximity with one another.
**FIG. 1B** shows a fully formed sandwich configuration creating a chamber formed from the one or more spacers, the first substrate, and the second substrate.
**FIG. 2A** shows a perspective view of an exemplary sample handling apparatus in a closed position.
**FIG. 2B** shows a perspective view of an exemplary sample handling apparatus in an open position.
**FIG. 3A** shows the first substrate angled over (superior to) the second substrate.
**FIG. 3B** shows that as the first substrate lowers, and/or as the second substrate rises, the dropped side of the first substrate may contact a drop of reagent medium.
**FIG. 3C** shows a full closure of the sandwich between the first substrate and the second substrate with one or more spacers contacting both the first substrate and the second substrate.
**FIG. 4A** shows a side view of the angled closure workflow.
**FIG. 4B** shows a top view of the angled closure workflow.
**FIG. 5** is a schematic diagram showing an example of a barcoded capture probe, as described herein.
**FIG. 6** shows a schematic illustrating a cleavable capture probe.
**FIG. 7** shows exemplary capture domains on capture probes.
**FIG. 8** shows an exemplary arrangement of barcoded features within an array.
**FIG. 9A** shows and exemplary workflow for performing templated capture and producing a ligation product, and **FIG. 9B** shows an exemplary workflow for capturing a ligation product from **FIG. 9A** on a substrate.
**FIG. 10** is a schematic diagram of an exemplary analyte capture agent.
**FIG. 11** is a schematic diagram depicting an exemplary interaction between a feature-immobilized capture probe **1124** and an analyte capture agent **1126.**
**FIG. 12A** is a schematic diagram showing an example of barcoded capture probes on a substrate where the capture probes are affixed to the substrate via their 3' ends.
**FIG. 12B** is a schematic diagram showing an example of barcoded capture probes on a substrate where the capture probes are affixed to the substrate via their 5' ends.
**FIG. 13A** is a schematic diagram showing an example of a substrate with barcoded capture probes that include a cleavable linker and a second substrate containing a biological sample in a sandwiching process.
**FIG. 13B** is a schematic diagram showing an example of a substrate with barcoded capture probes that include a cleavable linker and wherein the biological sample is mounted on the substrate.
**FIG. 14A** is a schematic diagram showing an example of a substrate with barcoded capture probes, wherein the analyte from a biological sample on the second substrate can be hybridized with two unique barcodes, whose combination can represent an individual spot on the substrate.
**FIG. 14B** is a schematic diagram showing an example of a substrate after the barcoded capture probes are released from the substrate and the analyte from a biological sample on the second substrate is hybridized with two unique barcodes, whose combination can represent an individual spot on the substrate.
**FIG. 15A** is a schematic diagram showing an example of a target analyte (e.g., mRNA) wherein the two unique barcodes are bound to the RNA-templated ligation (RTL) probes.
**FIG. 15B** shows an example of combinations of the barcodes that can represent different locations on a substrate, wherein both barcodes can be in a single spot and the barcode combination represents the actual barcoded spot on the substrate.
**FIG. 15C** shows an example of combinations of the barcodes that can represent different locations on a substrate, wherein a single barcode can be in a single spot and the barcode combination represents a shared neighboring area around a spot (each red diamond).
**FIG. 16** is a schematic diagram showing an example of a target analyte (e.g., mRNA) wherein the two unique barcodes are bound to the RNA-templated ligation (RTL) probes, and they include different quality control (QC) sequences.
**FIG. 17** is a schematic illustrating an exemplary workflow protocol utilizing a sandwiching process.
**FIG. 18A** is a schematic diagram showing an example of a substrate with two barcodes including cleavable linkers that can be cleaved at different time points (e.g., t1 and t2).
**FIG. 18B** is a schematic diagram showing an example of the first set of barcodes being released from the substrate at a first time point t1.
**FIG. 18C** is a schematic diagram showing an example of the second set of barcodes being released from the substrate at a second time point t2.
**FIG. 19A** is a schematic diagram showing an example of a dual spatial barcoding method wherein a first set of barcodes can be released from the substrate to label a target analyte on the second substrate (e.g., tissue slide) and a second set of barcodes that can capture the target analyte on the substrate.
**FIG. 19B** is a schematic diagram showing an example of the first set of barcodes being released from the substrate to label the target analyte on the second substrate.
**FIG. 19C** is a schematic diagram showing an example of the second set of barcodes capturing the target analyte bound to the first barcode after the target analyte is released from the second substrate.

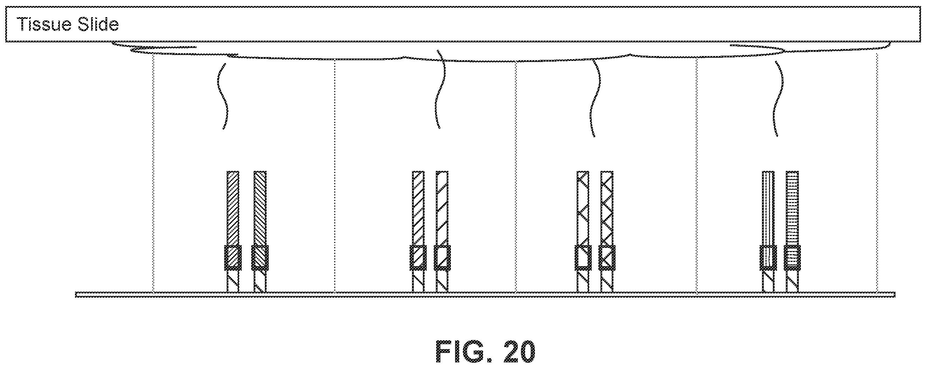

**FIG. 20** is a schematic diagram showing an example of a well array, wherein a well includes two unique barcodes and can be used to limit lateral displacement from the barcode origins.
**FIG. 21** is a schematic illustrating an exemplary workflow protocol utilizing a sandwiching process, wherein a plurality of barcoded capture probes are at a single spot on a substrate, filling a chamber between the first substrate and second substrate with a reagent medium and a plurality of splint oligonucleotides, releasing the plurality of barcoded capture probes from the first substrate wherein the plurality of splint oligonucleotides hybridize to the barcoded capture probes, and the target analytes are released from the second substrate and collected.
**FIG. 22** is a schematic illustrating an exemplary workflow protocol utilizing a sandwiching process.
**FIG. 23A** is a schematic diagram showing an example of a plurality of barcoded capture probes hybridized to a plurality of splint oligonucleotides, wherein a barcoded capture probe is hybridized to a target analyte (e.g., mRNA).
**FIG. 23B** shows an example of combinations of the barcodes that can represent different locations on a substrate, wherein a single barcode can be in a single spot and the unique combinations of four barcodes represent a shared neighboring area (each red diamond).
**FIG. 24A** is a schematic diagram showing an example of a plurality of barcoded capture probes hybridized to a target analyte (e.g., mRNA), wherein each barcoded capture probe can include a unique molecular identifier (UMI).
**FIG. 24B** shows an example of using combinations of the plurality of splint oligonucleotides to determine a relative distance from a spot and suppress potential mislocalization of the splint oligonucleotides.
**FIG. 25A** is a schematic diagram showing an example of a substrate with barcoded capture probes that include a cleavable linker and a second substrate containing a biological sample in a sandwiching process. **FIG. 25A** also shows a mask applied to the substrate with the barcoded capture probes. Regions of Interest (ROIs) are identified, and the mask has been selectively applied to the substrate with barcoded capture probes in areas that do not align or overlap with the regions of interest.
**FIG. 25B** is a schematic diagram showing the substrate with barcoded capture probes exposed to a light source. Exposure results in selective release of barcoded capture probes in areas that align or overlap with the regions of interest, e.g., where the mask has not been applied and barcoded capture probes are not released (e.g., remain attached to the substrate) in areas of the substrate that remain protected by the mask.
**FIG. 25C** is a schematic diagram showing released barcoded capture probes hybridizing with target analytes or an analyte derived molecule.

### DETAILED DESCRIPTION

### A. Spatial Analysis Methods

Spatial analysis methodologies and compositions described herein can provide a vast amount of analyte and/or expression data for a variety of analytes within a biological sample at high spatial resolution, while retaining native spatial context. Spatial analysis methods and compositions can include, e.g., the use of a capture probe including a spatial barcode (e.g., a nucleic acid sequence that provides information as to the location or position of an analyte within a cell or a tissue sample (e.g., mammalian cell or a mammalian tissue sample) and a capture domain that is capable of binding to an analyte (e.g., a protein and/or a nucleic acid) produced by and/or present in a cell. Spatial analysis methods and compositions can also include the use of a capture probe having a capture domain that captures an intermediate agent for indirect detection of an analyte. For example, the intermediate agent can include a nucleic acid sequence (e.g., a barcode) associated with the intermediate agent. Detection of the intermediate agent is therefore indicative of the analyte in the cell or tissue sample. Intermediate agents (e.g., ligation products or other sequences) can serve as proxies of target analytes in the methods and compositions herein.

Non-limiting aspects of spatial analysis methodologies and compositions are described in U.S. Patent Nos. 11,447,807, 11,352,667, 11,168,350, 11,104,936, 11,008,608, 10,995,361, 10,913,975, 10,774,374, 10,724,078, 10,640,816, 10,494,662, 10,480,022, 10,364,457, 10,317,321, 10,059,990, 10,041,949, 10,030,261, 10,002,316, 9,879,313, 9,783,841, 9,727,810, 9,593,365, 8,951,726, 8,604,182, and 7,709,198; U.S. Patent Application Publication Nos. 2020/0239946, 2020/0080136, 2020/0277663, 2019/0330617, 2020/0256867, 2020/0224244, 2019/0085383, and 2013/0171621; PCT Publication Nos. WO2018/091676, WO2020/176788, WO2017/144338, and WO2016/057552; Non-patent literature references Rodriques et al., Science 363(6434):1463-1467, 2019; Lee et al., Nat. Protoc. 10(3):442-458, 2015; Trejo et al., PLoS ONE 14(2):e0212031, 2019; Chen et al., Science 348(6233):aaa6090, 2015; Gao et al., BMC Biol. 15:50, 2017; and Gupta et al., Nature Biotechnol. 36:1197-1202, 2018; the Visium Spatial Gene Expression Reagent Kits User Guide (e.g., Rev F, dated January 2022); and/or the Visium Spatial Gene Expression Reagent Kits - Tissue Optimization User Guide (e.g., Rev E, dated February 2022), both of which are available at the 10x Genomics Support Documentation website, and can be used herein in any combination. WO 2021/133849 A1 discloses spatial transcription profiling by proximity ligation of two probes where target RNA provides the template for splinted ligation (RNA templated ligation). The ligation product can be captured via a capture domain on an addressable array. Alternatively, the spatially barcoded capture probes can be released into the biological sample from the array. WO 2020/123309 A1 discloses RNA templated ligation of target specific probes comprising capture binding domains. Spatially barcoded capture probes comprising capture domains are released from the array. WO 2020/123309 A1 also discloses splinted ligation between spatially barcoded capture probes. Further non-limiting aspects of spatial analysis methodologies and compositions are described herein.

Some general terminology that may be used in this disclosure can be found in Section (I)(b) of PCT Publication No. WO2020/176788 and/or U.S. Patent Application Publication No. 2020/0277663. Typically, a "barcode" is a label, or identifier, which conveys or is capable of conveying information (e.g., information about an analyte in a sample, a bead, and/or a capture probe). A barcode can be part of an analyte, or independent of an analyte. A barcode can be attached to an analyte. A particular barcode can be unique relative to other barcodes. For the purpose of this disclosure, an "analyte" can include any biological substance, structure, moiety, or component to be analyzed. The term "target" can similarly refer to an analyte of interest.

Analytes can be broadly classified into one of two groups: nucleic acid analytes, and non-nucleic acid analytes. Examples of nucleic acid analytes include, but are not limited to, DNA, RNA (e.g., mRNA), and combinations thereof. Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral proteins (e.g., viral capsid, viral envelope, viral coat, viral accessory, viral glycoproteins, viral spike, etc.), extracellular and intracellular proteins, antibodies, and antigen binding fragments. In some embodiments, the analyte(s) can be localized to subcellular location(s), including, for example, organelles, e.g., mitochondria, Golgi apparatus, endoplasmic reticulum, chloroplasts, endocytic vesicles, exocytic vesicles, vacuoles, lysosomes, etc. In some embodiments, analyte(s) can be peptides or proteins, including without limitation antibodies and enzymes. Additional examples of analytes can be found in Section (I)(c) of PCT Publication No. WO2020/176788 and/or U.S. Patent Application Publication No. 2020/0277663. In some embodiments, an analyte can be detected indirectly, such as through detection of an intermediate agent, for example, a ligation product or an analyte capture agent (e.g., an oligonucleotide-conjugated antibody), such as those described herein.

A "biological sample" is typically obtained from the subject for analysis using any of a variety of techniques including, but not limited to, biopsy, surgery, and laser capture microscopy (LCM), and generally includes cells and/or other biological material from the subject. In some embodiments, a biological sample can be a tissue section. In some embodiments, the biological sample (e.g., tissue sample) is a tissue microarray (TMA). A tissue microarray contains multiple representative tissue samples - which can be from different tissues or organisms - assembled on a single histologic slide. The TMA can therefore allow for high throughput analysis of multiple specimens at the same time. Tissue microarrays are paraffin blocks produced by extracting cylindrical tissue cores from different paraffin donor blocks and re-embedding these into a single recipient (microarray) block at defined array coordinates.

The biological sample as used herein can be any suitable biological sample described herein or known in the art. In some embodiments, the biological sample is a tissue. In some embodiments, the tissue sample is a solid tissue sample. In some embodiments, the biological sample is a tissue section. In some embodiments, the tissue is flash-frozen and sectioned. Any suitable method described herein or known in the art can be used to flash-freeze and section the tissue sample. In some embodiments, the biological sample, e.g., the tissue, is flash-frozen using liquid nitrogen before sectioning. In some embodiments, the biological sample, e.g., a tissue sample, is flash-frozen using nitrogen (e.g., liquid nitrogen), isopentane, or hexane.

In some embodiments, the biological sample, e.g., the tissue, is embedded in a matrix e.g., optimal cutting temperature (OCT) compound to facilitate sectioning. OCT compound is a formulation of clear, water-soluble glycols and resins, providing a solid matrix to encapsulate biological (e.g., tissue) specimens. In some embodiments, the sectioning is performed bycryosectioning, for example using a microtome. In some embodiments, the methods further comprise a thawing step, after the cryosectioning.

The biological sample can be from a mammal. In some instances, the biological sample is from a human, mouse, or rat. In addition to the subjects described above, the biological sample can be obtained from non-mammalian organisms (e.g., a plants, an insect, an arachnid, a nematode (e.g., *Caenorhabditis elegans),* a fungi, an amphibian, or a fish (e.g., zebrafish)). A biological sample can be obtained from a prokaryote such as a bacterium, e.g., *Escherichia coli, Staphylococci* or *Mycoplasma pneumoniae;* an archaea; a virus such as Hepatitis C virus or human immunodeficiency virus; or a viroid. A biological sample can be obtained from a eukaryote, such as a patient derived organoid (PDO) or patient derived xenograft (PDX). The biological sample can include organoids, a miniaturized and simplified version of an organ produced in vitro in three dimensions that shows realistic micro-anatomy. Organoids can be generated from one or more cells from a tissue, embryonic stem cells, and/or induced pluripotent stem cells, which can self-organize in three-dimensional culture owing to their self-renewal and differentiation capacities. In some embodiments, an organoid is a cerebral organoid, an intestinal organoid, a stomach organoid, a lingual organoid, a thyroid organoid, a thymic organoid, a testicular organoid, a hepatic organoid, a pancreatic organoid, an epithelial organoid, a lung organoid, a kidney organoid, a gastruloid, a cardiac organoid, or a retinal organoid. Subjects from which biological samples can be obtained can be healthy or asymptomatic individuals, individuals that have or are suspected of having a disease (e.g., cancer) or a pre-disposition to a disease, and/or individuals that are in need of therapy or suspected of needing therapy.

Biological samples can be derived from a homogeneous culture or population of the subjects or organisms mentioned herein or alternatively from a collection of several different organisms, for example, in a community or ecosystem.

Biological samples can include one or more diseased cells. A diseased cell can have altered metabolic properties, gene expression, protein expression, and/or morphologic features. Examples of diseases include inflammatory disorders, metabolic disorders, nervous system disorders, and cancer. Cancer cells can be derived from solid tumors, hematological malignancies, cell lines, or obtained as circulating tumor cells.

In some embodiments, the biological sample, e.g., the tissue sample, is fixed in a fixative including alcohol, for example methanol. In some embodiments, instead of methanol, acetone, or an acetone-methanol mixture can be used. In some embodiments, the fixation is performed after sectioning. In some instances, the biological sample is not fixed with paraformaldehyde (PFA). In some instances, when the biological sample is fixed with a fixative including an alcohol (e.g., methanol or acetone-methanol mixture), it is not decrosslinked afterward. In some preferred embodiments, the biological sample is fixed with a fixative including an alcohol (e.g., methanol or an acetone-methanol mixture) after freezing and/or sectioning. In some instances, the biological sample is flash-frozen, and then the biological sample is sectioned and fixed (e.g., using methanol, acetone, or an acetone-methanol mixture). In some instances when methanol, acetone, or an acetone-methanol mixture is used to fix the biological sample, the sample is not decrosslinked at a later step. In instances when the biological sample is frozen (e.g., flash frozen using liquid nitrogen and embedded in OCT) followed by sectioning and alcohol (e.g., methanol, acetone-methanol) fixation or acetone fixation, the biological sample is referred to as "fresh frozen". In some embodiments, fixation of the biological sample e.g., using acetone and/or alcohol (e.g., methanol, acetone-methanol) is performed while the sample is mounted on a substrate (e.g., glass slide, such as a positively charged glass slide).

In some embodiments, the biological sample, e.g., the tissue sample, is fixed e.g., immediately after being harvested from a subject. In such embodiments, the fixative is preferably an aldehyde fixative, such as paraformaldehyde (PFA) or formalin. In some embodiments, the fixative induces crosslinks within the biological sample. In some embodiments, after fixing e.g., by formalin or PFA, the biological sample is dehydrated via sucrose gradient. In some instances, the fixed biological sample is treated with a sucrose gradient and then embedded in a matrix e.g., OCT compound. In some instances, the fixed biological sample is not treated with a sucrose gradient, but rather is embedded in a matrix e.g., OCT compound after fixation. In some embodiments when a fixed frozen tissue sample is treated with a sucrose gradient, it can be rehydrated with an ethanol gradient. In some embodiments, the PFA or formalin fixed biological sample, which can be optionally dehydrated via sucrose gradient and/or embedded in OCT compound, is then frozen e.g., for storage or shipment. In such instances, the biological sample is referred to as "fixed frozen". In preferred embodiments, a fixed frozen biological sample is not treated with methanol. In preferred embodiments, a fixed frozen biological sample is not paraffin embedded. Thus, in preferred embodiments, a fixed frozen biological sample is not deparaffinized. In some embodiments, a fixed frozen biological sample is rehydrated in an ethanol gradient.

In some instances, the biological sample (e.g., a fixed frozen tissue sample) is treated with a citrate buffer. Citrate buffer can be used for antigen retrieval to decrosslink antigens and fixation medium in the biological sample. Thus, any suitable decrosslinking agent can be used in addition to or alternatively to citrate buffer. In some embodiments, for example, the biological sample (e.g., a fixed frozen tissue sample) is decrosslinked with TE buffer.

In any of the foregoing, the biological sample can further be stained, imaged, and/or destained. For example, in some embodiments, a fresh frozen tissue sample or fixed frozen tissue sample is stained (e.g., via eosin and/or hematoxylin), imaged, destained (e.g., via HCl), or a combination thereof. In some embodiments, when a fresh frozen tissue sample is fixed in methanol, it is treated with isopropanol prior to being stained (e.g., via eosin and/or hematoxylin), imaged, destained (e.g., via HCl), or a combination thereof. In some embodiments when a fixed frozen tissue sample is treated with a sucrose gradient, it can be rehydrated with an ethanol gradient before being stained, (e.g., via eosin and/or hematoxylin), imaged, destained (e.g., via HCl), decrosslinked (e.g., via TE buffer or citrate buffer), or a combination thereof. In some embodiments, the biological sample can undergo further fixation (e.g., while mounted on a substrate), stained, imaged, and/or destained. For example, a fixed frozen biological sample may be subject to an additional fixing step (e.g., using PFA) before optional ethanol rehydration, staining, imaging, and/or destaining.

In any of the foregoing, the biological sample can be fixed using PAXgene. For example, the biological sample can be fixed using PAXgene in addition, or alternatively to, a fixative disclosed herein or known in the art (e.g., alcohol, acetone, acetone-alcohol, formalin, paraformaldehyde). PAXgene is a non-cross-linking mixture of different alcohols, acid and a soluble organic compound that preserves morphology and bio-molecules. It is a two-reagent fixative system in which tissue is firstly fixed in a solution containing methanol and acetic acid then stabilized in a solution containing ethanol. See, Ergin B. et al., J Proteome Res. 2010 Oct 1;9(10):5188-96; Kap M. et al., PLoS One.; 6(11):e27704 (2011); and Mathieson W. et al., Am J Clin Pathol.; 146(1):25-40 (2016), for a description and evaluation of PAXgene for tissue fixation. Thus, in some embodiments, when the biological sample, e.g., the tissue sample, is fixed in a fixative including alcohol, the fixative is PAXgene. In some embodiments, a fresh frozen tissue sample is fixed with PAXgene. In some embodiments, a fixed frozen tissue sample is fixed with PAXgene.

In some embodiments, the biological sample, e.g., the tissue sample is fixed, for example in methanol, acetone, acetone-methanol, PFA, PAXgene or is formalin-fixed and paraffin-embedded (FFPE). In some embodiments, the biological sample comprises intact cells. In some embodiments, the biological sample is a cell pellet, e.g., a fixed cell pellet, e.g., an FFPE cell pellet. FFPE samples are used in some instances in the RTL methods disclosed herein. A limitation of direct RNA capture for fixed samples is that the RNA integrity of fixed (e.g., FFPE) samples can be lower than a fresh sample, thereby making it more difficult to capture RNA directly, e.g., by capture of a common sequence such as a poly(A) tail of an mRNA molecule. However, by utilizing RTL probes that hybridize to RNA target sequences in the transcriptome, one can avoid a requirement for RNA analytes to have both a poly(A) tail and target sequences intact. Accordingly, RTL probes can be utilized to beneficially improve capture and spatial analysis of fixed samples. The biological sample, e.g., tissue sample, can be stained, and imaged prior, during, and/or after each step of the methods described herein. Any of the methods described herein or known in the art can be used to stain and/or image the biological sample. In some embodiments, the imaging occurs prior to destaining the sample. In some embodiments, the biological sample is stained using an H&E staining method. In some embodiments, the tissue sample is stained and imaged for about 10 minutes to about 2 hours (or any of the subranges of this range described herein). Additional time may be needed for staining and imaging of different types of biological samples.

The tissue sample can be obtained from any suitable location in a tissue or organ of a subject, e.g., a human subject. In some instances, the sample is a mouse sample. In some instances, the sample is a human sample. In some embodiments, the sample can be derived from skin, brain, breast, lung, liver, kidney, prostate, tonsil, thymus, testes, bone, lymph node, ovary, eye, heart, or spleen. In some instances, the sample is a human or mouse breast tissue sample. In some instances, the sample is a human or mouse brain tissue sample. In some instances, the sample is a human or mouse lung tissue sample. In some instances, the sample is a human or mouse tonsil tissue sample. In some instances, the sample is a human or mouse liver tissue sample. In some instances, the sample is a human or mouse bone, skin, kidney, thymus, testes, or prostate tissue sample. In some embodiments, the tissue sample is derived from normal or diseased tissue. In some embodiments, the sample is an embryo sample. The embryo sample can be a non-human embryo sample. In some instances, the sample is a mouse embryo sample.

Non-limiting examples of stains include histological stains (e.g., hematoxylin and/or eosin) and immunological stains (e.g., fluorescent stains). The biological sample can be stained using Can-Grunwald, Giemsa, hematoxylin and eosin (H&E), Jenner's, Leishman, Masson's trichrome, Papanicolaou, Romanowsky, silver, Sudan, Wright's, and/or Periodic Acid Schiff (PAS) staining techniques. In some instances, PAS staining is performed after formalin or acetone fixation. In some embodiments, a biological sample (e.g., a fixed and/or stained biological sample) can be imaged. Biological samples are also described in Section (I)(d) of PCT Publication No. WO2020/176788 and/or U.S. Patent Application Publication No. 2020/0277663.

The following embodiments can be used with any of the methods described herein. In some embodiments, the biological sample is imaged. In some embodiments, the biological sample is visualized or imaged using bright field microscopy. In some embodiments, the biological sample is visualized or imaged using fluorescence microscopy. Additional methods of visualization and imaging are known in the art. Non-limiting examples of visualization and imaging include expansion microscopy, bright field microscopy, dark field microscopy, phase contrast microscopy, electron microscopy, fluorescence microscopy, reflection microscopy, interference microscopy and confocal microscopy. In some embodiments, the sample is stained and imaged prior to adding reagents for analyzing captured analytes as disclosed herein to the biological sample.

In some embodiments, the method includes staining the biological sample. In some embodiments, the staining includes the use of hematoxylin and eosin. In some embodiments, a biological sample can be stained using any number of biological stains, including but not limited to, acridine orange, Bismarck brown, carmine, coomassie blue, cresyl violet, DAPI, eosin, ethidium bromide, acid fuchsine, hematoxylin, Hoechst stains, iodine, methyl green, methylene blue, neutral red, Nile blue, Nile red, osmium tetroxide, propidium iodide, rhodamine, or safranin. In some instances, the biological sample can be stained using known staining techniques, including Can-Grunwald, Giemsa, hematoxylin and eosin (H&E), Jenner's, Leishman, Masson's trichrome, Papanicolaou, Romanowsky, silver, Sudan, Wright's, and/or Periodic Acid Schiff (PAS) staining techniques. PAS staining is typically performed after formalin or acetone fixation.

In some embodiments, the staining includes the use of a detectable label selected from the group consisting of a radioisotope, a fluorophore, a chemiluminescent compound, a bioluminescent compound, or a combination thereof.

In some embodiments, a biological sample is permeabilized with one or more permeabilization reagents. For example, permeabilization of a biological sample can facilitate analyte capture. Exemplary permeabilization agents and conditions are described in Section (I)(d)(ii)(13) or the Exemplary Embodiments Section of PCT Publication No. WO2020/176788 and/or U.S. Patent Application Publication No. 2020/0277663. Briefly, in any of the methods described herein, the method includes a step of permeabilizing the biological sample. For example, the biological sample can be permeabilized to facilitate transfer of the extension products to the capture probes on the array. In some embodiments, the permeabilizing includes the use of an organic solvent (e.g., acetone, ethanol, and methanol), a detergent (e.g., saponin, Triton X-100^{™}, Tween-20^{™}, or sodium dodecyl sulfate (SDS)), an enzyme (an endopeptidase, an exopeptidase, a protease), or combinations thereof. In some embodiments, the permeabilizing includes the use of an endopeptidase, a protease, SDS, polyethylene glycol tert-octylphenyl ether, polysorbate 80, and polysorbate 20, N-lauroylsarcosine sodium salt solution, saponin, Triton X-100^{™}, Tween-20^{™}, or combinations thereof. In some embodiments, the endopeptidase is pepsin. In some embodiments, the endopeptidase is Proteinase K. Additional methods for sample permeabilization are described, for example, in Jamur et al., Method Mol. Biol. 588:63-66, 2010.

Array-based spatial analysis methods involve the transfer of one or more analytes from a biological sample to an array of features on a substrate, where each feature is associated with a unique spatial location on the array. Subsequent analysis of the transferred analytes includes determining the identity of the analytes and the spatial location of the analytes within the biological sample. The spatial location of an analyte within the biological sample is determined based on the feature to which the analyte is bound (e.g., directly or indirectly) on the array, and the feature's relative spatial location within the array.

A "capture probe" refers to any molecule capable of capturing (directly or indirectly) and/or labelling an analyte (e.g., an analyte of interest) in a biological sample. In some embodiments, the capture probe is a nucleic acid or a polypeptide. In some embodiments, the capture probe includes a barcode (e.g., a spatial barcode and/or a unique molecular identifier (UMI) and a capture domain). In some instances, the capture probe includes a homopolymer sequence, such as a poly(T) sequence. In some embodiments, a capture probe can include a cleavage domain and/or a functional domain (e.g., a primer-binding site, such as for next-generation sequencing (NGS)). See, e.g., Section (II)(b) (e.g., subsections (i)-(vi)) of PCT Publication No. WO2020/176788 and/or U.S. Patent Application Publication No. 2020/0277663. Generation of capture probes can be achieved by any appropriate method, including those described in Section (II)(d)(ii) of PCT Publication No. WO2020/176788 and/or U.S. Patent Application Publication No. 2020/0277663.

In some instances, a capture probe and a nucleic acid analyte (or any other nucleic acid to nucleic acid interaction) occurs because the sequences of the two nucleic acids are substantially complementary to one another. By "substantial," "substantially" and the like, two nucleic acid sequences can be complementary when at least 60% of the nucleotide residues of one nucleic acid sequence are complementary to nucleotide residues in the other nucleic acid sequence. The complementary residues within a particular complementary nucleic acid sequence need not always be contiguous with each other and can be interrupted by one or more non-complementary residues within the complementary nucleic acid sequence. In some embodiments, at least 60%, but less than 100%, of the residues of one of the two complementary nucleic acid sequences are complementary to residues in the other nucleic acid sequence. In some embodiments, at least 70%, 80%, 90%, 95% or 99% of the residues of one nucleic acid sequence are complementary to residues in the other nucleic acid sequence. Sequences are said to be "substantially complementary" when at least 60% (e.g., at least 70%, at least 80%, or at least 90%) of the residues of one nucleic acid sequence are complementary to residues in the other nucleic acid sequence.

In some embodiments, more than one analyte type (e.g., nucleic acids and proteins) from a biological sample can be detected (e.g., simultaneously or sequentially) using any appropriate multiplexing technique, such as those described in Section (IV) of PCT Publication No. WO2020/176788 and/or U.S. Patent Application Publication No. 2020/0277663.

In some embodiments, detection of one or more analytes (e.g., protein analytes) can be performed using one or more analyte capture agents. As used herein, an "analyte capture agent" refers to an agent that interacts with an analyte (e.g., an analyte in a biological sample) and with a capture probe (e.g., a capture probe attached to a substrate or a feature) to identify the analyte. In some embodiments, the analyte capture agent includes: (i) an analyte binding moiety (e.g., that binds to an analyte), for example, an antibody or antigen-binding fragment thereof; (ii) analyte binding moiety barcode; and (iii) an analyte capture sequence. As used herein, the term "analyte binding moiety barcode" refers to a barcode that is associated with or otherwise identifies the analyte binding moiety. As used herein, the term "analyte capture sequence" refers to a region or moiety configured to hybridize to, bind to, couple to, or otherwise interact with a capture domain of a capture probe. In some cases, an analyte binding moiety barcode (or portion thereof) may be able to be removed (e.g., cleaved) from the analyte capture agent. Additional description of analyte capture agents can be found in Section (II)(b)(ix) of PCT Publication No. WO2020/176788 and/or Section (II)(b)(viii) U.S. Patent Application Publication No. 2020/0277663.

In some embodiments, the biological sample is mounted on a first substrate and the substrate comprising the array of capture probes is on a second substrate. During this process, one or more analytes or analyte derivatives (e.g., intermediate agents; e.g., ligation products) are released from the biological sample and migrated to the second substrate comprising an array of capture probes. In some embodiments, the release and migration of the analytes or analyte derivatives to the second substrate comprising the array of capture probes occurs in a manner that preserves the original spatial context of the analytes in the biological sample. This method can be referred to as a sandwiching process, which is described e.g., in U.S. Patent Application Pub. No. 2021/0189475 and PCT Pub. Nos. WO 2021/252747 A1, WO 2022/061152 A2, and WO 2022/140028 A1.

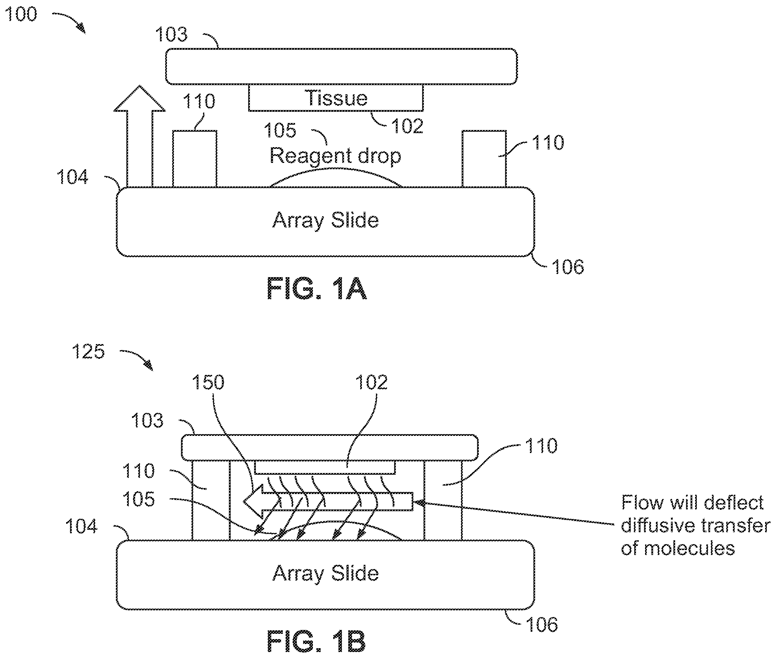

FIG. 1A shows an exemplary sandwiching process 100 where a first substrate (e.g., slide 103), including a biological sample 102 (e.g., a tissue section), and a second substrate (e.g., array slide 104 including an array having spatially barcoded capture probes 106) are brought into proximity with one another. As shown in FIG. 1A a liquid reagent drop (e.g., permeabilization solution 105) is introduced on the second substrate in proximity to the capture probes 106 and in between the biological sample 102 and the second substrate (e.g., slide 104 including an array having spatially barcoded capture probes 106). The permeabilization solution 105 may release analytes or analyte derivatives (e.g., intermediate agents; e.g., ligation products) that can be captured by the capture probes of the array 106.

During the exemplary sandwiching process, the first substrate is aligned with the second substrate, such that at least a portion of the biological sample is aligned with at least a portion of the capture probes (e.g., aligned in a sandwich configuration). As shown, the second substrate (e.g., array slide 104) is in an inferior position to the first substrate (e.g., slide 103). In some embodiments, the first substrate (e.g., slide 103) may be positioned superior to the second substrate (e.g., slide 104). A reagent medium 105 within a gap between the first substrate (e.g., slide 103) and the second substrate (e.g., slide 104) creates a liquid interface between the two substrates. The reagent medium may be a permeabilization solution which permeabilizes and/or digests the biological sample 102. In some embodiments wherein the biological sample 102 has been pre-permeabilized, the reagent medium is not a permeabilization solution. In some embodiments, analytes (e.g., mRNA transcripts) and/or analyte derivatives (e.g., intermediate agents; e.g., ligation products) of the biological sample 102 may release from the biological sample, and actively or passively migrate (e.g., diffuse) across the gap toward the capture probes on the array 106. Alternatively, in certain embodiments, migration of the analyte or analyte derivative (e.g., intermediate agent; e.g., ligation product) from the biological sample is performed actively (e.g., electrophoretic, by applying an electric field to promote migration). Exemplary methods of electrophoretic migration are described in WO 2020/176788, and US. Patent Application Pub. No. 2021/0189475.

As further shown, one or more spacers 110 may be positioned between the first substrate (e.g., slide 103) and the second substrate (e.g., array slide 104 including spatially barcoded capture probes 106). The one or more spacers 110 may be configured to maintain a separation distance between the first substrate and the second substrate. While the one or more spacers 110 is shown as disposed on the second substrate, the spacer may additionally or alternatively be disposed on the first substrate.

In some embodiments, the one or more spacers 110 is configured to maintain a separation distance between first and second substrates that is between about 2 microns and 1 mm (e.g., between about 2 microns and 800 microns, between about 2 microns and 700 microns, between about 2 microns and 600 microns, between about 2 microns and 500 microns, between about 2 microns and 400 microns, between about 2 microns and 300 microns, between about 2 microns and 200 microns, between about 2 microns and 100 microns, between about 2 microns and 25 microns, or between about 2 microns and 10 microns), measured in a direction orthogonal to the surface of first substrate that supports the biological sample. In some instances, the separation distance is about 2, 3, 4, 5, 6, 7, 8, 9, 10, 11, 12, 13, 14, 15, 16, 17, 18, 19, 20, 21, 22, 23, 24, or 25 microns. In some embodiments, the separation distance is less than 50 microns. In some embodiments, the separation distance is less than 25 microns. In some embodiments, the separation distance is less than 20 microns. The separation distance may include a distance of at least 2 µm.

FIG. 1B shows a fully formed sandwich configuration 125 creating a chamber 150 formed from the one or more spacers 110, the first substrate (e.g., the slide 103), and the second substrate (e.g., the slide 104 including an array 106 having spatially barcoded capture probes) in accordance with some example implementations. In the example of FIG. 1B, the liquid reagent (e.g., the permeabilization solution 105) fills the volume of the chamber 150 and may create a permeabilization buffer that allows analytes (e.g., mRNA transcripts and/or other molecules) or analyte derivatives (e.g., intermediate agents; e.g., ligation products) to diffuse from the biological sample 102 toward the capture probes of the second substrate (e.g., slide 104). In some aspects, flow of the permeabilization buffer may deflect transcripts and/or molecules from the biological sample 102 and may affect diffusive transfer of analytes or analyte derivatives (e.g., intermediate agents; e.g., ligation products) for spatial analysis. A partially or fully sealed chamber 150 resulting from the one or more spacers 110, the first substrate, and the second substrate may reduce or prevent flow from undesirable convective movement of transcripts and/or molecules over the diffusive transfer from the biological sample 102 to the capture probes.

The sandwiching process methods described above can be implemented using a variety of hardware components. For example, the sandwiching process methods can be implemented using a sample holder (also referred to herein as a support device, a sample handling apparatus, and an array alignment device). Further details on support devices, sample holders, sample handling apparatuses, or systems for implementing a sandwiching process are described in, e.g., US. Patent Application Pub. No. 2021/0189475, and PCT Publ. No. WO 2022/061152 A2.

In some embodiments of a sample holder, the sample holder can include a first member including a first retaining mechanism configured to retain a first substrate comprising a biological sample. The first retaining mechanism can be configured to retain the first substrate disposed in a first plane. The sample holder can further include a second member including a second retaining mechanism configured to retain a second substrate disposed in a second plane. The sample holder can further include an alignment mechanism connected to one or both of the first member and the second member. The alignment mechanism can be configured to align the first and second members along the first plane and/or the second plane such that the sample contacts at least a portion of the reagent medium when the first and second members are aligned and within a threshold distance along an axis orthogonal to the second plane. The adjustment mechanism may be configured to move the second member along the axis orthogonal to the second plane and/or move the first member along an axis orthogonal to the first plane.

In some embodiments, the adjustment mechanism includes a linear actuator. In some embodiments, the linear actuator is configured to move the second member along an axis orthogonal to the plane of the first member and/or the second member. In some embodiments, the linear actuator is configured to move the first member along an axis orthogonal to the plane of the first member and/or the second member. In some embodiments, the linear actuator is configured to move the first member, the second member, or both the first member and the second member at a velocity of at least 0.1 mm/sec. In some embodiments, the linear actuator is configured to move the first member, the second member, or both the first member and the second member with an amount of force of at least 0.1 lbs.

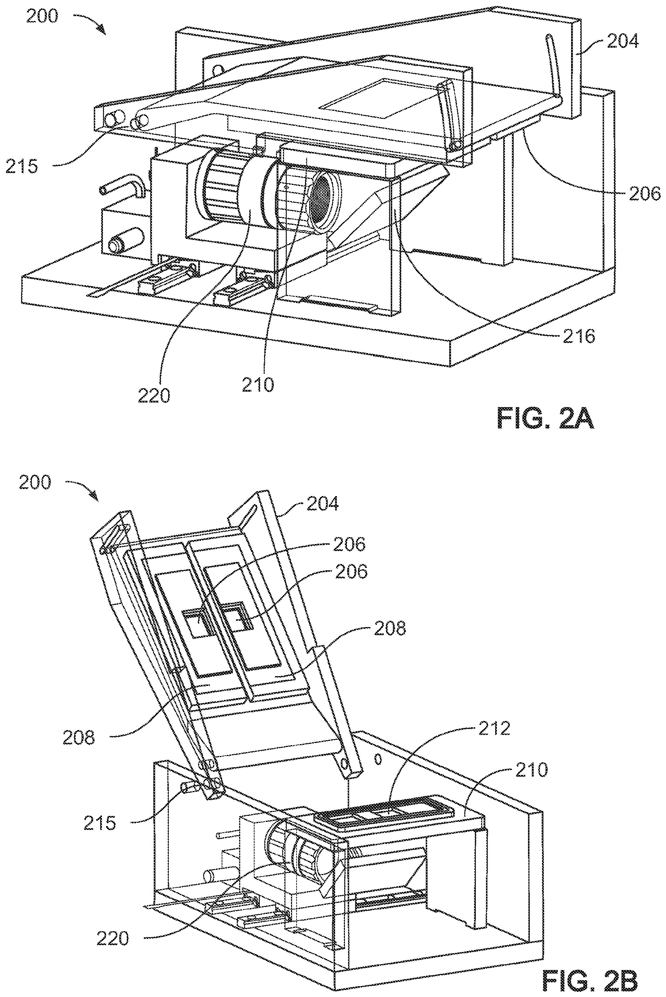

FIG. 2A is a perspective view of an example sample handling apparatus 200 in a closed position in accordance with some example implementations. As shown, the sample handling apparatus **200** includes a first member **204,** a second member **210,** optionally an image capture device **220,** a first substrate **206,** optionally a hinge **215,** and optionally a mirror **216.** The hinge **215** may be configured to allow the first member **204** to be positioned in an open or closed configuration by opening and/or closing the first member **204** in a clamshell manner along the hinge **215.**

**FIG. 2B** is a perspective view of the example sample handling apparatus **200** in an open position in accordance with some example implementations. As shown, the sample handling apparatus **200** includes one or more first retaining mechanisms **208** configured to retain one or more first substrates 206. In the example of **FIG. 2B****,** the first member **204** is configured to retain two first substrates **206,** however the first member **204** may be configured to retain more or fewer first substrates **206.**

In some aspects, when the sample handling apparatus **200** is in an open position (e.g., in **FIG. 2B****), the** first substrate **206** and/or the second substrate **212** may be loaded and positioned within the sample handling apparatus **200** such as within the first member **204** and the second member **210,** respectively. As noted, the hinge **215** may allow the first member **204** to close over the second member **210** and form a sandwich configuration.

In some aspects, after the first member **204** closes over the second member **210,** an adjustment mechanism of the sample handling apparatus **200** may actuate the first member **204** and/or the second member **210** to form the sandwich configuration for the permeabilization step (e.g., bringing the first substrate **206** and the second substrate **212** closer to each other and within a threshold distance for the sandwich configuration). The adjustment mechanism may be configured to control a speed, an angle, a force, or the like of the sandwich configuration.

In some embodiments, the biological sample (e.g., sample 102 from FIG. 1A) may be aligned within the first member 204 (e.g., via the first retaining mechanism 208) prior to closing the first member 204 such that a desired region of interest of the sample is aligned with the barcoded array of the second substrate (e.g., the slide 104 from FIG. 1A), e.g., when the first and second substrates are aligned in the sandwich configuration. Such alignment may be accomplished manually (e.g., by a user) or automatically (e.g., via an automated alignment mechanism). After or before alignment, spacers may be applied to the first substrate 206 and/or the second substrate 212 to maintain a minimum spacing between the first substrate 206 and the second substrate 212 during sandwiching. In some aspects, the permeabilization solution (e.g., permeabilization solution 305) may be applied to the first substrate 206 and/or the second substrate **212.** The first member **204** may then close over the second member 210 and form the sandwich configuration. Analytes or analyte derivatives (e.g., intermediate agents; e.g., ligation products) may be captured by the capture probes of the array and may be processed for spatial analysis.

In some embodiments, during the permeabilization step, the image capture device 220 may capture images of the overlap area between the biological sample and the capture probes on the array 106. If more than one first substrates 206 and/or second substrates 212 are present within the sample handling apparatus 200, the image capture device 220 may be configured to capture one or more images of one or more overlap areas.

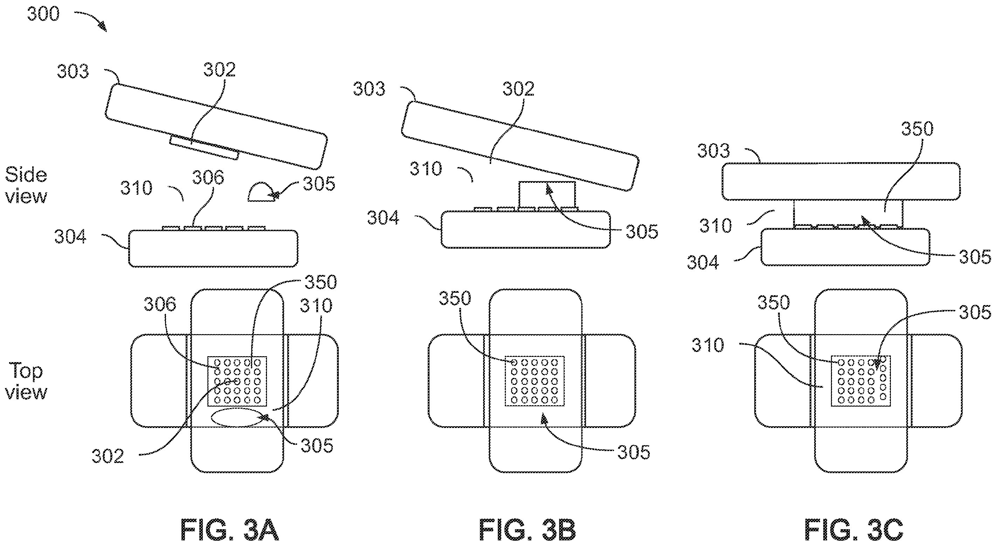

Provided herein are methods for delivering a fluid to a biological sample disposed on an area of a first substrate and an array disposed on a second substrate. **FIGs. 3A-3C** depict a side view and a top view of an exemplary angled closure workflow **300** for sandwiching a first substrate (e.g., slide **303)** having a biological sample **302** and a second substrate (e.g., slide **304** having capture probes **306)** in accordance with some exemplary implementations.

**FIG. 3A** depicts the first substrate (e.g., the slide **303** including a biological sample **302)** angled over (superior to) the second substrate (e.g., slide **304).** As shown, reagent medium (e.g., permeabilization solution) **305** is located on the spacer **310** toward the righthand side of the side view in **FIG. 3A****.** While **FIG. 3A** depicts the reagent medium on the right hand side of side view, it should be understood that such depiction is not meant to be limiting as to the location of the reagent medium on the spacer.

**FIG. 3B** shows that as the first substrate lowers, and/or as the second substrate rises, the dropped side of the first substrate (e.g., a side of the slide **303** angled toward the second substrate) may contact the reagent medium **305.** The dropped side of the first substrate may urge the reagent medium **305** toward the opposite direction (e.g., towards an opposite side of the spacer **310,** towards an opposite side of the first substrate relative to the dropped side). For example, in the side view of **FIG. 3B** the reagent medium **305** may be urged from right to left as the sandwich is formed.

In some embodiments, the first substrate and/or the second substrate are further moved to achieve an approximately parallel arrangement of the first substrate and the second substrate.

**FIG. 3C** depicts a full closure of the sandwich between the first substrate and the second substrate with the spacer **310** contacting both the first substrate and the second substrate and maintaining a separation distance and optionally the approximately parallel arrangement between the two substrates. As shown in the top view of **FIG. 3C****,** the spacer **310** fully encloses and surrounds the biological sample **302** and the capture probes **306,** and the spacer **310** forms the sides of chamber **350** which holds a volume of the reagent medium **305.**

While **FIG. 3C** depicts the first substrate (e.g., the slide **303** including biological sample **302)** angled over (superior to) the second substrate (e.g., slide **304)** and the second substrate comprising the spacer **310,** it should be understood that an exemplary angled closure workflow can include the second substrate angled over (superior to) the first substrate and the first substrate comprising the spacer **310.**

It may be desirable that the reagent medium be free from air bubbles between the substrates to facilitate transfer of target analytes with spatial information. Additionally, air bubbles present between the substrates may obscure at least a portion of an image capture of a desired region of interest. Accordingly, it may be desirable to ensure or encourage suppression and/or elimination of air bubbles between the two substrates (e.g., slide **303** and slide **304)** during a permeabilization step (e.g., step **104).** In some aspects, it may be possible to reduce or eliminate bubble formation between the substrates using a variety of filling methods and/or closing methods. In some instances, the first substrate and the second substrate are arranged in an angled sandwich assembly as described herein. For example, during the sandwiching of the two substrates (e.g., the slide **303** and the slide **304),** an angled closure workflow may be used to suppress or eliminate bubble formation.

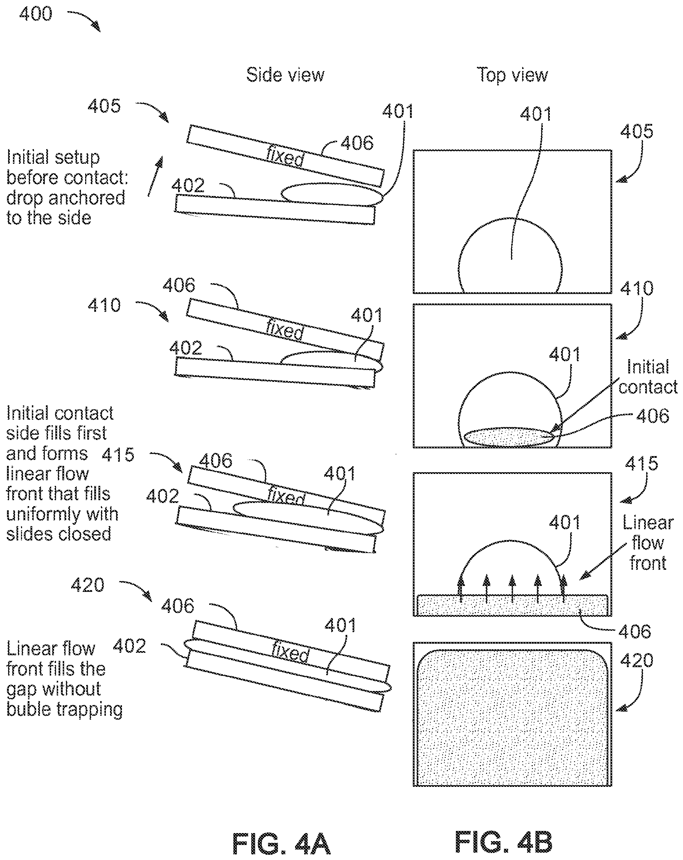

**FIG. 4A** is a side view of the angled closure workflow **400** in accordance with some exemplary implementations. **FIG. 4B** is a top view of the angled closure workflow **400** in accordance with some exemplary implementations. As shown at **405,** reagent medium **401** is positioned to the side of the substrate **402.**

At step **410,** the dropped side of the angled substrate **406** contacts the reagent medium **401** first. The contact of the substrate **406** with the reagent medium **401** may form a linear or low curvature flow front that fills uniformly with the slides closed.

At step **415,** the substrate **406** is further lowered toward the substrate **402** (or the substrate **402** is raised up toward the substrate **406)** and the dropped side of the substrate **406** may contact and may urge the reagent medium toward the side opposite the dropped side and creating a linear or low curvature flow front that may prevent or reduce bubble trapping between the substrates.

At step **420,** the reagent medium **401** fills the gap between the substrate **406** and the substrate **402.** The linear flow front of the liquid reagent may form by squeezing the **401** volume along the contact side of the substrate **402** and/or the substrate **406.** Additionally, capillary flow may also contribute to filling the gap area.

In some embodiments, the reagent medium (e.g., 105 in FIG. 1A) comprises a permeabilization agent. In some embodiments, following initial contact between the biological sample and a permeabilization agent, the permeabilization agent can be removed from contact with the biological sample (e.g., by opening the sample holder). Suitable agents for this purpose include, but are not limited to, organic solvents (e.g., acetone, ethanol, and methanol), cross-linking agents (e.g., paraformaldehyde), detergents (e.g., saponin, Triton X-100^{™}, Tween-20^{™}, or sodium dodecyl sulfate (SDS)), and enzymes (e.g., trypsin, proteases (e.g., proteinase K). In some embodiments, the detergent is an anionic detergent (e.g., SDS or N-lauroylsarcosine sodium salt solution).

In some embodiments, the reagent medium comprises a lysis reagent. Lysis solutions can include ionic surfactants such as, for example, sarkosyl and sodium dodecyl sulfate (SDS). More generally, chemical lysis agents can include, without limitation, organic solvents, chelating agents, detergents, surfactants, and chaotropic agents. In some embodiments, the reagent medium comprises a protease. Exemplary proteases include, e.g., pepsin, trypsin, elastase, and proteinase K. In some embodiments, the reagent medium comprises a nuclease. In some embodiments, the nuclease comprises an RNase. In some embodiments, the RNase is selected from RNase A, RNase C, RNase H, and RNase I. In some embodiments, the reagent medium comprises one or more of sodium dodecyl sulfate (SDS) or a sodium salt thereof, proteinase K, pepsin, N-lauroylsarcosine, and RNase.

In some embodiments, the reagent medium comprises polyethylene glycol (PEG). In some embodiments, the PEG is from about PEG 2K to about PEG 16K. In some embodiments, the PEG is PEG 2K, 3K, 4K, 5K, 6K, 7K, 8K, 9K, 10K, 11K, 12K, 13K, 14K, 15K, or 16K. In some embodiments, the PEG is present at a concentration from about 2% to 25%, from about 4% to about 23%, from about 6% to about 21%, or from about 8% to about 20% (v/v).

In certain embodiments a dried permeabilization reagent is applied or formed as a layer on the first substrate or the second substrate or both prior to contacting the biological sample with the array. For example, a permeabilization reagent can be deposited in solution on the first substrate or the second substrate or both and then dried.

In some instances, the aligned portions of the biological sample and the array are in contact with the reagent medium for about 1 minute, about 5 minutes, about 10 minutes, about 12 minutes, about 15 minutes, about 18 minutes, about 20 minutes, about 25 minutes, about 30 minutes, about 36 minutes, about 45 minutes, or about an hour. In some instances, the aligned portions of the biological sample and the array are in contact with the reagent medium for about 1-60 minutes.

In some instances, the device is configured to control a temperature of the first and second substrates. In some embodiments, the temperature of the first and second members is lowered to a first temperature that is below room temperature.

There are at least two methods to associate a spatial barcode with one or more neighboring cells, such that the spatial barcode identifies the one or more cells, and/or contents of the one or more cells, as associated with a particular spatial location. One method is to promote analytes or analyte proxies (e.g., intermediate agents) out of a cell and towards a spatially-barcoded array (e.g., including spatially-barcoded capture probes). Another method is to cleave spatially-barcoded capture probes from an array and promote the spatially-barcoded capture probes towards and/or into or onto the biological sample.

In some cases, capture probes may be configured to prime, replicate, and consequently yield optionally barcoded extension products from a template (e.g., a DNA or RNA template, such as an analyte or an intermediate agent (e.g., a ligation product or an analyte capture agent), or a portion thereof), or derivatives thereof (see, e.g., Section (II)(b)(vii) of PCT Publication No. WO2020/176788 and/or U.S. Patent Application Publication No. 2020/0277663 regarding extended capture probes). In some cases, capture probes may be configured to form ligation products with a template (e.g., a DNA or RNA template, such as an analyte or an intermediate agent, or portion thereof), thereby creating ligation products that can serve as proxies for a template.

As used herein, an "extended capture probe" refers to a capture probe having additional nucleotides added to the terminus (e.g., 3' or 5' end) of the capture probe thereby extending the overall length of the capture probe. For example, an "extended 3' end" indicates additional nucleotides were added to the most 3' nucleotide of the capture probe to extend the length of the capture probe, for example, by polymerization reactions used to extend nucleic acid molecules including templated polymerization catalyzed by a polymerase (e.g., a DNA polymerase or a reverse transcriptase). In some embodiments, extending the capture probe includes adding to a 3' end of a capture probe a nucleic acid sequence that is complementary to a nucleic acid sequence of an analyte or intermediate agent specifically bound to the capture domain of the capture probe. In some embodiments, the capture probe is extended using reverse transcription. In some embodiments, the capture probe is extended using one or more DNA polymerases. The extended capture probes can include the sequence of the capture domain, the sequence of the spatial barcode of the capture probe, and the complementary sequence of the template used for extension of the capture probe.

In some embodiments, extended capture probes are amplified (e.g., in bulk solution or on the array) to yield quantities that are sufficient for downstream analysis, e.g., via sequencing. In some embodiments, extended capture probes (e.g., DNA molecules) can act as templates for an amplification reaction (e.g., a polymerase chain reaction).

Additional variants of spatial analysis methods, including in some embodiments, an imaging step, are described in Section (II)(a) of PCT Publication No. WO2020/176788 and/or U.S. Patent Application Publication No. 2020/0277663. Analysis of captured analytes (and/or intermediate agents or portions thereof), for example, including sample removal, extension of capture probes using the captured analyte or an intermediate agent as a template, sequencing (e.g., of a cleaved extended capture probe and/or a cDNA molecule complementary to an extended capture probe), sequencing on the array (e.g., using, for example, in situ hybridization or in situ ligation approaches), temporal analysis, and/or proximity capture, is described in Section (II)(g) of PCT Publication No. WO2020/176788 and/or U.S. Patent Application Publication No. 2020/0277663. Some quality control measures are described in Section (II)(h) of PCT Publication No. WO2020/176788 and/or U.S. Patent Application Publication No. 2020/0277663.

Spatial information can provide information of biological and/or medical importance. For example, the methods and compositions described herein can allow for: identification of one or more biomarkers (e.g., diagnostic, prognostic, and/or for determination of efficacy of a treatment) of a disease or disorder; identification of a candidate drug target for treatment of a disease or disorder; identification (e.g., diagnosis) of a subject as having a disease or disorder; identification of stage and/or prognosis of a disease or disorder in a subject; identification of a subject as having an increased likelihood of developing a disease or disorder; monitoring of progression of a disease or disorder in a subject; determination of efficacy of a treatment of a disease or disorder in a subject; identification of a patient subpopulation for which a treatment is effective for a disease or disorder; modification of a treatment of a subject with a disease or disorder; selection of a subject for participation in a clinical trial; and/or selection of a treatment for a subject with a disease or disorder. Exemplary methods for identifying spatial information of biological and/or medical importance can be found in U.S. Patent Application Publication Nos. 2021/0140982, 2021/0198741, and/or 2021/0199660.

Spatial information can provide information of biological importance. For example, the methods and compositions described herein can allow for: identification of transcriptome and/or proteome expression profiles (e.g., in healthy and/or diseased tissue); identification of multiple analyte types in close proximity (e.g., nearest neighbor or proximity based analysis); determination of up- and/or down-regulated genes and/or proteins in diseased tissue; characterization of tumor microenvironments; characterization of tumor immune responses; characterization of cells types and their co-localization in healthy and diseased tissue; and identification of genetic variants within tissues (e.g., based on gene and/or protein expression profiles associated with specific disease or disorder biomarkers).

Typically, for spatial array-based methods, a substrate functions as a support for direct or indirect attachment of capture probes to features of the array. A "feature" is an entity that acts as a support or repository for various molecular entities used in spatial analysis. In some embodiments, some or all of the features in an array are functionalized for analyte capture. Exemplary substrates are described in Section (II)(c) of PCT Publication No. WO2020/176788 and/or U.S. Patent Application Publication No. 2020/0277663. Exemplary features and geometric attributes of an array can be found in Sections (II)(d)(i), (II)(d)(iii), and (II)(d)(iv) of PCT Publication No. WO2020/176788 and/or U.S. Patent Application Publication No. 2020/0277663.

Generally, analytes and/or intermediate agents (or portions thereof) can be captured when contacting a biological sample with a substrate including capture probes (e.g., a substrate with capture probes embedded, spotted, printed, fabricated on the substrate, or a substrate with features (e.g., beads, wells) comprising capture probes). As used herein, "contact," "contacted," and/or "contacting," a biological sample with a substrate refers to any contact (e.g., direct or indirect) such that capture probes can interact (e.g., bind covalently or non-covalently (e.g., hybridize)) with analytes from the biological sample. Capture can be achieved actively (e.g., using electrophoresis) or passively (e.g., using diffusion). Analyte capture is further described in Section (II)(e) of PCT Publication No. WO2020/176788 and/or U.S. Patent Application Publication No. 2020/0277663.

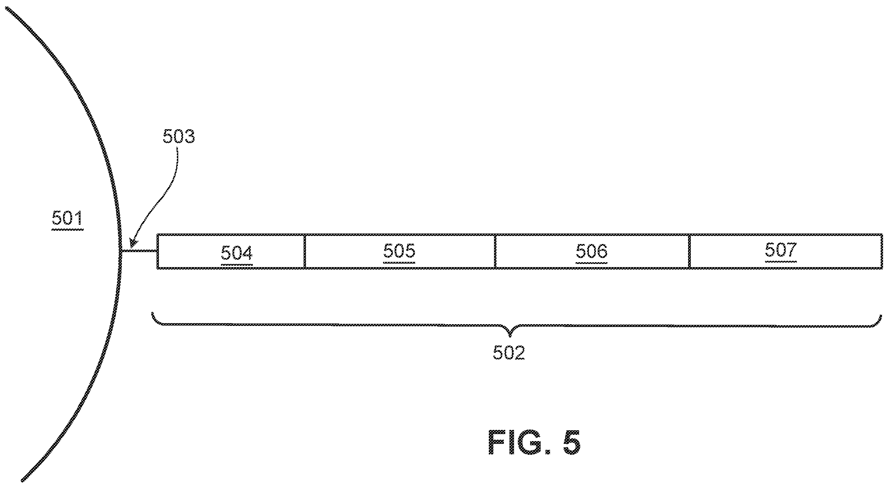

**FIG. 5** is a schematic diagram showing an exemplary capture probe, as described herein. As shown, the capture probe **502** is optionally coupled to a feature 501 by a cleavage domain **503,** such as a disulfide linker. The capture probe can include a **functional** sequence **504** thatis useful for subsequent processing. The functional sequence 504 can include all or a part of sequencer specific flow cell attachment sequence (e.g., a P5 or P7 sequence), all or a part of a sequencing primer sequence, (e.g., a R]1 primer binding site, a R2 primer binding site), or combinations thereof. The capture probe can also include a spatial barcode ]}505. The capture probe can also include a unique molecular identifier (UMI) sequence 506. While **FIG. 5** shows the spatial barcode **505** as being located upstream (5') of UMI sequence **506,** it is to be understood that capture probes wherein UMI sequence **506** is located upstream (5') of the spatial barcode **505** is also suitable for use in any of the methods described herein. The capture probe can also include a capture domain 507 to facilitate capture of a target analyte. The capture domain can have a sequence complementary to a sequence of a nucleic acid analyte. The capture domain can have a sequence complementary to a connected probe described herein. The capture domain can have a sequence complementary to a capture handle sequence (or analyte capture sequence) present in an analyte capture agent. The capture domain can have a sequence complementary to a splint oligonucleotide. A splint oligonucleotide, in addition to having a sequence complementary to a capture domain of a capture probe, can have a sequence complementary to a sequence of a nucleic acid analyte, a portion of a connected probe described herein, a capture handle sequence described herein, and/or a methylated adaptor described herein.

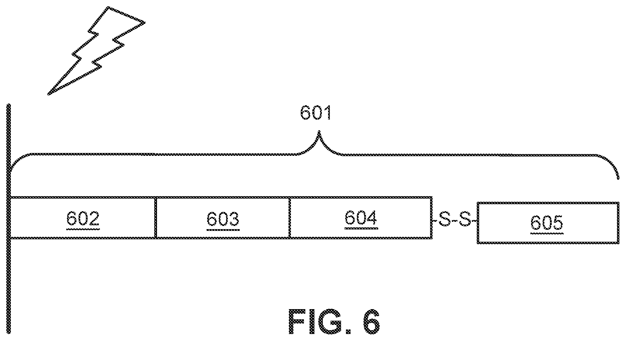

FIG. 6 is a schematic illustrating a cleavable capture probe, wherein the cleaved capture probe can enter into a cell and bind to analytes within the sample. The capture probe 601 contains a cleavage domain 602, a cell penetrating peptide 603, a reporter molecule 604, and a disulfide bond (-S-S-). 605 represents all other parts of a capture probe, for example a spatial barcode and a capture domain.

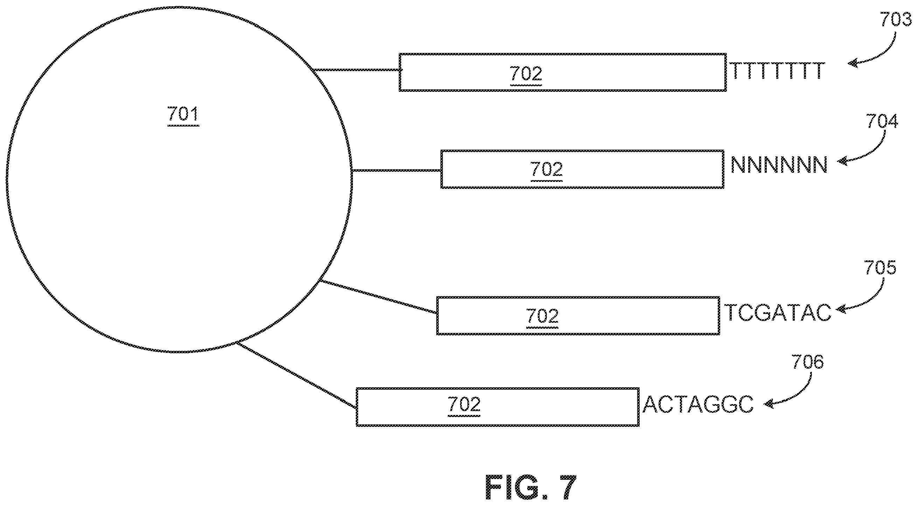

FIG. 7 is a schematic diagram of an exemplary multiplexed spatially-barcoded feature. In FIG. 7, the feature 701 can be coupled to spatially-barcoded capture probes, wherein the spatially-barcoded probes of a particular feature can possess the same spatial barcode but have different capture domains designed to associate the spatial barcode of the feature with more than one target analyte. For example, a feature may include four different types of spatially-barcoded capture probes, each type of spatially-barcoded capture probe possessing the spatial barcode 702. One type of capture probe associated with the feature includes the spatial barcode 702 in combination with a poly(T) capture domain 703, designed to capture mRNA target analytes. A second type of capture probe associated with the feature includes the spatial barcode 702 in combination with a random N-mer capture domain 704 for gDNA analysis. A third type of capture probe associated with the feature includes the spatial barcode 702 in combination with a capture domain complementary to the analyte capture agent of interest 705. A fourth type of capture probe associated with the feature includes the spatial barcode 702 in combination with a capture probe that can specifically bind a nucleic acid molecule 706 that can function in a CRISPR assay (e.g., CRISPR/Cas9). While only four different capture probe-barcoded constructs are shown in FIG. 7, capture-probe barcoded constructs can be tailored for analyses of any given analyte associated with a nucleic acid and capable of binding with such a construct. For example, the schemes shown in FIG. 7 can also be used for concurrent analysis of other analytes disclosed herein, including, but not limited to: (a) mRNA, a lineage tracing construct, cell surface or intracellular proteins and metabolites, and gDNA; (b) mRNA, accessible chromatin (e.g., ATAC-seq, DNase-seq, and/or MNase-seq) cell surface or intracellular proteins and metabolites, and a perturbation agent (e.g., a CRISPR crRNA/sgRNA, TALEN, zinc finger nuclease, and/or antisense oligonucleotide as described herein); (c) mRNA, cell surface or intracellular proteins and/or metabolites, a barcoded labelling agent (e.g., the MHC multimers described herein), and a V(D)J sequence of an immune cell receptor (e.g., T-cell receptor). In some embodiments, a perturbation agent can be a small molecule, an antibody, a drug, an aptamer, a miRNA, a physical environmental (e.g., temperature change), or any other known perturbation agents. See, e.g., Section (II)(b) (e.g., subsections (i)-(vi)) of WO 2020/176788 and/or U.S. Patent Application Publication No. 2020/0277663. Generation of capture probes can be achieved by any appropriate method, including those described in Section (II)(d)(ii) of WO 2020/176788 and/or U.S. Patent Application Publication No. 2020/0277663.

The functional sequences can generally be selected for compatibility with any of a variety of different sequencing systems, e.g., Ion Torrent Proton or PGM, Illumina sequencing instruments, PacBio, Oxford Nanopore, etc., and the requirements thereof. In some embodiments, functional sequences can be selected for compatibility with non-commercialized sequencing systems. Examples of such sequencing systems and techniques, for which suitable functional sequences can be used, include (but are not limited to) Ion Torrent Proton or PGM sequencing, Illumina sequencing, PacBio SMRT sequencing, and Oxford Nanopore sequencing. Further, in some embodiments, functional sequences can be selected for compatibility with other sequencing systems, including non-commercialized sequencing systems.

In some embodiments, the spatial barcode 505 and functional sequences 504 are common to all of the probes attached to a given feature. In some embodiments, the UMI sequence 506 of a capture probe attached to a given feature is different from the UMI sequence of a different capture probe attached to the given feature.

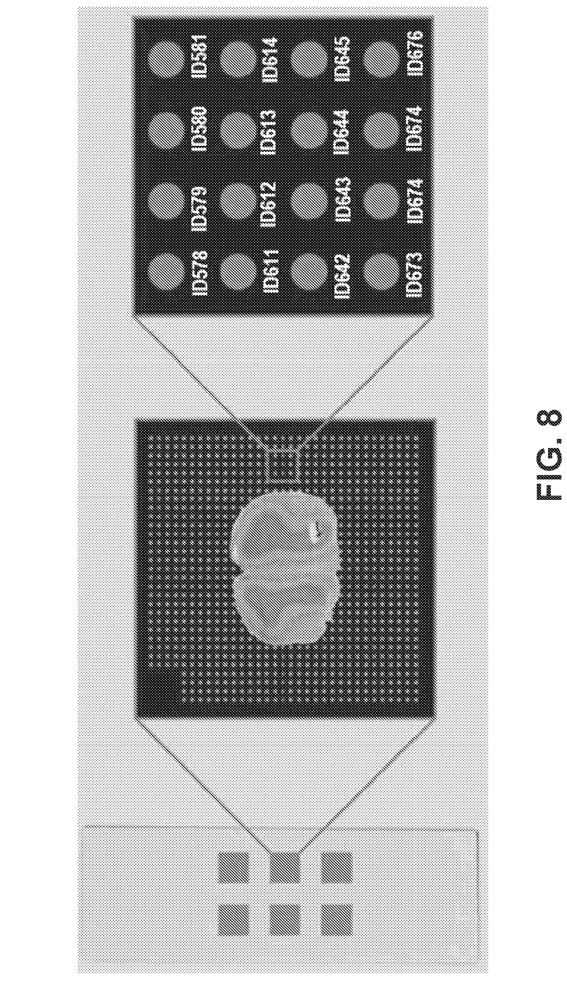

FIG. 8 depicts an exemplary arrangement of barcoded features within an array. From left to right, FIG. 8 shows (L) a slide including six spatially-barcoded arrays, (C) an enlarged schematic of one of the six spatially-barcoded arrays, showing a grid of barcoded features in relation to a biological sample, and (R) an enlarged schematic of one section of an array, showing the specific identification of multiple features within the array (labelled as ID578, ID579, ID560, etc.).

In some embodiments, more than one analyte type (e.g., nucleic acids and proteins) from a biological sample can be detected (e.g., simultaneously or sequentially) using any appropriate multiplexing technique, such as those described in Section (IV) of PCT Publication No. WO2020/176788 and/or U.S. Patent Application Publication No. 2020/0277663.

In some cases, spatial analysis can be performed by attaching and/or introducing a molecule (e.g., a peptide, a lipid, or a nucleic acid molecule) having a barcode (e.g., a spatial barcode) to a biological sample (e.g., to a cell in a biological sample). In some embodiments, a plurality of molecules (e.g., a plurality of nucleic acid molecules) having a plurality of barcodes (e.g., a plurality of spatial barcodes) are introduced to a biological sample (e.g., to a plurality of cells in a biological sample) for use in spatial analysis. In some embodiments, after attaching and/or introducing a molecule having a barcode to a biological sample, the biological sample can be physically separated (e.g., dissociated) into single cells or cell groups for analysis. Some such methods of spatial analysis are described in Section (III) of PCT Publication No. WO2020/176788 and/or U.S. Patent Application Publication No. 2020/0277663.

In some cases, spatial analysis can be performed by detecting multiple oligonucleotides that hybridize to an analyte. In some instances, for example, spatial analysis can be performed using RNA-templated ligation (RTL). Methods of RTL have been described previously. See, e.g., Credle et al., Nucleic Acids Res. 2017 Aug 21; 45(14):e128. Typically, RTL includes hybridization of two oligonucleotides to adjacent sequences on an analyte (e.g., an RNA molecule, such as an mRNA molecule). In some instances, the oligonucleotides are DNA molecules. In some instances, one of the oligonucleotides includes at least two ribonucleic acid bases at the 3' end and/or the other oligonucleotide includes a phosphorylated nucleotide at the 5' end. In some instances, one of the two oligonucleotides includes a capture probe binding domain (e.g., a poly(A) sequence, a non-homopolymeric sequence). After hybridization to the analyte, a ligase (e.g., a T4 RNA ligase (Rnl2), a PBCV-1 DNA ligase, a *Chlorella* virus DNA ligase, a single-stranded DNA ligase, or a T4 DNA ligase) ligates the two oligonucleotides together, creating a ligation product. In some instances, the two oligonucleotides hybridize to sequences that are not adjacent to one another. For example, hybridization of the two oligonucleotides creates a gap between the hybridized oligonucleotides. In some instances, a polymerase (e.g., a DNA polymerase) can extend one of the oligonucleotides prior to ligation. After ligation, the ligation product is released from the analyte. In some instances, the ligation product is released using an endonuclease (e.g., RNase H). In some instances, the ligation product is removed using heat. In some instances, the ligation product is removed using KOH. The released ligation product can then be captured by capture probes (e.g., instead of direct capture of an analyte) on an array, optionally amplified, and sequenced, thus determining the location and optionally the abundance of the analyte in the biological sample.

In some instances, one or both of the oligonucleotides may hybridize to genomic DNA (gDNA) which can lead to false positive sequencing data from ligation events on gDNA (off target) in addition to the desired (on target) ligation events on target nucleic acids, (e.g., mRNA). Thus, in some embodiments, the disclosed methods can include contacting the biological sample with a deoxyribonuclease (DNase). The DNase can be an endonuclease or exonuclease. In some embodiments, the DNase digests single- and/or double-stranded DNA. Suitable DNases include, without limitation, a DNase I and a DNase II. Use of a DNase as described can mitigate false positive sequencing data from off target gDNA ligation events.

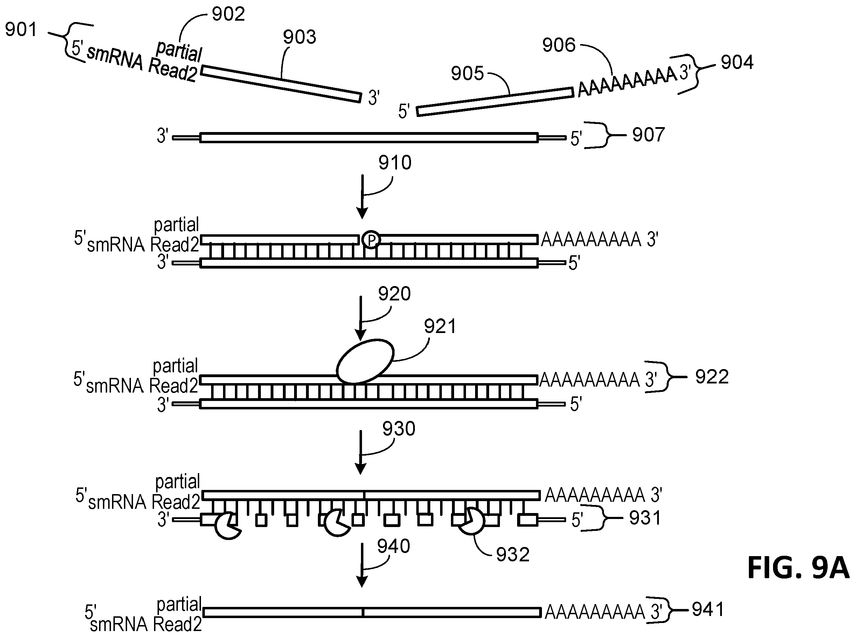

A non-limiting example of templated ligation methods disclosed herein is depicted in **FIG. 9A****.** After a biological sample is contacted with a substrate including a plurality of capture probes and contacted with (a) a first probe **901** having a target-hybridization sequence **903** and a primer sequence **902** and (b) a second probe **904** having a target-hybridization sequence **905** and a capture domain (e.g., a poly-A sequence) **906,** the first probe **901** and a second probe **904** hybridize **910** to an analyte **907.** A ligase **921** ligates **920** the first probe to the second probe thereby generating a ligation product **922.** The ligation product is released **930** from the analyte **931** by digesting the analyte using an endoribonuclease **932.** The sample is permeabilized **940** and the ligation product **941** is able to hybridize to a capture probe on the substrate. Methods and composition for spatial detection using templated ligation have been described in PCT Publ. No. WO 2021/133849 A1, U.S. Pat. Nos. 11,332,790 and 11,505,828.

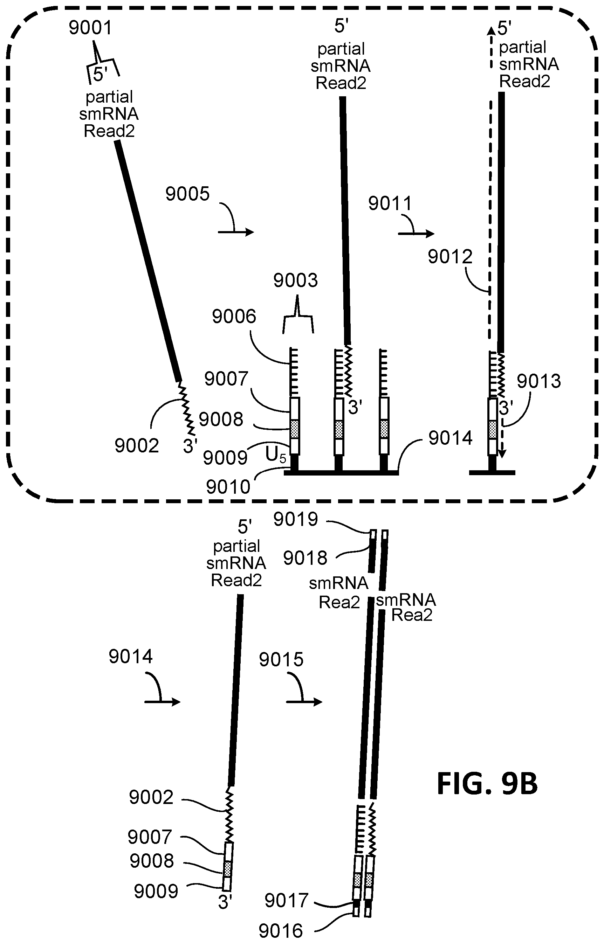

In some embodiments, as shown in **FIG. 9B****,** the ligation product **9001** includes a capture probe capture domain **9002,** which can bind to a capture probe **9003** (e.g., a capture probe immobilized, directly or indirectly, on a substrate **9004).** In some embodiments, methods provided herein include contacting **9005** a biological sample with a substrate **9004,** wherein the capture probe **9003** is affixed to the substrate (e.g., immobilized to the substrate, directly or indirectly). In some embodiments, the capture probe capture domain **9002** of the ligation product specifically binds to the capture domain 9006. The capture probe can also include a unique molecular identifier (UMI) 9007, a spatial barcode **9008,** a functional sequence **9009,** and a cleavage domain 9010.

In some embodiments, methods provided herein include permeabilization of the biological sample such that the capture probe can more easily capture the ligation products (i.e., compared to no permeabilization). In some embodiments, reverse transcription (RT) reagents can be added to permeabilized biological samples. Incubation with the RT reagents can extend the capture probes **9011** to produce spatially-barcoded full-length cDNA **9012** and **9013** from the captured ligation products.

In some embodiments, the extended ligation products can be denatured **9014** from the capture probe and transferred (e.g., to a clean tube) for amplification, and/or library construction. The spatially-barcoded ligation products can be amplified **9015** via PCR prior to library construction. P5 **9016** and P7 **9019** can be used as sequences that are complementary to sequencing probes for immobilization of the library on the sequencing flow cell and i5 **9017** and i7 **9018** can be used as sample indexes. The amplicons can then be sequenced using paired-end sequencing using TruSeq Read 1 and TruSeq Read 2 as sequencing primer sites.

In some embodiments, detection of one or more analytes (e.g., protein analytes) can be performed using one or more analyte capture agents. As used herein, an "analyte capture agent" refers to an agent that interacts with an analyte (e.g., an analyte in a biological sample) and with a capture probe (e.g., a capture probe attached to a substrate or a feature) to identify the analyte. In some embodiments, the analyte capture agent includes: (i) an analyte binding moiety (e.g., that binds to an analyte), for example, an antibody or antigen-binding fragment thereof; (ii) analyte binding moiety barcode; and (iii) an analyte capture sequence. As used herein, the term "analyte binding moiety barcode" refers to a barcode that is associated with or otherwise identifies the analyte binding moiety. As used herein, the term "analyte capture sequence" refers to a region or moiety configured to hybridize to, bind to, couple to, or otherwise interact with a capture domain of a capture probe. In some cases, an analyte binding moiety barcode (or portion thereof) may be able to be removed (e.g., cleaved) from the analyte capture agent. Additional description of analyte capture agents can be found in Section (II)(b)(ix) of PCT Publication No. WO2020/176788 and/or Section (II)(b)(viii) U.S. Patent Application Publication No. 2020/0277663.

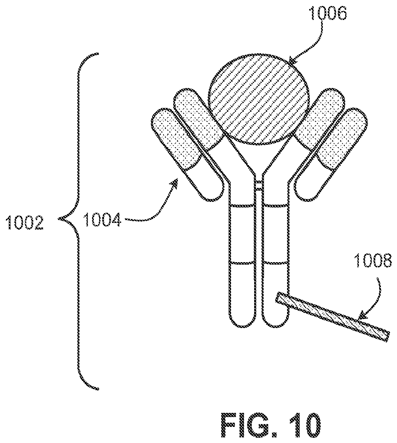

**FIG. 10** is a schematic diagram of an exemplary analyte capture agent **1002** comprised of an analyte-binding moiety **1004** and an analyte-binding moiety barcode domain 1008. The exemplary analyte-binding moiety **1004** is a molecule capable of binding to an analyte **1006** and the analyte capture agent is capable of interacting with a spatially-barcoded capture probe. The analyte-binding moiety can bind to the analyte **1006** with high affinity and/or with high specificity. The analyte capture agent can include an analyte-binding moiety barcode domain **1008** which serves to identify the analyte binding moiety, and a capture domain which can hybridize to at least a portion or an entirety of a capture domain of a capture probe. The analyte-binding moiety **1004** can include a polypeptide and/or an aptamer. The analyte-binding moiety **1004** can include an antibody or antibody fragment (e.g., an antigen-binding fragment).

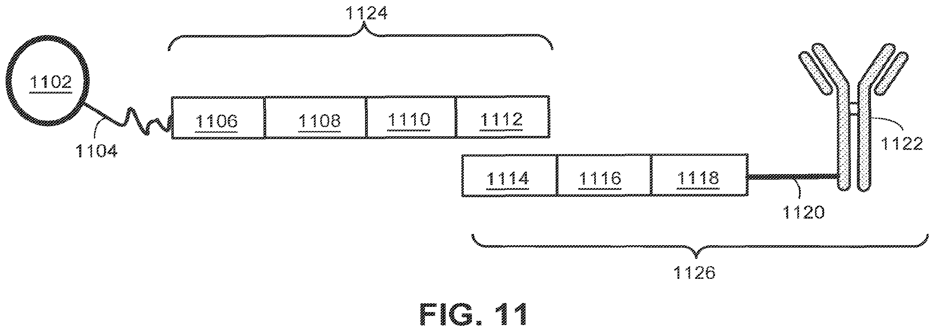

**FIG. 11** is a schematic diagram depicting an exemplary interaction between a feature-immobilized capture probe **1124** and an analyte capture agent **1126.** The feature-immobilized capture probe **1124** can include a spatial barcode **1108** as well as functional sequences **1106** and a UMI **1110,** as described elsewhere herein. The capture probe can be affixed **1104** to a feature such as a bead **1102.** The capture probe can also include a capture domain **1112** that is capable of binding to an analyte capture agent **1126.** The analyte-binding moiety barcode domain of the analyte capture agent **1126** can include a functional sequence **1118,** analyte binding moiety barcode **1116,** and an analyte capture sequence **1114** that is capable of binding (e,g, hybridizing) to the capture domain **1112** of the capture probe **1124.** The analyte capture agent can also include a linker **1120** that allows the analyte-binding moiety barcode domain (e.g., including the functional sequence **1118,** analyte binding barcode **1116,** and analyte capture sequence **1114)** to couple to the analyte binding moiety **1122.** In some embodiments, the linker is a cleavable linker. In some embodiments, the cleavable linker is a photo-cleavable linker, a UV-cleavable linker, or an enzyme cleavable linker. In some instances, the cleavable linker is a disulfide linker. A disulfide linker can be cleaved by use of a reducing agent, such as dithiothreitol (DTT), Beta-mercaptoethanol (BME), or Tris (2-carboxyethyl) phosphine (TCEP).

During analysis of spatial information, sequence information for a spatial barcode associated with an analyte is obtained, and the sequence information can be used to provide information about the spatial distribution of the analyte in the biological sample. Various methods can be used to obtain the spatial information. In some embodiments, specific capture probes and the analytes they capture are associated with specific locations in an array of features on a substrate. For example, specific spatial barcodes can be associated with specific array locations prior to array fabrication, and the sequences of the spatial barcodes can be stored (e.g., in a database) along with specific array location information, so that each spatial barcode uniquely maps to a particular array location.

Alternatively, specific spatial barcodes can be deposited at predetermined locations in an array of features during fabrication such that at each location, only one type of spatial barcode is present so that spatial barcodes are uniquely associated with a single feature of the array. Where necessary, the arrays can be decoded using any of the methods described herein so that spatial barcodes are uniquely associated with array feature locations, and this mapping can be stored as described above.

When sequence information is obtained for capture probes and/or analytes during analysis of spatial information, the locations of the capture probes and/or analytes can be determined by referring to the stored information that uniquely associates each spatial barcode with an array feature location. In this manner, specific capture probes and captured analytes are associated with specific locations in the array of features. Each array feature location represents a position relative to a coordinate reference point (e.g., an array location, a fiducial marker) for the array. Accordingly, each feature location has an "address" or location in the coordinate space of the array.

Some exemplary spatial analysis workflows are described in the Exemplary Embodiments section of PCT Publication No. WO2020/176788 and/or U.S. Patent Application Publication No. 2020/0277663. See, for example, the Exemplary embodiment starting with "In some non-limiting examples of the workflows described herein, the sample can be immersed..." of PCT Publication No. WO2020/176788 and/or U.S. Patent Application Publication No. 2020/0277663. See also, e.g., the Visium Spatial Gene Expression Reagent Kits User Guide (e.g., Rev F, dated January 2022); and/or the Visium Spatial Gene Expression Reagent Kits - Tissue Optimization User Guide (e.g., Rev E, dated February 2022).

In some embodiments, spatial analysis can be performed using dedicated hardware and/or software, such as any of the systems described in Sections (II)(e)(ii) and/or (V) of PCT Publication No. WO2020/176788 and/or U.S. Patent Application Publication No. 2020/0277663, or any of one or more of the devices or methods described in Sections *Control Slide for Imaging, Methods of Using Control Slides and Substrates for, Systems of Using Control Slides and Substrates for Imaging,* and/or *Sample and Array Alignment Devices and Methods, Informational labels* of PCT Publication No. WO2020/123320.

Suitable systems for performing spatial analysis can include components such as a chamber (e.g., a flow cell or sealable, fluid-tight chamber) for containing a biological sample. The biological sample can be mounted for example, in a biological sample holder. One or more fluid chambers can be connected to the chamber and/or the sample holder via fluid conduits, and fluids can be delivered into the chamber and/or sample holder via fluidic pumps, vacuum sources, or other devices coupled to the fluid conduits that create a pressure gradient to drive fluid flow. One or more valves can also be connected to fluid conduits to regulate the flow of reagents from reservoirs to the chamber and/or sample holder.

The systems can optionally include a control unit that includes one or more electronic processors, an input interface, an output interface (such as a display), and a storage unit (e.g., a solid state storage medium such as, but not limited to, a magnetic, optical, or other solid state, persistent, writeable and/or re-writeable storage medium). The control unit can optionally be connected to one or more remote devices via a network. The control unit (and components thereof) can generally perform any of the steps and functions described herein. Where the system is connected to a remote device, the remote device (or devices) can perform any of the steps or features described herein. The systems can optionally include one or more detectors (e.g., CCD, CMOS) used to capture images. The systems can also optionally include one or more light sources (e.g., LED-based, diode-based, lasers) for illuminating a sample, a substrate with features, analytes from a biological sample captured on a substrate, and various control and calibration media.

The systems can optionally include software instructions encoded and/or implemented in one or more of tangible storage media and hardware components such as application specific integrated circuits. The software instructions, when executed by a control unit (and in particular, an electronic processor) or an integrated circuit, can cause the control unit, integrated circuit, or other component executing the software instructions to perform any of the method steps or functions described herein.

In some cases, the systems described herein can detect (e.g., register an image) the biological sample on the array. Exemplary methods to detect the biological sample on an array are described in PCT Publication No. WO2021/102003 and/or U.S. Patent Application Publication No. 2021/0150707.

Prior to transferring analytes from the biological sample to the array of features on the substrate, the biological sample can be aligned with the array. Alignment of a biological sample and an array of features including capture probes can facilitate spatial analysis, which can be used to detect differences in analyte presence and/or level within different positions in the biological sample, for example, to generate a three-dimensional map of the analyte presence and/or level. Exemplary methods to generate a two- and/or three-dimensional map of the analyte presence and/or level are described in PCT Publication No. WO2020/053655 and spatial analysis methods are generally described in PCT Publication No. WO2021/102039 and/or U.S. Patent Application Publication No. 2021/0155982.

In some cases, a map of analyte presence and/or level can be aligned to an image of a biological sample using one or more fiducial markers, e.g., objects placed in the field of view of an imaging system which appear in the image produced, as described in the *Substrate Attributes* Section, *Control Slide for Imaging* Section of PCT Publication Nos. WO2020/123320, WO 2021/102005, and/or U.S. Patent Application Publication No. 2021/0158522. Fiducial markers can be used as a point of reference or measurement scale for alignment (e.g., to align a sample and an array, to align two substrates, to determine a location of a sample or array on a substrate relative to a fiducial marker) and/or for quantitative measurements of sizes and/or distances.

### B. Methods of High-Density and/or Multiple Barcoding

### (a) Introduction

Provided herein are methods, devices, compositions, and systems for spatially barcoding a target nucleic acid in a biological sample. In some embodiments, a method of high-density barcoding can include (a) providing an array comprising a first plurality of capture probes, wherein a first capture probe of the first plurality of capture probes comprises: (i) a first spatial barcode and (ii) a first capture domain, and wherein the first capture probe is attached to a substrate by its 3' end, and a second plurality of capture probes, wherein a second capture probe of the second plurality of capture probes comprises: (iii) a second spatial barcode and (iv) a second capture domain, and wherein the second capture probe is attached to the substrate by its 3' end; (b) hybridizing a first probe oligonucleotide and a second probe oligonucleotide to the target nucleic acid, wherein the first probe oligonucleotide and the second probe oligonucleotide each comprise a sequence that is substantially complementary to a first sequence and a second sequence of the target nucleic acid, respectively, and wherein the first probe oligonucleotide comprises a first capture probe binding domain and the second probe oligonucleotide comprises a second capture probe binding domain; (c) coupling the first probe oligonucleotide and the second probe oligonucleotide, thereby generating a ligation product; (d) releasing the first capture probe and/or the second capture probe from the substrate; and (e) hybridizing (i) the first capture domain of the first capture probe to the first capture probe binding domain of the ligation product, and optionally, (ii) the second capture domain of the second capture probe to the second capture probe binding domain of the ligation product.

In some embodiments, a method of multiple barcoding can include (a) providing an array comprising a first plurality of capture probes, wherein a first capture probe of the first plurality of capture probes comprises: (i) a first capture domain, (ii) a first spatial barcode, and (iii) a ligation domain substantially complementary to a portion of a first splint oligonucleotide of a plurality of first splint oligonucleotides, and optionally, a second plurality of capture probes, wherein a second capture probe of the second plurality of capture probes comprises (iv) a second ligation domain substantially complementary to a portion of the first splint oligonucleotide, (v) a second spatial barcode, and (vi) a third ligation domain substantially complementary to a portion of a second splint oligonucleotide of a plurality of second splint oligonucleotides; (b) hybridizing a first probe oligonucleotide and a second probe oligonucleotide to the target nucleic acid, wherein the first probe oligonucleotide and the second probe oligonucleotide each comprise a sequence that is substantially complementary to a first sequence and a second sequence of the target nucleic acid, respectively, and wherein the first probe oligonucleotide comprises a first capture probe binding domain; (c) coupling the first probe oligonucleotide and the second probe oligonucleotide, thereby generating a ligation product; (d) releasing the first capture probe, and optionally, the second capture probe from the array; and (e) hybridizing the first capture domain of the first capture probe to the first capture probe binding domain of the ligation product.

Also provided herein are methods, devices, compositions, and systems for spatially barcoding a target nucleic acid in one or more regions of interest from a biological sample. Thus, provided herein are methods of spatially barcoding a target nucleic acid in a biological sample, the method including: (a) providing a substrate including an array including a first plurality of capture probes, where a first capture probe of the first plurality of capture probes incudes: (i) a first spatial barcode and (ii) a first capture domain, and where the first capture probe is attached to the substrate by its 3' end, and a second plurality of capture probes, where a second capture probe of the second plurality of capture probes incudes: (iii) a second spatial barcode and (iv) a second capture domain, and where the second capture probe is attached to the substrate by its 3' end; (b) hybridizing a first probe oligonucleotide and a second probe oligonucleotide to the target nucleic acid, where the first probe oligonucleotide and the second probe oligonucleotide each include a sequence that is substantially complementary to a first sequence and a second sequence of the target nucleic acid, respectively, and where the first probe oligonucleotide incudes a first capture probe binding domain and the second probe oligonucleotide includes a second capture probe binding domain; (c) coupling the first probe oligonucleotide and the second probe oligonucleotide, thereby generating a ligation product; (d) determining a region of interest in the biological sample; (e) applying a mask to the substrate, such that the mask does not align with the substrate, wherein the first capture probe and/or the second capture probe are aligned with the region of interest before release; (f) releasing the first capture probe and/or the second capture probe from the region of interest; and (g) hybridizing (i) the first capture domain of the first capture probe to the first capture probe binding domain of the ligation product, and optionally, (ii) the second capture domain of the second capture probe to the second capture probe binding domain of the ligation product, thereby spatially barcoding the target nucleic acid in the biological sample.

Also provided herein are methods for spatially barcoding a target nucleic acid in a biological sample, the method including: (a) providing an array including a first plurality of capture probes, where a first capture probe of the first plurality of capture probes incudes: (i) a first capture domain, (ii) (ii) a first spatial barcode, and (iii) (iii) a ligation domain substantially complementary to a portion of a first splint oligonucleotide of a plurality of first splint oligonucleotides, and optionally, a second plurality of capture probes, where a second capture probe of the second plurality of capture probes incudes: (iv) a second ligation domain substantially complementary to a portion of the first splint oligonucleotide, (v) a second spatial barcode, and (vi) a third ligation domain substantially complementary to a portion of a second splint oligonucleotide of a plurality of second splint oligonucleotides; (b) hybridizing a first probe oligonucleotide and a second probe oligonucleotide to the target nucleic acid, where the first probe oligonucleotide and the second probe oligonucleotide each include a sequence that is substantially complementary to a first sequence and a second sequence of the target nucleic acid, respectively, and where the first probe oligonucleotide incudes a first capture probe binding domain; (c) coupling the first probe oligonucleotide and the second probe oligonucleotide, thereby generating a ligation product; (d) determining a region of interest in the biological sample; (e) selectively releasing the first capture probe, and optionally, the second capture probe from the array into the region of interest; and (f) hybridizing the first capture domain of the first capture probe to the first capture probe binding domain of the ligation product, thereby spatially barcoding the target nucleic acid.

In some embodiments, the methods provided herein can also be used to determine the location of the target nucleic acid in a biological sample. In some embodiments, the methods can include aligning (e.g., sandwiching) a first substrate (e.g., an array) that includes a plurality of barcoded capture probes with a second substrate having the biological sample, thereby "sandwiching" the biological sample between the two substrates. Upon alignment of the biological sample with the substrate having a plurality of probes, the barcoded capture probes on the first substrate can be released from the substrate and migrate to the biological sample in order to interact with (e.g., hybridize to) an analyte or intermediate agent such as a ligation product. After this interaction, the location of the target nucleic acid in a biological sample can be determined, as provided herein.

In some embodiments, the methods can be performed without "sandwiching" the biological sample between the two substrates. In some embodiments, the methods can include contacting the biological sample to the substrate (e.g., an array) that includes a plurality of barcoded capture probe, wherein the capture probes can directly interact with the target nucleic acid in the biological sample.

Embodiments of the methods, devices, compositions, and kits disclosed herein are provided below.

### (b) Exemplary First and Second Substrates

A substrate, e.g., a first substrate and/or a second substrate, can generally have any suitable form or format. For example, a substrate can be flat, curved, e.g., convexly or concavely curved. For example, a substrate can be curved towards the area where the interaction between a biological sample, e.g., tissue sample, and a substrate takes place. In some embodiments, a substrate is flat, e.g., planar, chip, or slide. A substrate can contain one or more patterned surfaces within the substrate (e.g., channels, wells, projections, ridges, divots, etc.).

A substrate, e.g., a first substrate and/or second substrate, can be of any desired shape. For example, a substrate can be typically a thin, flat shape (e.g., a square or a rectangle). In some embodiments, a substrate structure has rounded corners (e.g., for increased safety or robustness). In some embodiments, a substrate structure has one or more cut-off corners (e.g., for use with a slide clamp or cross-table). In some embodiments wherein a substrate structure is flat, the substrate structure can be any appropriate type of support having a flat surface (e.g., a chip or a slide such as a microscope slide).

First and/or second substrates can optionally include various structures such as, but not limited to, projections, ridges, and channels. A substrate can be micropatterned to limit lateral diffusion of analytes (e.g., to improve resolution of the spatial analysis). A substrate modified with such structures can be modified to allow association of analytes, features (e.g., beads), or probes at individual sites. For example, the sites where a substrate is modified with various structures can be contiguous or non-contiguous with other sites.

In some embodiments, the surface of a substrate is modified to contain one or more wells, using techniques such as, but not limited to, stamping, microetching, or molding techniques. In some embodiments in which a substrate includes one or more wells, the substrate can be a concavity slide or cavity slide. For example, wells can be formed by one or more shallow depressions on the surface of the substrate. In some embodiments, where a substrate includes one or more wells, the wells can be formed by attaching a cassette (e.g., a cassette containing one or more chambers) to a surface of the substrate structure.

In some embodiments where the substrate is modified to contain one or more structures, including but not limited to, wells, projections, ridges, features, or markings, the structures can include physically altered sites. For example, a substrate modified with various structures can include physical properties, including, but not limited to, physical configurations, magnetic or compressive forces, chemically functionalized sites, chemically altered sites, and/or electrostatically altered sites. In some embodiments where the substrate is modified to contain various structures, including but not limited to wells, projections, ridges, features, or markings, the structures are applied in a pattern. Alternatively, the structures can be randomly distributed.

In some embodiments, a substrate can include one or more markings on its surface, e.g., to provide guidance for aligning at least a portion of the biological sample with a plurality of capture probes on the substrate during a sandwich process disclosed herein. For example, the substrate can include a sample area indicator identifying the sample area. In some embodiments, during a sandwiching process described herein the sample area indicator on the first substrate is aligned with an area of the second substrate comprising a plurality of capture probes. In some embodiments, the substrate can include a fiducial marker. In some embodiments, the substrate does not comprise a fiducial marker. In some embodiments, the first substrate does not comprise a fiducial marker and the second substrate comprises a fiducial marker. Such markings can be made using techniques including, but not limited to, printing, sand-blasting, and depositing on the surface.

In some embodiments, imaging can be performed using one or more fiducial markers, e.g., objects placed in the field of view of an imaging system which appear in the image produced. Fiducial markers are typically used as a point of reference or measurement scale. Fiducial markers can include, but are not limited to, detectable labels such as fluorescent, radioactive, chemiluminescent, and colorimetric labels. The use of fiducial markers to stabilize and orient biological samples is described, for example, in Carter et al., Applied Optics 46:421-427, 2007). In some embodiments, a fiducial marker can be a physical particle (e.g., a nanoparticle, a microsphere, a nanosphere, a bead, a post, or any of the other exemplary physical particles described herein or known in the art).

In some embodiments, a fiducial marker can be present on a substrate to provide orientation of the biological sample. In some embodiments, a microsphere can be coupled to a substrate to aid in orientation of the biological sample. In some examples, a microsphere coupled to a substrate can produce an optical signal (e.g., fluorescence). In some embodiments, a quantum dot can be coupled to the substrate to aid in the orientation of the biological sample. In some examples, a quantum dot coupled to a substrate can produce an optical signal.

In some embodiments, a fiducial marker can be an immobilized molecule with which a detectable signal molecule can interact to generate a signal. For example, a marker nucleic acid can be linked or coupled to a chemical moiety capable of fluorescing when subjected to light of a specific wavelength (or range of wavelengths). Although not required, it can be advantageous to use a marker that can be detected using the same conditions (e.g., imaging conditions) used to detect a labelled cDNA.

In some embodiments, a fiducial marker can be randomly placed in the field of view. For example, an oligonucleotide containing a fluorophore can be randomly printed, stamped, synthesized, or attached to a substrate (e.g., a glass slide) at a random position on the substrate. A tissue section can be contacted with the substrate such that the oligonucleotide containing the fluorophore contacts, or is in proximity to, a cell from the tissue section or a component of the cell (e.g., an mRNA or DNA molecule). An image of the substrate and the tissue section can be obtained, and the position of the fluorophore within the tissue section image can be determined (e.g., by reviewing an optical image of the tissue section overlaid with the fluorophore detection). In some embodiments, fiducial markers can be precisely placed in the field of view (e.g., at known locations on a substrate). In this instance, a fiducial marker can be stamped, attached, or synthesized on the substrate and contacted with a biological sample. Typically, an image of the sample and the fiducial marker is taken, and the position of the fiducial marker on the substrate can be confirmed by viewing the image.

In some embodiments, a fiducial marker can be an immobilized molecule (e.g., a physical particle) attached to the substrate. For example, a fiducial marker can be a nanoparticle, e.g., a nanorod, a nanowire, a nanocube, a nanopyramid, or a spherical nanoparticle. In some examples, the nanoparticle can be made of a heavy metal (e.g., gold). In some embodiments, the nanoparticle can be made from diamond. In some embodiments, the fiducial marker can be visible by eye.

A wide variety of different substrates can be used for the foregoing purposes. In general, a substrate can be any suitable support material. Exemplary substrates include, but are not limited to, glass, modified and/or functionalized glass, hydrogels, films, membranes, plastics (including e.g., acrylics, polystyrene, copolymers of styrene and other materials, polypropylene, polyethylene, polybutylene, polyurethanes, TeflonTM, cyclic olefins, polyimides etc.), nylon, ceramics, resins, Zeonor, silica or silica-based materials including silicon and modified silicon, carbon, metals, inorganic glasses, optical fiber bundles, and polymers, such as polystyrene, cyclic olefin copolymers (COCs), cyclic olefin polymers (COPs), polypropylene, polyethylene polycarbonate, or combinations thereof.

Among the examples of substrate materials discussed above, polystyrene is a hydrophobic material suitable for binding negatively charged macromolecules because it normally contains few hydrophilic groups. For nucleic acids immobilized on glass slides, by increasing the hydrophobicity of the glass surface the nucleic acid immobilization can be increased. Such an enhancement can permit a relatively more densely packed formation (e.g., provide improved specificity and resolution).

In another example, a substrate can be a flow cell. Flow cells can be formed of any of the foregoing materials, and can include channels that permit reagents, solvents, features, and analytes to pass through the flow cell. In some embodiments, a hydrogel embedded biological sample is assembled in a flow cell (e.g., the flow cell is utilized to introduce the hydrogel to the biological sample). In some embodiments, a hydrogel embedded biological sample is not assembled in a flow cell. In some embodiments, the hydrogel embedded biological sample can then be prepared and/or isometrically expanded as described herein.

In some embodiments, the biological sample is placed (e.g., mounted) on a substrate. The substrate can be any solid or semi-solid support upon which a biological sample can be mounted. In some instances, the substrate is a slide. In some embodiments, the slide is a glass slide. In some embodiments, the substrate is made of glass, silicon, paper, hydrogel, polymer monoliths, or other material known in the art. In some embodiments, the substrate is comprised of an inert material or matrix (e.g., glass slides) that has been functionalized by, for example, treating the substrate with a material comprising reactive groups which facilitate mounting of the biological sample.

In some embodiments, the substrate comprises a plurality (e.g., array) of capture probes, each comprising a spatial barcode and a capture domain. In some embodiments, the substrate does not comprise a plurality (e.g., array) of capture probes.

### (c) High-Density Spatial Barcoding of Nucleic Acid Analytes using RNA-Templated Ligation

In some embodiments, the methods, devices, compositions, and kits described herein utilize RNA-templated ligation to detect the analyte. In some embodiments, the methods described herein include hybridizing a first probe oligonucleotide and a second probe oligonucleotide to the target nucleic acid, where the first probe oligonucleotide and the second probe oligonucleotide each comprise a sequence that is substantially complementary to a first sequence and a second sequence of the target nucleic acid, respectively, and where the first probe oligonucleotide comprises a first capture probe binding domain and the second probe oligonucleotide comprises a second capture probe binding domain; coupling the first probe oligonucleotide and the second probe oligonucleotide, thereby generating a ligation product; releasing the first capture probe and/or the second capture probe from the array; and hybridizing (i) the first capture probe to the first capture probe binding domain of the ligation product, and optionally, (ii) the second capture probe to the second capture probe binding domain of the ligation product.

As used herein, spatial "RNA-templated ligation," or "RTL" or simply "templated ligation" is a process wherein individual probe oligonucleotides (e.g., a first probe oligonucleotide, a second probe oligonucleotide) in a probe pair hybridize to adjacent sequences of an analyte (e.g., an RNA molecule, a DNA molecule) in a biological sample (e.g., a tissue sample). The RTL probe oligonucleotides are then coupled (e.g., ligated) together, thereby creating a connected probe (e.g., a ligation product). RNA-templated ligation is disclosed in PCT Publ. Nos. WO 2022/140028 A1, WO 2021/133849 A1, and U.S. Publ. No. US 2021/0285046 A1.

One advantage to using RTL is that it allows for enhanced detection of analytes (e.g., low expressing analytes) because both probe oligonucleotides must hybridize to the analyte in order for the coupling (e.g., ligating) reaction to occur. As used herein, "coupling" refers to an interaction between two probe oligonucleotides that results in a single connected probe that comprises the two probe oligonucleotides. In some embodiments, coupling is achieved through ligation. In some instances, coupling is achieved through extension of one probe oligonucleotide to the second probe oligonucleotide followed by ligation. In some embodiments, coupling is achieved through hybridization (e.g., using a third probe oligonucleotide that hybridized to each of the two probe oligonucleotides) followed by extension of one probe oligonucleotide or gap filling of the sequence between the two probe oligonucleotides using the third probe oligonucleotide as a template.

The connected probe (e.g., ligation product) that results from the coupling (e.g., ligation) of the two probe oligonucleotides can serve as a proxy for the target analyte, as such it is sometimes called an analyte derived molecule. Further, it is appreciated that probe oligonucleotide pairs can be designed to cover any gene of interest. For example, a pair of probe oligonucleotides can be designed so that each analyte, e.g., a whole exome, a transcriptome, a genome, can conceivably be detected using probe oligonucleotide pairs.

In some embodiments, the first probe oligonucleotide and the second probe oligonucleotide hybridize to adjacent sequences on the target nucleic acid. In some embodiments, the first probe oligonucleotide and the second probe oligonucleotide hybridize to non-adjacent sequences on the target nucleic acid. In some embodiments, a gap-filling reaction extends the first probe oligonucleotide or second probe oligonucleotide. In some embodiments, the coupling of the first probe oligonucleotide and the second probe oligonucleotide comprises ligating the first probe oligonucleotide and the second probe oligonucleotide. In some embodiments, the coupling of the first probe oligonucleotide and the second probe oligonucleotide comprises ligating the first probe oligonucleotide and the second probe oligonucleotide via a ligase. Ligation can be performed enzymatically or chemically, as described herein. For example, the first and second probe oligonucleotides are hybridized to the first and second target regions of the analyte, and the probe oligonucleotides are subjected to a nucleic acid reaction to ligate them together. For example, the probes may be subjected to an enzymatic ligation reaction using a ligase (e.g., T4 RNA ligase (Rnl2), an enzyme isolated from *Acanthocystis turfacea* chlorella virus 1 (ATCV1) (also referred to as an ATCV1 ligase), Chlorella virus DNA ligase, or a T4 DNA ligase). See, e.g., Zhang L., et al.; Archaeal RNA ligase from Thermococcus kodakarensis for template dependent ligation RNA Biol. 2017; 14(1): 36-44 for a description of KOD ligase. A skilled artisan will understand that various reagents, buffers, cofactors, etc. may be included in a ligation reaction depending on the ligase being used.

In some embodiments, the methods include hybridizing the first and second probe oligonucleotide pairs (e.g., RTL probes) to adjacent or nearby sequences of a target nucleic acid (e.g., RNA; e.g., mRNA) of interest. In some embodiments, the probe oligonucleotide pairs include sequences that are complementary or substantially complementary to a target nucleic acid. For example, in some embodiments, each probe oligonucleotide includes a sequence that is complementary or substantially complementary to an mRNA of interest (e.g., to a portion of the sequence of an mRNA of interest). In some embodiments, each target nucleic acid includes a first target region and a second target region. In some embodiments, the methods include providing a plurality of first probe oligonucleotides and a plurality of second probe oligonucleotides, where a pair of probe oligonucleotides for a target nucleic acid comprises both a first and second probe oligonucleotide. In some embodiments, a first probe oligonucleotide hybridizes to a first target region of the nucleic acid, and the second probe oligonucleotide hybridizes to a second, adjacent or nearly adjacent target region of the nucleic acid.

In some instances, the probe oligonucleotides are DNA molecules. In some instances, the first probe oligonucleotide is a DNA molecule. In some instances, the second probe oligonucleotide is a DNA molecule. In some instances, the first probe oligonucleotide comprises at least two ribonucleic acid bases at the 3' end. In some instances, the second probe oligonucleotide comprises a phosphorylated nucleotide at the 5' end.

RTL probes can be designed using methods known in the art. In some instances, probe pairs are designed to cover an entire transcriptome of a species (e.g., a mouse or a human). In some instances, RTL probes are designed to cover a subset of a transcriptome (e.g., a mouse or a human). In some instances, the methods disclosed herein utilize about 500, about 1000, about 2000, about 3000, about 4000, about 5000, about 6000, about 7000, about 8000, about 9000, about 10,000, about 15,000, about 20,000, or more probe pairs.

In some embodiments, one of the probe oligonucleotides of the pair of probe oligonucleotides for RTL includes a poly(A) sequence or a complement thereof. In some instances, the poly(A) sequence or a complement thereof is on the 5' end of one of the probe oligonucleotides. In some instances, the poly(A) sequence or a complement thereof is on the 3' end of one of the probe oligonucleotides. In some embodiments, one probe oligonucleotide of the pair of probe oligonucleotides for RTL includes a degenerate or UMI sequence. In some embodiments, the UMI sequence is specific to a particular target or set of targets. In some instances, the UMI sequence or a complement thereof is on the 5' end of one of the probe oligonucleotides. In some instances, the UMI sequence or a complement thereof is on the 3' end of one of the probe oligonucleotides.

In some embodiments, the first and second target regions of an analyte are directly adjacent to one another. In some embodiments, the complementary sequences to which the first probe oligonucleotide and the second probe oligonucleotide hybridize are 1, 2, 3, 4, 5, 6, 7, 8, 9, 10, about 15, about 20, about 25, about 30, about 35, about 40, about 45, about 50, about 55, about 60, about 65, about 70, about 75, about 80, about 85, about 90, about 95, about 100, about 125, or about 150 nucleotides away from each other. Gaps between the probe oligonucleotides may first be filled prior to coupling (e.g., ligation), using, for example, dNTPs in combination with a polymerase such as polymerase mu, DNA polymerase, RNA polymerase, reverse transcriptase, VENT polymerase, Taq polymerase, and/or any combinations, derivatives, and variants (e.g., engineered mutants) thereof. In some embodiments, when the first and second probe oligonucleotides are separated from each other by one or more nucleotides, deoxyribonucleotides are used to extend and couple (e.g., ligate) the first and second probe oligonucleotides.

In some embodiments, the first probe oligonucleotide and the second probe oligonucleotide hybridize to an analyte on the same transcript. In some instances, the first probe oligonucleotide and the second probe oligonucleotide hybridize to an analyte on the same exon. In some instances, the first probe oligonucleotide and the second probe oligonucleotide hybridize to an analyte on different exons. In some instances, the first probe oligonucleotide and the second probe oligonucleotide hybridize to an analyte that is the result of a translocation event (e.g., in the setting of cancer). In some embodiments, the methods provided herein make it possible to identify alternative splicing events, translocation events, and mutations that change the hybridization rate of one or both probe oligonucleotides (e.g., single nucleotide polymorphisms, insertions, deletions, point mutations).

In some embodiments, the first and/or second probe as disclosed herein includes at least two ribonucleic acid bases at the 3' end; a functional sequence; a phosphorylated nucleotide at the 5' end; and/or a capture probe binding domain. In some embodiments, the functional sequence is a primer sequence.

As used herein, a "capture probe binding domain" is a sequence that is complementary to a particular capture domain present in a capture probe. In some embodiments, the capture probe binding domain includes a poly(A) sequence. In some embodiments, the capture probe binding domain includes a poly-uridine sequence, a poly-thymidine sequence, or a combination thereof. In some embodiments, the capture probe binding domain includes a random sequence (e.g., a random hexamer or octamer). In some embodiments, the capture probe binding domain is complementary to a capture domain in a capture probe that detects a particular target(s) of interest. In some embodiments, a capture probe binding domain blocking moiety that interacts with the capture probe binding domain is provided. In some embodiments, a capture probe binding domain blocking moiety includes a sequence that is complementary or substantially complementary to a capture probe binding domain. In some embodiments, a capture probe binding domain blocking moiety prevents the capture probe binding domain from binding the capture probe when present. In some embodiments, a capture probe binding domain blocking moiety is removed prior to binding the capture probe binding domain (e.g., present in a connected probe (e.g., a ligation product)) to a capture probe. In some embodiments, a capture probe binding domain blocking moiety comprises a poly-uridine sequence, a poly-thymidine sequence, or a combination thereof.

In some embodiments, a first capture probe binding domain comprises a first sequence complementary to at least a portion of the first capture domain of the first capture probe, and a second capture probe binding domain comprises a second sequence complementary to at least a portion of the second capture domain of the second capture probe. In some embodiments, the first sequence complementary to at least a portion of the first capture domain of the first capture probe comprises a poly(A) sequence. In some embodiments, the second sequence complementary to at least a portion of the second capture domain of the second capture probe comprises a fixed sequence. As used herein, a "fixed sequence" is a non-random sequence. A "fixed sequence" can be complementary to the non-templated sequence that is incorporated into the extension product(s) described herein. For example, it is only necessary for the capture probe binding domain (e.g., a first capture probe binding domain, a second capture probe binding domain, etc.) and the capture domain to be substantially complementary to each other, such that the capture domain including a fixed sequence can hybridize to and/or capture the ligation product(s).

In some embodiments, the first capture probe hybridized to the first capture probe binding domain of the ligation product is extended with a polymerase, thereby generating a complement of the ligation product. In some embodiments, the complement of the ligation product is ligated to the second capture probe. In some embodiments, the ligation comprises the use of a ligase.

Hybridization of the probe oligonucleotides to the target analyte can occur at a target having a sequence that is 100% complementary to the probe oligonucleotide(s). In some embodiments, hybridization can occur at a target having a sequence that is at least (e.g. at least about) 80%, at least (e.g. at least about) 85%, at least (e.g. at least about) 90%, at least (e.g. at least about) 95%, at least (e.g. at least about) 96%, at least (e.g. at least about) 97%, at least (e.g. at least about) 98%, or at least (e.g. at least about) 99% complementary to the probe oligonucleotide(s). After hybridization, in some embodiments, the first probe oligonucleotide is extended. After hybridization, in some embodiments, the second probe oligonucleotide is extended. For example, in some embodiments, a first probe oligonucleotide hybridizes to a target sequence upstream of a second oligonucleotide probe, whereas in other embodiments, a first probe oligonucleotide hybridizes to a target sequence downstream of a second probe oligonucleotide.

In some embodiments, hybridization of probe oligonucleotides occurs in a reaction that occurs for a period of overnight, about 1 hour, 30 minutes, about 15 minutes, about 10 minutes, about 5 minutes, about 1 minute or less.

In some embodiments, methods disclosed herein can include a wash step after hybridizing the first and the second probe oligonucleotides. The wash step removes any unbound probe oligonucleotides and can be performed using any technique known in the art. In some embodiments, a pre-hybridization buffer is used to wash the sample. In some embodiments, a phosphate buffer is used. In some embodiments, multiple wash steps are performed to remove unbound oligonucleotides. For example, it is advantageous to decrease the amount of unhybridized probes present in a biological sample as they may interfere with downstream applications and methods.

In some embodiments, after hybridization of probe oligonucleotides (e.g., first and the second probe oligonucleotides) to the target analyte in the biological sample, the probe oligonucleotides (e.g., the first probe oligonucleotide and the second probe oligonucleotide) are coupled (e.g., ligated) together, creating a single connected probe (e.g., a ligation product) that is complementary to the target analyte.

In some embodiments, the first probe oligonucleotide and the second probe oligonucleotides are on a contiguous nucleic acid sequence. In some embodiments, the first probe oligonucleotide is on the 3' end of the contiguous nucleic acid sequence. In some embodiments, the first probe oligonucleotide is on the 5' end of the contiguous nucleic acid sequence. In some embodiments, the second probe oligonucleotide is on the 3' end of the contiguous nucleic acid sequence. In some embodiments, the second probe oligonucleotide is on the 5' end of the contiguous nucleic acid sequence.

In some embodiments, the method further includes hybridizing a third probe oligonucleotide to the first probe oligonucleotide and the second probe oligonucleotide such that the first probe oligonucleotide and the second probe oligonucleotide abut each other. In some embodiments, the third probe oligonucleotide comprises a sequence that is at least 80%, at least 85%, at least 90%, at least 95%, at least 96%, at least 97%, at least 98%, or at least 99% complementary to a portion of the first probe oligonucleotide that hybridizes to the third probe oligonucleotide. In some embodiments, the third probe oligonucleotide comprises a sequence that is 100% complementary to a portion of the first probe oligonucleotide that hybridizes to the third probe oligonucleotide. In some embodiments, the third probe oligonucleotide comprises a sequence that is at least 80%, at least 85%, at least 90%, at least 95%, at least 96%, at least 97%, at least 98%, or at least 99% complementary to a portion of the second probe oligonucleotide that hybridizes to the third probe oligonucleotide. In some embodiments, the third probe oligonucleotide comprises a sequence that is 100% complementary to a portion of the second probe oligonucleotide that hybridizes to the third probe oligonucleotide.

In some embodiments, the release of the capture probes from the array can be performed using any of the methods of removal/cleavage described herein. In some embodiments, the releasing of the first capture probe and/or the second capture probe comprises cleaving the first capture probe, and optionally, the second capture probe from the substrate. In some embodiments, the first capture probe comprises a first cleavable linker and the second capture probe comprises a second cleavable linker. In some embodiments, the first cleavable linker and the second cleavable linker comprise a restriction enzyme site. In some embodiments, the first cleavable linker and the second cleavable linker comprise a photocleavable linker. In some embodiments, the first cleavable linker and the second cleavable linker are different. In some embodiments, the first cleavable linker and the second cleavable linker are the same. In some embodiments, the first cleavable linker and the second cleavable linker are cleaved at the same time. In some embodiments, the first cleavable linker is cleaved prior to the second cleavable linker being cleaved, and wherein the first capture domain hybridizes to the first capture probe binding domain of the ligation product at a first time point and the second capture domain hybridizes to the second capture probe binding domain of the ligation product at a second time point. In some embodiments, the first capture probe comprises a cleavable linker and the second capture probe does not have a cleavable linker. In some embodiments, the first capture probe does not have a cleavable linker and the second capture probe comprises a cleavable linker.

In some embodiments, the method can further comprise i) cleaving the first cleavable linker; ii) permeabilizing the biological sample; iii) hybridizing the first capture domain of the first capture probe to the first capture probe binding domain of the ligation product, thereby generating a first capture probe/ligation product complex; and iv) hybridizing the first capture probe/ligation product complex to the second capture probe on the array via the second capture probe binding domain. In such embodiments, the second capture probe does not include a cleavable linker.

In some embodiments, the cleavable linker comprises nucleotides with photo-sensitive chemical bonds; an ultrasonic cleavage domain; one or more labile chemical bond; a sequence that is recognized by one or more enzymes capable of cleaving a nucleic acid molecule; a poly(U) sequence capable of being cleaved by a mixture of Uracil DNA glycosylase (UDG) and the DNA glycosylase-lyase Endonuclease VIII; one or more disulfide bonds; or any combination thereof. In some embodiments, removal of capture probes from the array can be performed physically by light or heat, chemically, enzymatically, or any combination thereof.

In some embodiments, the capture probe is released from the surface of the substrate (e.g., array) by physical means. Methods for disrupting the interaction between nucleic acid molecules are known in the art. One method for releasing the DNA molecules (i.e., of stripping the array of capture probes) is to use a solution (e.g., a first reagent medium) that interferes with the bonds between nucleotides. In some embodiments, the capture probe is released by heating the first reagent medium to at least 85°C, e.g., at least 90°C, 91°C, 92°C, 93°C, 94°C, 95°C, 96°C, 97°C, 98°C, or 99°C. In some embodiments, the first reagent medium includes salts, surfactants, etc. that can further destabilize the interaction between the array and the capture probe.

In some embodiments, the first reagent medium can include a salt, polyethylene glycol (PEG), and/or a detergent selected from sodium dodecyl sulfate (SDS), sarkosyl, saponin, a nonionic surfactant, a polysorbate surfactant, or any combination thereof.

In some embodiments, the release of the capture probe (e.g., the first capture probe and/or the second capture probe) from the array and the coupling of the released capture probe with the ligation product in the biological sample occurs in about 30 minutes or less, about 15 minutes or less, about 10 minutes or less, about 5 minutes or less, or about 1 minute, or less.

In some embodiments, the first reagent medium further includes a detergent. In some embodiments, the detergent is selected from sodium dodecyl sulfate (SDS), sarkosyl, saponin, Triton X-100TM, or Tween-20TM. In some embodiments, the first reagent medium includes less than 5 w/v% of a detergent selected from sodium dodecyl sulfate (SDS) and sarkosyl. In some embodiments, the first reagent medium does not include SDS or sarkosyl.

In some embodiments, the first reagent medium comprises polyethylene glycol (PEG). In some embodiments, the PEG is from about PEG 2K to about PEG 16K. In some embodiments, the PEG is PEG 2K, 3K, 4K, 5K, 6K, 7K, 8K, 9K, 10K, 11K, 12K, 13K, 14K, 15K, or 16K. In some embodiments, the PEG is present at a concentration from about 2% to 25%, from about 4% to about 23%, from about 6% to about 21%, or from about 8% to about 20% (v/v).

In some embodiments, the methods described herein can further include releasing the ligation product from the target nucleic acid. In some instances, release of the ligation product is achieved by adding a second reagent medium to the biological sample. The second reagent medium may include a protease (e.g., pepsin or protease K) to permeabilize the sample. Additionally, or alternatively, the second reagent medium includes an agent (e.g., a nuclease) to digest the target nucleic acid hybridized to the ligation product. In some embodiments, the agent for releasing the ligation product comprises a nuclease. In some embodiments, the nuclease is an endonuclease. In some embodiments, the nuclease is an exonuclease. To release the ligation product from the target nucleic acid, an endoribonuclease such as an RNase (e.g., RNase A, RNase C, RNase H, or RNase I) can be used. In some embodiments, the ligation product is released enzymatically. In some embodiments, an endoribonuclease is used to release the ligation product from the nucleic acid. In some embodiments, the endoribonuclease is one or more of RNase H. In some embodiments, the RNase H is RNase H1 or RNase H2.

In some embodiments, the second reagent medium includes a wetting agent.

In some embodiments, the second reagent medium comprises a permeabilization agent such as proteinase K (to permeabilize the biological sample) and a releasing agent such as an endonuclease such as RNase (to release the ligation product from the nucleic acid). In some embodiments, the permeabilization step and releasing step occur at the same time (e.g., by the addition of the second reagent medium). In some embodiments, the protease is selected from trypsin, pepsin, elastase, or Proteinase K. In some embodiments, the protease is an endopeptidase. Endopeptidases that can be used include but are not limited to trypsin, chymotrypsin, elastase, thermolysin, pepsin, clostripan, glutamyl endopeptidase (GluC), ArgC, peptidyl-asp endopeptidase (ApsN), endopeptidase LysC and endopeptidase LysN. In some embodiments, the endopeptidase is pepsin.

In some embodiments, the second reagent medium further includes a detergent. In some embodiments, the detergent is selected from sodium dodecyl sulfate (SDS), sarkosyl, saponin, Triton X-100^{™}, or Tween-20^{™}. In some embodiments, the second reagent medium includes less than 5 w/v% of a detergent selected from sodium dodecyl sulfate (SDS) and sarkosyl. In some embodiments, the second reagent medium includes as least 5% w/v% of a detergent selected from SDS and sarkosyl. In some embodiments, the second reagent medium does not include SDS or sarkosyl.

In some embodiments, the biological sample and the array are contacted with the second reagent medium for about 1 to about 60 minutes (e.g., about 1 to about 55 minutes, about 1 to about 50 minutes, about 1 to about 45 minutes, about 1 to about 40 minutes, about 1 to about 35 minutes, about 1 to about 30 minutes, about 1 to about 25 minutes, about 1 to about 20 minutes, about 1 to about 15 minutes, about 1 to about 10 minutes, about 1 to about 5 minutes, about 5 to about 60 minutes, about 5 to about 55 minutes, about 5 to about 50 minutes, about 5 to about 45 minutes, about 5 to about 40 minutes, about 5 to about 35 minutes, about 5 to about 30 minutes, about 5 to about 25 minutes, about 5 to about 20 minutes, about 5 to about 15 minutes, about 5 to about 10 minutes, about 10 to about 60 minutes, about 10 to about 55 minutes, about 10 to about 50 minutes, about 10 to about 45 minutes, about 10 to about 40 minutes, about 10 to about 35 minutes, about 10 to about 30 minutes, about 10 to about 25 minutes, about 10 to about 20 minutes, about 10 to about 15 minutes, about 15 to about 60 minutes, about 15 to about 55 minutes, about 15 to about 50 minutes, about 15 to about 45 minutes, about 15 to about 40 minutes, about 15 to about 35 minutes, about 15 to about 30 minutes, about 15 to about 25 minutes, about 15 to about 20 minutes, about 20 to about 60 minutes, about 20 to about 55 minutes, about 20 to about 50 minutes, about 20 to about 45 minutes, about 20 to about 40 minutes, about 20 to about 35 minutes, about 20 to about 30 minutes, about 20 to about 25 minutes, about 25 to about 60 minutes, about 25 to about 55 minutes, about 25 to about 50 minutes, about 25 to about 45 minutes, about 25 to about 40 minutes, about 25 to about 35 minutes, about 25 to about 30 minutes, about 30 to about 60 minutes, about 30 to about 55 minutes, about 30 to about 50 minutes, about 30 to about 45 minutes, about 30 to about 40 minutes, about 30 to about 35 minutes, about 35 to about 60 minutes, about 35 to about 55 minutes, about 35 to about 50 minutes, about 35 to about 45 minutes, about 35 to about 40 minutes, about 40 to about 60 minutes, about 40 to about 55 minutes, about 40 to about 50 minutes, about 40 to about 45 minutes, about 45 to about 60 minutes, about 45 to about 55 minutes, about 45 to about 50 minutes, about 50 to about 60 minutes, about 50 to about 55 minutes, or about 55 to about 60 minutes). In some embodiments, the biological sample and the array are contacted with the second reagent medium for about 30 minutes.

In some embodiments, the ligation product includes a capture probe binding domain, which can hybridize to a cleaved capture probe (e.g., a first capture probe and/or a second capture probe). In some embodiments, a cleaved capture probe can include a spatial barcode, a primer, and the capture domain. In some embodiments, the capture probe includes a UMI. In some embodiments, the capture probe binding domain of the ligation product specifically binds (e.g., hybridizes) to the capture domain of the cleaved capture probe.

In some embodiments, methods provided herein can include mounting a biological sample on a second substrate, then aligning the substrate with a first substrate including an array, such that at least a portion of the biological sample is aligned with at least a portion of the array, wherein the array includes a plurality of capture probes (e.g., to be cleaved). After hybridization of the ligation product to the cleaved capture probe, downstream methods as disclosed herein can be performed.

In some embodiments, at least 50% of the ligation products from the portion of the biological sample aligned with the portion of the array are captured by cleaved capture probes in the biological sample. In some embodiments, at least 50%, at least 55%, at least 60%, at least 65%, at least 70%, at least 75%, at least 85%, at least 90%, at least 95%, at least 96%, at least 97%, at least 98%, at least 99%, or 100% of ligation products are detected in spots in the biological sample.

In some embodiments, a cleaved capture probe includes a poly(T) sequence. In some embodiments, the cleaved capture probe includes a sequence specific to the analyte. In some embodiments, the cleaved capture probe includes a functional domain (e.g., a primer). In some embodiments, the cleaved capture probe further includes one or more functional domains, a unique molecular identifier (UMI), a cleavage domain, and combinations thereof. In some embodiments, the capture probe binding domain includes a sequence complementary to a capture domain of a capture probe that detects a target analyte of interest or an analyte derived molecule (e.g., a ligation product). In some embodiments, the analyte is DNA. In some embodiments, the analyte is RNA. In some embodiments, the analyte is mRNA.

In some embodiments, the methods described herein further include amplifying the ligation product after the cleaved capture probe interacts with the ligation product. In some embodiments, the entire ligation product and cleaved capture probe is amplified. In some embodiments, only part of the ligation product and cleaved capture probe is amplified. In some embodiments, amplification is isothermal. In some embodiments, amplification is not isothermal. Amplification can be performed using any of the methods described herein such as, but not limited to, a strand-displacement amplification reaction, a rolling circle amplification reaction, a ligase chain reaction, a transcription-mediated amplification reaction, an isothermal amplification reaction, and/or a loop-mediated amplification reaction. In some embodiments, amplifying the ligation product creates an amplified ligation product that includes (i) all or part of sequence of the ligation product specifically bound to the capture domain, or a complement thereof, and (ii) all or a part of the sequence of the spatial barcode, or a complement thereof.

In some embodiments, the methods described herein can further include determining (i) all or a part of the sequence of the ligation product, or a complement thereof, and (ii) the sequence of the first spatial barcode, or a complement thereof and, optionally, the sequence of the second spatial barcode, or a complement thereof. In some embodiments, the method further includes using the determined sequence of (i) and (ii) to determine the location the target nucleic acid in the biological sample. In some embodiments, the determining comprises sequencing. In some embodiments, the methods described herein further comprise using the combination of the determined sequence of the first spatial barcode, or a complement thereof, and, optionally, the determined sequence of the second spatial barcode, or a complement thereof, to determine the location of the target nucleic acid in the biological sample. In some embodiments, the first capture probe, and optionally, the second capture probe further comprise a quality control (QC) sequence.

### (d) Multiple Barcoding of Nucleic Acid Analytes

In some embodiments, the methods, devices, compositions, and systems described herein use multiple barcodes to detect a target nucleic acid analyte. In some embodiments, the methods described herein include providing an array comprising a first plurality of capture probes, wherein a first capture probe of the first plurality of capture probes comprises (i) a first capture domain, (ii) a first spatial barcode, and (iii) a ligation domain substantially complementary to a portion of a first splint oligonucleotide of a plurality of first splint oligonucleotides, and optionally, a second plurality of capture probes, wherein a second capture probe of the second plurality of capture probes comprises (iv) a second ligation domain substantially complementary to a portion of the first splint oligonucleotide, (v) a second spatial barcode, and (vi) a third ligation domain substantially complementary to a portion of a second splint oligonucleotide of a plurality of second splint oligonucleotides; (b) hybridizing a first probe oligonucleotide and a second probe oligonucleotide to the target nucleic acid, wherein the first probe oligonucleotide and the second probe oligonucleotide each comprise a sequence that is substantially complementary to a first sequence and a second sequence of the target nucleic acid, respectively, and wherein the first probe oligonucleotide comprises a first capture probe binding domain; (c) coupling the first probe oligonucleotide and the second probe oligonucleotide, thereby generating a ligation product; (d) releasing the first capture probe, and optionally, the second capture probe from the array; and (e) hybridizing the first capture domain of the first capture probe to the first capture probe binding domain of the ligation product, thereby spatially barcoding the target nucleic acid.

In some embodiments, the methods further comprise contacting the biological sample with the first plurality of splint oligonucleotides, wherein the first splint oligonucleotide comprises: (i) a sequence complementary to the ligation domain and (ii) a sequence complementary to the second ligation domain. In some embodiments, the methods further comprise hybridizing the first splint oligonucleotide to (i) the ligation domain and (ii) the second ligation domain.

In some embodiments, capture probes (e.g., a first capture probe and a second capture probe, a second capture probe and a third capture probe, etc.) are coupled with the aid of a splint oligonucleotide. In some embodiments, a first splint oligonucleotide includes a sequence that is substantially complementary to the ligation domain of the first capture probe and a sequence that is substantially complementary to the second ligation domain of the second capture probe. The splint oligonucleotide can be between 10 and 100 (e.g., about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100) nucleotides in length. In some embodiments, the first splint oligonucleotide aids in coupling (e.g., ligation) of the first capture probe and the second capture probe. Methods including a splint oligonucleotide have been described in U.S. Patent Pub. No. 2019/0055594A1.

In some embodiments, the methods described herein further comprise releasing a third capture probe of a plurality of third capture probes from the array, wherein the third capture probe of the plurality of third capture probes comprises (i) a fourth ligation domain substantially complementary to a portion of the second splint oligonucleotide, (ii) a third spatial barcode, and (iii) a fifth ligation domain substantially complementary to a portion of a third splint oligonucleotide of a third plurality of splint oligonucleotides. In some embodiments, the method further comprises contacting the biological sample with the second plurality of splint oligonucleotides, where the second splint oligonucleotide comprises (i) a sequence substantially complementary to the third ligation domain and (ii) a sequence substantially complementary to the fourth ligation domain. In some embodiments, the method further comprises hybridizing the second splint oligonucleotide to (i) the third ligation domain and (ii) the fourth ligation domain.

In some embodiments, the methods further comprise releasing a fourth capture probe of a plurality of fourth capture probes from the array, wherein the fourth capture probe of the plurality of fourth capture probes comprises (i) a sixth ligation domain substantially complementary to a portion of the third splint oligonucleotide, (ii) a fourth spatial barcode, and (iii) a ligation domain substantially complementary to a portion of a fourth splint oligonucleotide of a fourth plurality of splint oligonucleotides. In some embodiments, the method further comprises contacting the biological sample with the third plurality of splint oligonucleotides, wherein the third splint oligonucleotide comprises: (i) a sequence complementary to the fifth ligation domain and (ii) a sequence complementary to the sixth ligation domain.

In some embodiments, the release of the capture probes from the array can be performed using any of the methods of removal/cleavage described herein. In some embodiments, the first plurality of capture probes, and optionally, the second plurality of capture probes, the third plurality of capture probes, and the fourth plurality of capture probes are released from the array simultaneously. In some embodiments, the first plurality of capture probes, and optionally, the second plurality of capture probes, the third plurality of capture probes, and the fourth plurality of capture probes are released from the array sequentially. In some embodiments, the first plurality of capture probes, the second plurality of capture probes, the third plurality of capture probes, and the fourth plurality of capture probes are released from the array via denaturation. In some embodiments, the releasing of the first plurality of capture probes, the second plurality of capture probes, the third plurality of capture probes, and/or the fourth plurality of capture probes comprises cleaving the capture probes from the array. In some embodiments, the first plurality of capture probes, the second plurality of capture probes, the third plurality of capture probes, and the fourth plurality of capture probes comprise a cleavage domain. In some embodiments, the cleavage domain is a photocleavable cleavage domain. In some embodiments, the cleavage domain is a restriction enzyme site. In some embodiments, the cleavage domain for the first plurality of capture probes, the second plurality of capture probes, the third plurality of capture probes, and the fourth plurality of capture probes is the same. In some embodiments, the cleavage domain for the first plurality of capture probes, the second plurality of capture probes, the third plurality of capture probes, and the fourth plurality of capture probes is different for each plurality of capture probes.

In some embodiments, the first plurality of splint oligonucleotides, the second plurality of splint oligonucleotides, and the third plurality of splint oligonucleotides are contacted with the biological sample simultaneously. In some embodiments, the first plurality of splint oligonucleotides, the second plurality of splint oligonucleotides, and the third plurality of splint oligonucleotides are contacted with the biological sample sequentially. In some embodiments, the first plurality of splint oligonucleotides, the second plurality of splint oligonucleotides, and the third plurality of splint oligonucleotides comprise the same sequence. In some embodiments, the first plurality of splint oligonucleotides, the second plurality of splint oligonucleotides, and the third plurality of splint oligonucleotides each comprise a different sequence.

In some embodiments, after the step of hybridizing the capture probes to the target nucleic acid (e.g., mRNA, ligation product), a wash step is performed to remove unbound capture probes. The wash step can be performed using any of the wash methods and solutions described herein. In some embodiments, after the washing step, the first and second capture probes are bound to (e.g., hybridized to) the target nucleic acid (e.g., mRNA, ligation product), and the splint oligonucleotide is bound to (e.g., hybridized to) the first and second oligonucleotides (e.g., at portions of the first and second probes that are not bound to the nucleic acid). In some embodiments, the first plurality of capture probes, the second plurality of capture probes, the third plurality of capture probes, and the fourth plurality of capture probes, and/or the first plurality of splint oligonucleotides, the second plurality of splint oligonucleotides, and the third plurality of splint oligonucleotides are added to the biological sample at the same time. In some embodiments, the first plurality of capture probes, the second plurality of capture probes, the third plurality of capture probes, and the fourth plurality of capture probes are added at a first time point, and the first plurality of splint oligonucleotides, the second plurality of splint oligonucleotides, and the third plurality of splint oligonucleotides are added to the biological sample at a second time point.

### (e) Selective Release of Barcoded Capture Probes in Regions of Interest

A biological sample can have regions that show morphological feature(s) that may indicate the presence of disease or the development of a disease phenotype. For example, morphological features at a specific site within a tumor biopsy sample can indicate the aggressiveness, therapeutic resistance, metastatic potential, migration, stage, diagnosis, and/or prognosis of cancer in a subject. A change in the morphological features at a specific site within a tumor biopsy sample often correlate with a change in the level or expression of an analyte in a cell within the specific site, which can, in turn, be used to provide information regarding the aggressiveness, therapeutic resistance, metastatic potential, migration, stage, diagnosis, and/or prognosis of cancer in a subject. A region or area within a biological sample that is selected for specific analysis (e.g., a region in a biological sample that has morphological features of interest) is often described as "a region of interest."

A region of interest in a biological sample can be used to analyze a specific area of interest within a biological sample, and thereby, focus experimentation and data gathering to a specific region of a biological sample (rather than an entire biological sample). This results in, for example, reduced costs and increased time efficiency of the analysis of a biological sample.

Further, in some instances, more than one region of interest is identified in a biological sample. For instance, a biological sample can include at least one, two, three, four, or more regions of interest and so on. Thus, while a "first" and "second" region are described here, it is understood that multiple regions (e.g., greater than 2; e.g., at least 3, 4, 5, 6, 7, 8, 9, 10, 11, 12, 13, 14, 15, or more) can be used in a single sample. In some embodiments, a first region of interest can be confined to a first portion of a biological sample, and a second region can be confined to a second portion of the biological sample.

A region of interest can be identified in a biological sample using a variety of different techniques, e.g., expansion microscopy, bright field microscopy, dark field microscopy, phase contrast microscopy, electron microscopy, fluorescence microscopy, reflection microscopy, interference microscopy, confocal microscopy, and visual identification (e.g., by eye), and combinations thereof. For example, the staining and imaging of a biological sample can be performed to identify a region of interest. In some examples, the region of interest can correspond to a specific structure of cytoarchitecture. In some embodiments, a biological sample can be stained prior to visualization to provide contrast between the different regions of the biological sample. The type of stain can be chosen depending on the type of biological sample and the region of the cells to be stained. In some embodiments, more than one stain can be used to visualize different aspects of the biological sample, e.g., different regions of the sample, specific cell structures (e.g., organelles), or different cell types. In other embodiments, the biological sample can be visualized or imaged without staining the biological sample.

In some embodiments, staining and imaging a biological sample prior to contacting the biological sample with a spatial array is performed to select samples for spatial analysis. In some embodiments, the staining includes applying a fiducial marker as described herein, including fluorescent, radioactive, chemiluminescent, or colorimetric detectable markers. In some embodiments, the staining and imaging of biological samples allows the user to identify the specific sample (or region of interest) the user wishes to assess.

Non-limiting examples of methods of identifying the first region of interest and the second region that may or may not be of interest also include identifying manually (e.g., visual inspection as described above) or using a tissue detection machine learning module, for example HALO AI (Indicia Labs) and ONCOTOPIX (Visiopharm), and as described in Tomita et al. (JAMA Network Open. 2019, 2(11) e1914645), Bychkov et al. (Scientific Reports, 2018, 8:3395), and Tsai and Tao, Electronics 2021, 10, 1662. In some embodiments, identifying a first region of interest and a second region include visual inspection following staining.

In some embodiments, the trained machine learning module includes at least one of a supervised learning module, a semi supervised learning module, an unsupervised learning module, a regression analysis module, a reinforcement learning module, a self-learning module, a feature learning module, a sparse dictionary learning module, an anomaly detection module, a generative adversarial network, a convolutional neural network, or an association rules module. For example, the first region of interest and the second region are identified by a supervised machine learning module. In another example, the first region of interest and the second region are identified by a tissue detection machine learning module.

In some examples, an array (e.g., any of the exemplary arrays described herein) can be contacted with only a portion of a biological sample (e.g., a cell, a tissue section, or a region of interest). In some examples, a biological sample is contacted with only a portion of an array (e.g., any of the exemplary arrays described herein). In some embodiments, capture probes on an array corresponding to regions of interest of a biological sample (e.g., proximal to the region of interest) can be selectively cleaved and analyzed. For example, capture probes on an array may be deactivated or eliminated outside of areas corresponding to regions of interest of a biological sample. In some embodiments, capture probes including a photocleavable bond and on the array in areas corresponding to regions of interest of a biological sample can be selectively cleaved by using light. A mirror, mirror array, a lens, a moving stage, and/or a photomask can be used to direct the light to regions of the array that correspond areas outside one or more regions of interest in the biological sample. Some embodiments include deactivating or eliminating capture probes, e.g., capture probes comprising a photocleavable bond as described herein, using light. In some embodiments, a laser, e.g., a scanning laser, can be used to deactivate or eliminate capture probes. In some embodiments, the eliminated member of the plurality of capture probes can be washed away. In some embodiments, regions of interest can be labeled with different heavy metals, and a laser can sequentially ablate these regions of interest before mass spectrometry identification. A laser can, for example, deactivate or eliminate capture probes through UV light destruction of DNA, heat, inducing a chemical reaction that prevents the capture probes from moving to the next step, inducing photocleavage of a photocleavable bond, or a combination thereof. In some examples, a portion of the array can be deactivated such that it does not interact with the analytes in the biological sample (e.g., optical deactivation, chemical deactivation, heat deactivation, or blocking of the capture probes in the array (e.g., using blocking probes)). In some examples, a region of interest can be removed from a biological sample and then the region of interest can be contacted to the array (e.g., any of the arrays described herein). A region of interest can be removed from a biological sample using microsurgery, laser capture microdissection, chunking, a microtome, dicing, trypsinization, labelling, and/or fluorescence-assisted cell sorting, and the like.

In some examples, a region of interest can be permeabilized or lysed while areas outside the region of interest are not permeabilized or lysed (e.g., Kashyap et al. Sci Rep. 2016; 6: 29579). For example, in some embodiments, a region of interest can be contacted with a hydrogel comprising a permeabilization or lysing reagent. In some embodiments, the area(s) outside the region of interest are not contacted with the hydrogel comprising the permeabilization or lysing reagent.

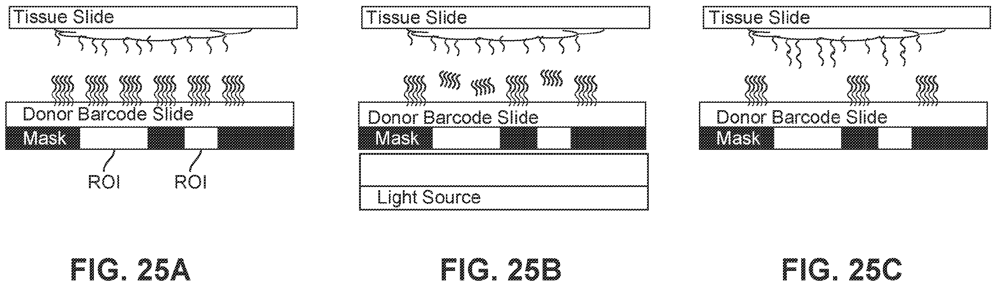

After identifying regions in a biological sample, in some embodiments, the methods disclosed include applying a mask to the substrate containing the array. In some embodiments, the mask is applied to a second surface of the substrate, while the array containing the plurality of capture probes is affixed to a first surface of the substrate. As shown in **FIGs. 25A-C**, the mask is applied to the side of the substrate (e.g., gene expression slide or slide) that is distal to the capture probes on the substrate. As used here, a mask is a composition that can be placed on the substrate, e.g., between the substrate's probes and an activation source (e.g., a light source) that reduces or prevents the interaction between the capture probes and the activation source.

A wide variety of different compositions can be used for the mask so long as it reduces or prevents the activation source (e.g., light) from activating (e.g., cleaving) the capture probes. Exemplary materials for the mask include, but are not limited to, hydrogels, films, membranes, plastics (including e.g., acrylics, polystyrene, copolymers of styrene and other materials, polypropylene, polyethylene, polybutylene, polyurethanes, Teflon^{™}, cyclic olefins, polyimides etc.), nylon, ceramics, resins, Zeonor, silica or silica-based materials including silicon and modified silicon, carbon, metals, inorganic glasses, optical fiber bundles, and polymers, such as polystyrene, cyclic olefin copolymers (COCs), cyclic olefin polymers (COPs), polypropylene, polyethylene polycarbonate, or combinations thereof.

The mask can prevent cleavage of some of the capture probes on an array. In doing so, the mask can take on any shape (regular, irregular, drawn, etc.). For instance, the mask can cover regions outside of a region of interest, thereby allowing light into a region of interest to promote capture probe cleavage.

After applying the mask to the distal side of the substrate (e.g., the substrate as shown in **FIGs. 25A-C**), an activation source is applied to the substrate or array containing the capture probes in order to release them from the substrate. In some instances, the activation source is light. In some instances, the activation source is visible light. In some instances, the activation source is not visible light (e.g., infrared light or ultraviolet light). In some embodiments, a light source can release a capture probe with a photo-cleavable linker (e.g., a photo-sensitive chemical bond). Light sources can be targeted to a region of interest on the array (e.g., a digital micro-device).

In some embodiments, a heat source can release a capture probe with a heat-cleavable linker. In some embodiments, the capture probe is released by applying a heated first reagent medium of at least 85°C, e.g., at least 90, 91, 92, 93, 94, 95, 96, 97, 98, or 99°C.

In some instances, the activation source is heat, and the mask reduces or prevents heat from permeating through it to interact with the capture probes. Heating can result in degradation of the cleavage domain and release of the attached capture probe from the array feature. In some embodiments, laser radiation is used to heat and degrade cleavage domains of capture probes at specific locations. In some embodiments, the cleavage domain can be an ultrasonic cleavage domain. For example, ultrasonic cleavage can depend on nucleotide sequence, length, pH, ionic strength, temperature, and the ultrasonic frequency.

Selective release of barcoded capture probes in one or more regions of interest includes releasing any of the barcoded capture probes and embodiments thereof described herein.

### (f) Exemplary Capture Probes

As described herein, a substrate can include an array comprising a plurality of capture probes, each having a capture domain and a spatial barcode. The capture domain interacts with the analyte (e.g., target nucleic acid) or analyte derived molecule, such as a ligation product. The spatial barcode is a sequence unique to the location on the substrate that can be used to identify the location on the substrate. In addition, the capture probes can have a primer sequence, which in some instances is more proximal to the substrate than the capture domain and spatial barcode. For example, in some instances, the capture probes can have a primer sequence which is 3' to the capture domain and spatial barcode. In some instances, the capture probes can have a primer sequence which is 5' to the capture domain and spatial barcode.

In some embodiments, a capture probe as described herein can be cleaved (e.g., using a first reagent medium), thereby allowing for release of the capture probe in order to migrate to the biological sample. In some embodiments, the capture probe is released from the substrate. The step of releasing the capture probe from the surface of the substrate can be achieved in a number of ways. In some embodiments, a capture probe is released from the array by nucleic acid cleavage and/or by denaturation (e.g., by heating to denature a double-stranded molecule).

In some instances, the release is performed by applying a first reagent medium. The first reagent medium can include a salt, polyethylene glycol (PEG), and/or a detergent selected from sodium dodecyl sulfate (SDS), sarkosyl, saponin, a nonionic surfactant, a polysorbate surfactant, or any combination thereof.

In some instances, the release of the capture probe from the array and the coupling of the released capture probe with the ligation product in the biological sample occurs in about 30 minutes or less, about 15 minutes or less, about 10 minutes or less, about 5 minutes or less, or about 1 minute, or less.

In some embodiments, the capture probe is released from the surface of the substrate (e.g., array) by physical means. Methods for disrupting the interaction between nucleic acid molecules are known in the art. A straightforward method for releasing the DNA molecules (i.e., of stripping the array of capture probes) is to use a solution (e.g., a first reagent medium) that interferes with the bonds between nucleotides. In some embodiments, the capture probe is released by applying heated first reagent medium of at least 85°C, e.g., at least 90, 91, 92, 93, 94, 95, 96, 97, 98, or 99°C. In some embodiments, the first reagent medium including salts, surfactants, etc. that can further destabilize the interaction between the array and the capture probe.

In some embodiments, where the capture probe includes a cleavage domain, the capture probe is released from the surface of the substrate by cleavage. For example, the cleavage domain of the capture probe can be cleaved by any of the methods described herein. In some embodiments, the capture probe is released from the surface of the substrate, e.g., via cleavage of a cleavage domain in the capture probe.

In some embodiments, the capture probes can be complementary a common nucleic acid sequence such as a poly(A) tail. In some embodiments, the capture probes can be complementary to a single analyte (e.g., a single gene). In some embodiments, the capture probes can be complementary to one or more analytes (e.g., analytes in a family of genes). In some embodiments, the capture probes can be for a panel of genes associated with a disease (e.g., cancer, Alzheimer's disease, Parkinson's disease).

A "cleavable linker" or "cleavage domain," as described herein, cleaves a capture probe, thereby releasing one or more segments or regions of the capture probe (e.g., capture domain, primer, spatial barcodes and/or UMIs). In some instances, the capture probe can be releasably, cleavably, or reversibly attached to a feature, or some other substrate, so that spatial barcodes and/or UMIs can be released or be releasable through cleavage of a linkage between the capture probe and the feature, or released through degradation of the underlying substrate or chemical substrate, allowing the spatial barcode(s) and/or UMI(s) of the cleaved capture probe to be accessed or be accessible by other reagents, or both.

Each capture probe can optionally include at least one cleavage domain. The cleavage domain represents the portion of the probe that is used to reversibly attach the probe to an array feature, as will be described further herein. Further, one or more segments or regions of the capture probe can optionally be released from the array feature by cleavage of the cleavage domain. As an example, spatial barcodes and/or universal molecular identifiers (UMIs) can be released by cleavage of the cleavage domain.

In some embodiments, the cleavage domain linking the capture probe to a feature is a bond capable of cleavage by an enzyme. An enzyme can be introduced to cleave the cleavage domain, resulting in release of the capture probe from the feature. As another example, heating can also result in degradation of the cleavage domain and release of the attached capture probe from the array feature. In some embodiments, laser radiation is used to heat and degrade cleavage domains of capture probes at specific locations. In some embodiments, the cleavage domain is a photo-sensitive chemical bond (e.g., a chemical bond that dissociates when exposed to light such as ultraviolet light). In some embodiments, the cleavage domain can be an ultrasonic cleavage domain. For example, ultrasonic cleavage can depend on nucleotide sequence, length, pH, ionic strength, temperature, and the ultrasonic frequency (e.g., 22 kHz, 44 kHz) (Grokhovsky, S.L., Specificity of DNA cleavage by ultrasound, Molecular Biology, 40(2), 276-283 (2006)).

Oligonucleotides with photo-sensitive chemical bonds (e.g., photo-cleavable linkers) have various advantages. They can be cleaved efficiently and rapidly (e.g., in nanoseconds and milliseconds). In some cases, photo-masks can be used such that only specific regions of the array are exposed to cleavable stimuli (e.g., exposure to UV light, exposure to light, exposure to heat induced by laser). When a photo-cleavable linker is used, the cleavable reaction is triggered by light, and can be highly selective to the linker and consequently biorthogonal. Typically, wavelength absorption for the photocleavable linker is located in the near-UV range of the spectrum. In some embodiments, λₘₐₓ of the photocleavable linker is from about 300 nm to about 400 nm, or from about 310 nm to about 365 nm. In some embodiments, λmax of the photocleavable linker is about 300 nm, about 312 nm, about 325 nm, about 330 nm, about 340 nm, about 345 nm, about 355 nm, about 365 nm, or about 400 nm.

Non-limiting examples of a photo-sensitive chemical bond that can be used in a cleavage domain include those described in Leriche et al. Bioorg Med Chem. 2012 Jan 15;20(2):571-82 and U.S. Publication No. 2017/0275669. For example, linkers that comprise photo-sensitive chemical bonds include 3-amino-3-(2-nitrophenyl)propionic acid (ANP), phenacyl ester derivatives, 8-quinolinyl benzenesulfonate, dicoumarin, 6-bromo-7-alkixycoumarin-4-ylmethoxycarbonyl, a bimane-based linker, and a bis-arylhydrazone based linker. In some embodiments, the photo-sensitive bond is part of a cleavable linker such as an ortho-nitrobenzyl (ONB) linker below: 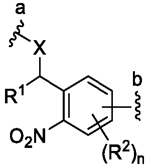 wherein:
X is selected from O and NH;
R¹ is selected from H and C₁₋₃ alkyl;
R² is selected from H and C₁₋₃ alkoxy;
n is 1, 2, or 3; and
a and b each represent either the point of attachment of the linker to the substrate, or the point of attachment of the linker to the capture probe.

In some embodiments, at least one spacer is included between the substrate and the ortho-nitrobenzyl (ONB) linker, and at least one spacer is included between the ortho-nitrobenzyl (ONB) linker and the capture probe. In some aspects of these embodiments, the spacer comprises at least one group selected from C1-6 alkylene, C2-6 alkenylene, C2-6 alkynylene, C=O, O, S, NH, -(C=O)O-, -(C=O)NH-, -S-S-, ethylene glycol, polyethyleneglycol, propylene glycol, and polypropyleneglycol, or any combination thereof. In some embodiments, X is O. In some embodiments, X is NH. In some embodiments, R¹ is H. In some embodiments, R¹ is C₁₋₃ alkyl. In some embodiments, R¹ is methyl. In some embodiments, R² is H. In some embodiments, R² is C₁₋₃ alkoxy. In some embodiments, R² is methoxy. In some embodiments, R¹ is H and R² is H. In some embodiments, R¹ is H and R² is methoxy. In some embodiments, R¹ is methyl and R² is H. In some embodiments, R¹ is methyl and R² is methoxy.
In some embodiments, the photocleavable linker has formula: 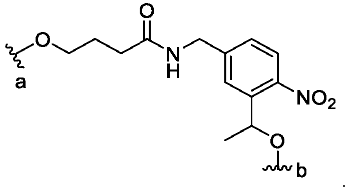 In some embodiments, the photocleavable linker has formula: 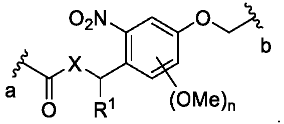 In some embodiments, the photocleavable linker has formula: 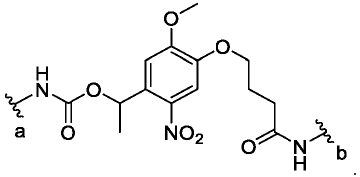 In some embodiments, the photocleavable linker has formula: 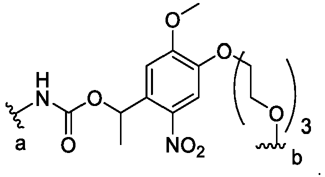 In some embodiments, the photocleavable linker has formula: 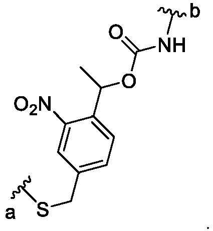 Without being bound to any particular theory, it is believed that excitation of the ortho-nitrobenzyl (ONB) linker leads to Norrish-type hydrogen abstraction in the γ-position, followed by formation of azinic acid, which is highly reactive and rearranges into nitroso compound, resulting in the complete cleavage of the linker, as shown on the following scheme: 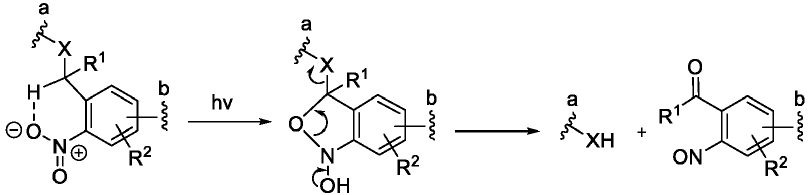 In some embodiments, the photocleavable linker is 3-amino-3-(2-nitrophenyl)propionic acid (ANP) linker: 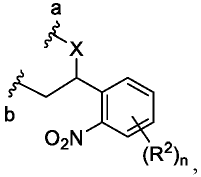 wherein X, R², n, a, and b are as described herein for the ortho-nitrobenzyl (ONB) linker.
In some embodiments, the photocleavable linker has formula: 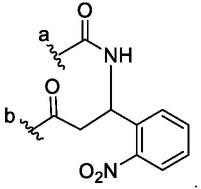 In some embodiments, the photocleavable linker is phenacyl ester linker: 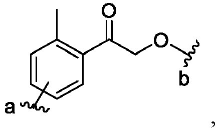 wherein a and b are as described herein for the ortho-nitrobenzyl (ONB) linker.

Other examples of photo-sensitive chemical bonds that can be used in a cleavage domain include halogenated nucleosides such as bromodeoxyuridine (BrdU). BrdU is an analog of thymidine that can be readily incorporated into oligonucleotides (e.g., in the cleavage domain of a capture probe), and is sensitive to UVB light (280-320 nm range). Upon exposure to UVB light, a photo-cleavage reaction occurs (e.g., at a nucleoside immediately 5' to the site of BrdU incorporation (Doddridge et al. Chem. Comm., 1998, 18:1997-1998 and Cook et al. Chemistry and Biology. 1999, 6:451-459)) that results in release of the capture probe from the feature.

Other examples of cleavage domains include labile chemical bonds such as, but not limited to, ester linkages (e.g., cleavable with an acid, a base, or hydroxylamine), a vicinal diol linkage (e.g., cleavable via sodium periodate), a Diels-Alder linkage (e.g., cleavable via heat), a sulfone linkage (e.g., cleavable via a base), a silyl ether linkage (e.g., cleavable via an acid), a glycosidic linkage (e.g., cleavable via an amylase), a peptide linkage (e.g., cleavable via a protease), an abasic or apurinic/apyrimidinic (AP) site (e.g., cleavable with an alkali or an AP endonuclease), or a phosphodiester linkage (e.g., cleavable via a nuclease (e.g., DNase)).

In some embodiments, the cleavage domain includes a sequence that is recognized by one or more enzymes capable of cleaving a nucleic acid molecule, e.g., capable of breaking the phosphodiester linkage between two or more nucleotides. A bond can be cleavable via other nucleic acid molecule targeting enzymes, such as restriction enzymes (e.g., restriction endonucleases). For example, the cleavage domain can include a restriction endonuclease (restriction enzyme) recognition sequence. Restriction enzymes cut double-stranded or single stranded DNA at specific recognition nucleotide sequences known as restriction sites. In some embodiments, a rare-cutting restriction enzyme, e.g., enzymes with a long recognition site (at least 8 base pairs in length), is used to reduce the possibility of cleaving elsewhere in the capture probe.

In some embodiments, the cleavage domain includes a poly(U) sequence which can be cleaved by a mixture of Uracil DNA glycosylase (UDG) and the DNA glycosylase-lyase Endonuclease VIII, commercially known as the USER^{™} enzyme. Releasable capture probes can be available for reaction once released. Thus, for example, an activatable capture probe can be activated by releasing the capture probes from a feature.

In some embodiments, where the capture probe is attached indirectly to a substrate, e.g., via a surface probe, the cleavage domain includes one or more mismatch nucleotides, so that the complementary parts of the surface probe and the capture probe are not 100% complementary (for example, the number of mismatched base pairs can be one, two, or three base pairs). Such a mismatch is recognized, e.g., by the MutY and T7 endonuclease I enzymes, which results in cleavage of the nucleic acid molecule at the position of the mismatch. As described herein a "surface probe" can be any moiety present on the surface of the substrate capable of attaching to an agent (e.g., a capture probe). In some embodiments, the surface probe is an oligonucleotide. In some embodiments, the surface probe is part of the capture probe.

In some embodiments, where the capture probe is attached to a feature indirectly, e.g., via a surface probe, the cleavage domain includes a nickase recognition site or sequence. Nickases are endonucleases which cleave only a single strand of a DNA duplex. Thus, the cleavage domain can include a nickase recognition site close to the 5' end of the surface probe (and/or the 5' end of the capture probe) such that cleavage of the surface probe or capture probe destabilizes the duplex between the surface probe and capture probe thereby releasing the capture probe) from the feature.

Nickase enzymes can also be used in some embodiments where the capture probe is attached to the feature directly. For example, the substrate can be contacted with a nucleic acid molecule that hybridizes to the cleavage domain of the capture probe to provide or reconstitute a nickase recognition site, e.g., a cleavage helper probe. Thus, contact with a nickase enzyme will result in cleavage of the cleavage domain thereby releasing the capture probe from the feature. Such cleavage helper probes can also be used to provide or reconstitute cleavage recognition sites for other cleavage enzymes, e.g., restriction enzymes.

Some nickases introduce single-stranded nicks only at particular sites on a DNA molecule, by binding to and recognizing a particular nucleotide recognition sequence. A number of naturally-occurring nickases have been discovered, of which at present the sequence recognition properties have been determined for at least four. Nickases are described in U.S. Patent No. 6,867,028. In general, any suitable nickase can be used to bind to a complementary nickase recognition site of a cleavage domain. Following use, the nickase enzyme can be removed from the assay or inactivated following release of the capture probes to prevent unwanted cleavage of the capture probes.

Examples of suitable capture domains that are not exclusively nucleic-acid based include, but are not limited to, proteins, peptides, aptamers, antigens, antibodies, and molecular analogs that mimic the functionality of any of the capture domains described herein.

In some embodiments, a cleavage domain is absent from the capture probe. Examples of substrates with attached capture probes lacking a cleavage domain are described for example in Macosko et al., (2015) Cell 161, 1202-1214.

In some embodiments, the region of the capture probe corresponding to the cleavage domain can be used for some other function. For example, an additional region for nucleic acid extension or amplification can be included where the cleavage domain would normally be positioned. In such embodiments, the region can supplement the functional domain or even exist as an additional functional domain. In some embodiments, the cleavage domain is present, but its use is optional.

In some embodiments, the capture probe is linked, (e.g., via a disulfide bond), to a feature. In some embodiments, the capture probe is linked to a feature via a propylene group (e.g., Spacer C3). A reducing agent can be added to the first reagent medium in order to break the various disulfide bonds, resulting in release of the capture probe including the spatial barcode sequence. In another example, heating can also result in degradation and release of the attached capture probe. In some embodiments, the heating is done by laser (e.g., laser ablation) and features at specific locations can be degraded. In addition to thermally cleavable bonds, disulfide bonds, photo-sensitive bonds, and UV sensitive bonds, other non-limiting examples of labile bonds that can be coupled to a capture probe (e.g., spatial barcode) include an ester linkage (e.g., cleavable with an acid, a base, or hydroxylamine), a vicinal diol linkage (e.g., cleavable via sodium periodate), a Diels-Alder linkage (e.g., cleavable via heat), a sulfone linkage (e.g., cleavable via a base), a silyl ether linkage (e.g., cleavable via an acid), a glycosidic linkage (e.g., cleavable via an amylase), a peptide linkage (e.g., cleavable via a protease), or a phosphodiester linkage (e.g., cleavable via a nuclease (e.g., DNase)).

In some embodiments, the cleavage domain includes a poly(U) sequence which can be cleaved by a mixture of Uracil DNA glycosylase (UDG) and the DNA glycosylase-lyase Endonuclease VIII, commercially known as the USER^{™} enzyme. In some embodiments, the cleavage domain can be a single U. In some embodiments, the cleavage domain can be an abasic site that can be cleaved with an abasic site-specific endonuclease (e.g., Endonuclease IV or Endonuclease VIII).

In some embodiments, the cleavage domain of the capture probe is a nucleotide sequence within the capture probe that is cleaved specifically, e.g., physically by light or heat, chemically, or enzymatically. The location of the cleavage domain within the capture probe will depend on whether or not the capture probe is immobilized on the substrate such that it has a free 3' end capable of functioning as an extension primer (e.g., by its 5' or 3' end). For example, if the capture probe is immobilized by its 5' end, the cleavage domain will be located 5' to the spatial barcode and/or UMI, and cleavage of said domain results in the release of part of the capture probe including the spatial barcode and/or UMI and the sequence 3' to the spatial barcode, and optionally part of the cleavage domain, from a feature. Alternatively, if the capture probe is immobilized by its 3' end, the cleavage domain will be located 3' to the capture domain (and spatial barcode) and cleavage of said domain results in the release of part of the capture probe including the spatial barcode and the sequence 3' to the spatial barcode from a feature. In some embodiments, cleavage results in partial removal of the cleavage domain. In some embodiments, cleavage results in complete removal of the cleavage domain, particularly when the capture probes are immobilized via their 3' end as the presence of a part of the cleavage domain can interfere with the hybridization of the capture domain and the target nucleic acid and/or its subsequent extension.

### (g) Kits and Compositions

In some embodiments, also provided herein are kits and compositions that include one or more reagents to spatially barcode a target nucleic acid in a biological sample.

Thus provided herein are kits including (a) an array comprising a first plurality of capture probes, wherein a first capture probe of the first plurality of capture probes comprises (i) a first spatial barcode and (ii) a first capture domain, and wherein the first capture probe is attached to a substrate by its 3' end, and a second plurality of capture probes, wherein a second capture probe of the second plurality of capture probes comprises (iii) a second spatial barcode and (iv) a second capture domain, and wherein the second capture probe is attached to the substrate by its 3' end; and (b) a plurality of probe pair oligonucleotides, wherein a probe pair oligonucleotide of the plurality of probe pair oligonucleotides comprises a first probe oligonucleotide and a second probe oligonucleotide, wherein the first probe oligonucleotide and the second probe oligonucleotide each comprise a sequence that is substantially complementary to a first sequence and a second sequence of the target nucleic acid, respectively, and wherein the first probe oligonucleotide comprises a first capture probe binding domain and the second probe oligonucleotide comprises a second capture probe binding domain.

Also provided herein are kits can including (a) an array comprising a first plurality of capture probes, wherein a first capture probe of the first plurality of capture probes comprises (i) a first capture domain, (ii) a first spatial barcode, and (iii) a ligation domain substantially complementary to a portion of a first splint oligonucleotide of a plurality of first splint oligonucleotides, and optionally, a second plurality of capture probes, wherein a second capture probe of the second plurality of capture probes comprises (iv) a second ligation domain substantially complementary to a portion of the first splint oligonucleotide, (v) a second spatial barcode, and (vi) a third ligation domain substantially complementary to a portion of the second splint oligonucleotide of a plurality of second splint oligonucleotides; (b) a plurality of probe pair oligonucleotides comprising a first probe oligonucleotide and a second probe oligonucleotide, wherein the first probe oligonucleotide and the second probe oligonucleotide each comprise a sequence that is substantially complementary to a first sequence and a second sequence of the target nucleic acid, respectively, and wherein the first probe oligonucleotide comprises a first capture probe binding domain; (c) a plurality of first splint oligonucleotides, wherein a first splint oligonucleotide of the plurality of splint oligonucleotides comprises a sequence substantially complementary to the ligation domain of the first capture probe and a sequence substantially complementary to the second ligation domain of the second capture probe; and (d) a ligase.

In some embodiments, a kit can further include instructions for performing any of the methods or steps provided herein. In some embodiments, a kit can include a substrate with one or more capture probes comprising a spatial barcode and a capture domain that captures a target nucleic acid. In some embodiments, the kit includes means for sequencing the nucleic acid. In some embodiments, the kit includes reagents to detect and sequence the nucleic acid.

Also provided herein are compositions including: (a) an array including a first plurality of capture probes, where a first capture probe of the first plurality of capture probes includes: (i) a first spatial barcode and (ii) a first capture domain, and where the first capture probe is attached to a substrate by its 3' end, and a second plurality of capture probes, where a second capture probe of the second plurality of capture probes includes: (iii) a second spatial barcode and (iv) a second capture domain, and where the second capture probe is attached to the substrate by its 3' end; and (b) a plurality of probe pair oligonucleotides, where a probe pair oligonucleotide of the plurality of probe pair oligonucleotides includes a first probe oligonucleotide and a second probe oligonucleotide, where the first probe oligonucleotide and the second probe oligonucleotide each include a sequence that is substantially complementary to a first sequence and a second sequence of the target nucleic acid, respectively, and where the first probe oligonucleotide includes a first capture probe binding domain and the second probe oligonucleotide includes a second capture probe binding domain.

In some embodiments, the first probe oligonucleotide and the second probe oligonucleotide are hybridized to the target nucleic acid. In some embodiments, the first probe and the second probe are ligated, thereby generating a ligation product. In some embodiments, the target nucleic acid is removed from the ligation product.

In some embodiments, the first capture probe, and optionally, the second capture probe are released from the array. In some embodiments, the first capture probe, and optionally, the second capture probe hybridize to the first capture probe binding domain and the second capture probe binding domain, respectively.

In some embodiments, the target nucleic acid is removed from the ligation product.

In some embodiments, the first capture probe is extended, thereby generating a complement of the ligation product.

In some embodiments, the complement of the ligation product is ligated to the second capture probe.

In some embodiments, the composition includes one or more of an RNase, a DNase, a protease, a lipase, and combinations thereof. In some embodiments, the protease is one or more of Proteinase K, pepsin, and collagenase. In some embodiments, the composition includes a ligase and a polymerase. In some embodiments, the ligase is one or more of a T4 RNA ligase (Rnl2), a PBCV-1 DNA ligase, a *Chlorella* virus DNA ligase, a single-stranded DNA ligase, or a T4 DNA ligase.

Also provided herein are compositions including: (a) an array including a first plurality of capture probes, where a first capture probe of the first plurality of capture probes includes: (i) a first capture domain, (ii) a first spatial barcode, and (iii) a ligation domain substantially complementary to a portion of a first splint oligonucleotide of a plurality of first splint oligonucleotides, and optionally, a second plurality of capture probes, where a second capture probe includes: (iv) a second ligation domain substantially complementary to a portion of the first splint oligonucleotide, (v) a second spatial barcode, and (vi) a third ligation domain substantially complementary to a portion of the second splint oligonucleotide of a plurality of second splint oligonucleotides; (b) a plurality of probe pair oligonucleotides including a first probe oligonucleotide and a second probe oligonucleotide, where the first probe oligonucleotide and the second probe oligonucleotide each include a sequence that is substantially complementary to a first sequence and a second sequence of the target nucleic acid, respectively, and where the first probe oligonucleotide includes a first capture probe binding domain.

In some embodiments, the first probe oligonucleotide and the second probe oligonucleotide are hybridized to the target nucleic acid.

In some embodiments, the first probe oligonucleotide and the second probe oligonucleotide are ligated, thereby generating a ligation product. In some embodiments, the target nucleic acid is removed from the ligation product.

In some embodiments, the composition includes a plurality of first splint oligonucleotides, where a first splint oligonucleotide of the plurality of first splint oligonucleotides includes a sequence substantially complementary to the ligation domain of the first capture probe and a sequence substantially complementary to the second ligation domain of the second capture probe.

In some embodiments, the first splint is hybridized to the ligation domain of the first capture probe and to the second ligation domain of the second capture probe.

In some embodiments, the composition includes a plurality of second splint oligonucleotides, where a second splint oligonucleotide of the plurality of second splint oligonucleotides includes a sequence substantially complementary to a third ligation domain of the second capture probe and a sequence substantially complementary to a fourth ligation domain of a third capture probe of a plurality of third capture probes.

In some embodiments, the second splint oligonucleotide is hybridized to the third ligation domain of the second capture probe and to the fourth ligation domain of the third capture probe.

In some embodiments, the composition includes a plurality of third splint oligonucleotides, where a third splint oligonucleotide of a plurality of third splint oligonucleotides includes a sequence substantially complementary to a fifth ligation domain of the third capture probe and a sequence substantially complementary to a sixth ligation domain of a fourth capture probe of a plurality of fourth capture probes.

In some embodiments, the third splint oligonucleotide is hybridized to the fifth ligation domain of the third capture probe and to the sixth ligation domain of the fourth capture probe.

In some embodiments, the first capture probe is ligated to the second capture probe, and optionally, the second capture probe is ligated to the third capture probe, and the third capture probe is ligated to the fourth capture probe.

In some embodiments, the first capture probe is extended, thereby generating a complement of the ligation product.

In some embodiments, the composition includes one or more of an RNase, a DNase, a protease, a lipase, and combinations thereof. In some embodiments, the protease is one or more of Proteinase K, pepsin, and collagenase. In some embodiments, the composition includes a ligase and a polymerase. In some embodiments, the ligase is one or more of a T4 RNA ligase (Rnl2), a PBCV-1 DNA ligase, a *Chlorella* virus DNA ligase, a single-stranded DNA ligase, or a T4 DNA ligase.

### EXAMPLES

### Example 1 - High-Density Barcoding of a Nucleic Acid Analyte

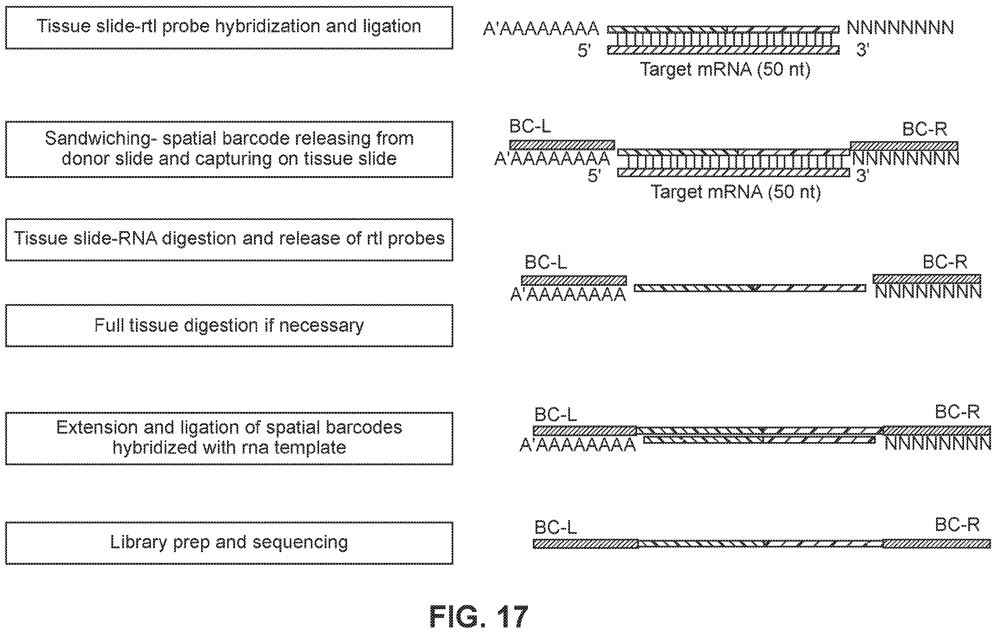

**FIG. 17** is an exemplary workflow of the methods described herein.

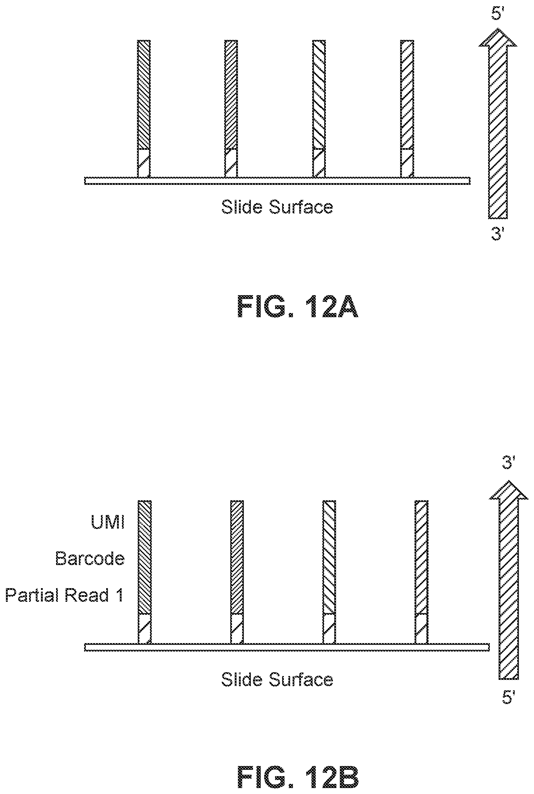

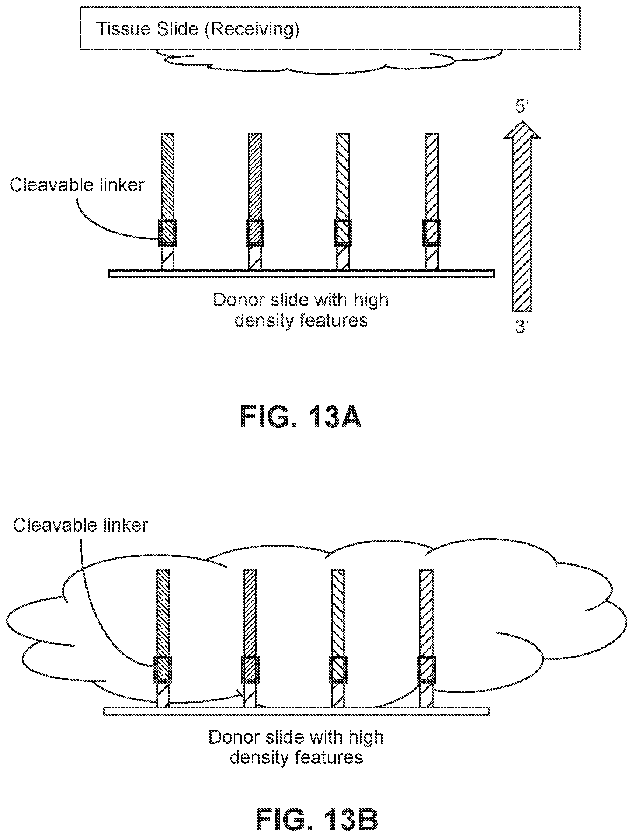

An array of capture probes is provided, wherein a capture probe includes a spatial barcode, a UMI, and a capture domain **(****FIGs. 12A-12B****).** The array can include a first plurality of capture probes and a second plurality of capture probes. In some examples, the spatial barcode of a first capture probe and a second capture probe can include the same nucleic acid sequence. In some examples, the spatial barcode of the first capture probe and the second capture probe include different nucleic acid sequences. In some examples, the capture domain of the first capture probe (e.g., the first capture domain) hybridizes to the first capture probe binding domain of a first probe oligonucleotide. In some examples, the capture domain of the second capture probe (e.g., the second capture domain) hybridizes to the second capture probe binding domain of a second probe oligonucleotide. A biological sample can be provided. For example, the biological sample can be mounted on a substrate **(****FIG. 13A****)** or provided directly on the array **(****FIG.13B****)** including the pluralities of capture probes.

A set of probes (e.g., probe oligonucleotides) can be contacted with the biological sample, where the probes include nucleic acid sequences that hybridize to target nucleic acid molecules (e.g., DNA, RNA, mRNA, etc.). In some examples, the probes hybridize to adjacent sequences in the target nucleic acid. In some examples, the probes hybridize to non-adjacent sequences on the target nucleic acid and a gap-filling reaction is performed to extend the first or second probe. The first probe oligonucleotide and the second probe oligonucleotide are ligated to one another while hybridized to the target nucleic acid and, after ligation, an endonuclease such as an RNase (e.g., any of the RNases described herein) degrades the target nucleic acid hybridized to the ligated probes (e.g., a ligation product), leaving a single-stranded DNA molecule (e.g., the ligation product).

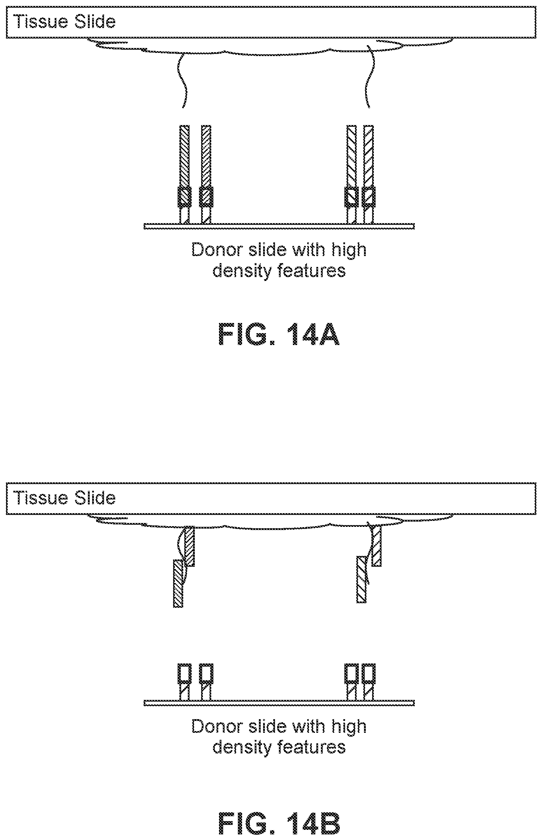

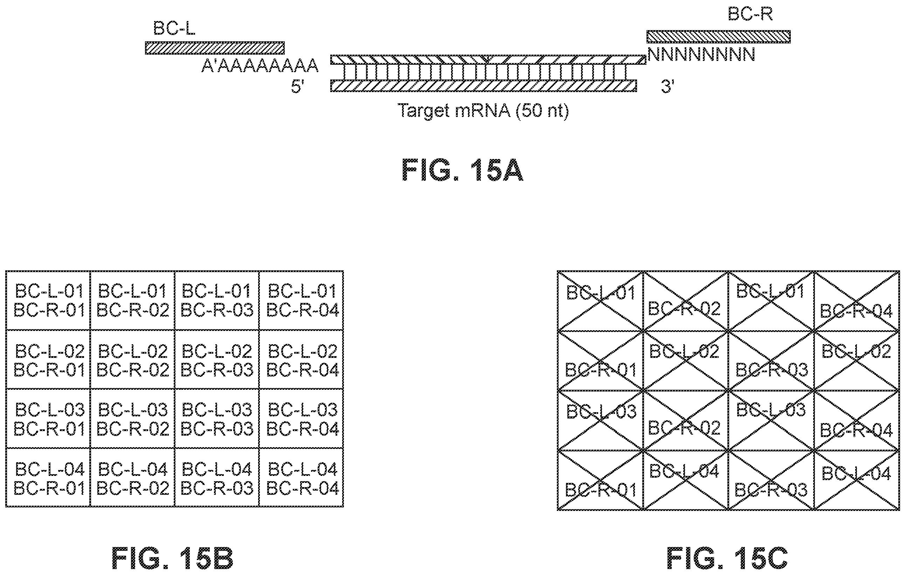

In some examples, the capture probes on the substrate are attached via their 3' end, thus enabling high-density spatial arrays to be synthesized **(****FIG. 14A****).** The capture probes are then released (e.g., cleaved) from the array, where they migrate (either passively or actively) towards the biological sample and hybridize to the ligation product via hybridization of their respective capture domains to the capture probe binding domains of the ligation product **(****FIG. 14B****).** **FIG. 15A** shows an embodiment where the first capture probe and the second capture probe are hybridized to the capture probe binding domains of the ligation product while the target nucleic acid is still hybridized to the ligation product, however, in some examples, the first capture probe and the second capture probe hybridize to the capture probe binding domains after the target nucleic acid is removed.

**FIG. 15B** shows an exemplary high-density barcoding pattern where each box represents a feature (e.g., as described herein). For example, the same spatial barcode e.g., BC-L-01 can be used across the first row in combination with a different BC-R spatial barcode (e.g., BC-R-01; BC-R-02; BC-R-03; BC-R-04; etc.). Similarly, the same spatial barcode e.g., BC-R-01 can be used down the first column in combination with a different BC-L spatial barcode (e.g., BC-L-01; BC-L-02; BC-L-03; BC-L04; etc.). **FIG. 15C** shows an exemplary high-density barcoding pattern where each box represents a feature, however, only a single barcode is used per feature. When the capture probes are released diffusion of the barcodes occurs and they can hybridize to the capture probe binding domains of the ligation product.

After hybridization of the ligation product to the capture probes, an extension step is performed, generating a complement of the ligation product having components of the capture probe (e.g., primer, spatial barcode, capture domain, etc.) and the ligation product. The extended product is then further processed and evaluated (e.g., via sequencing) **(****FIG. 17****).**

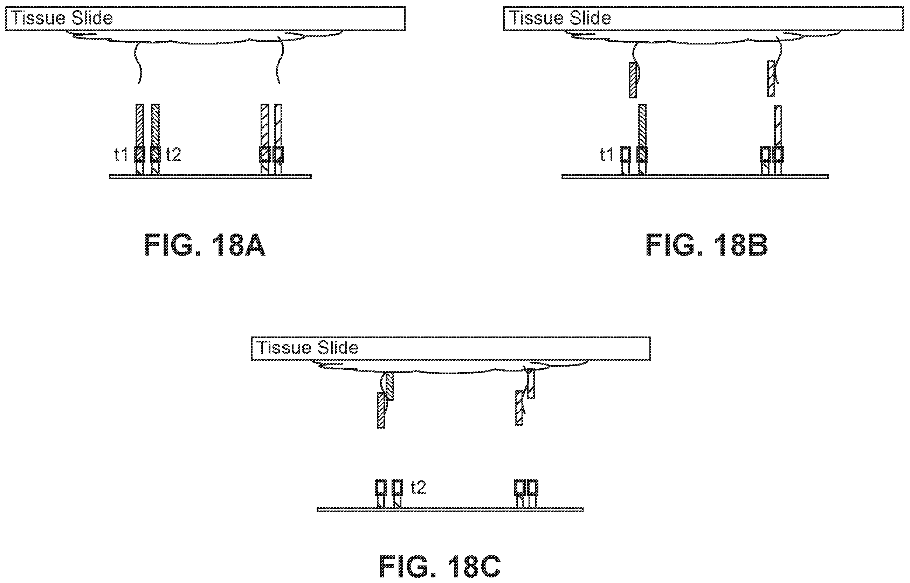

In some examples, the array includes a first plurality of capture probes and a second plurality of capture probes. In some examples, the first plurality of capture probes is released (e.g., cleaved) at a first time point (t1) and interact with the first capture probe binding domain. In some examples, the second plurality of capture probes is released at a second time point (t2) and interact with the second capture probe binding domain **(****FIGs. 18A-18C****).**

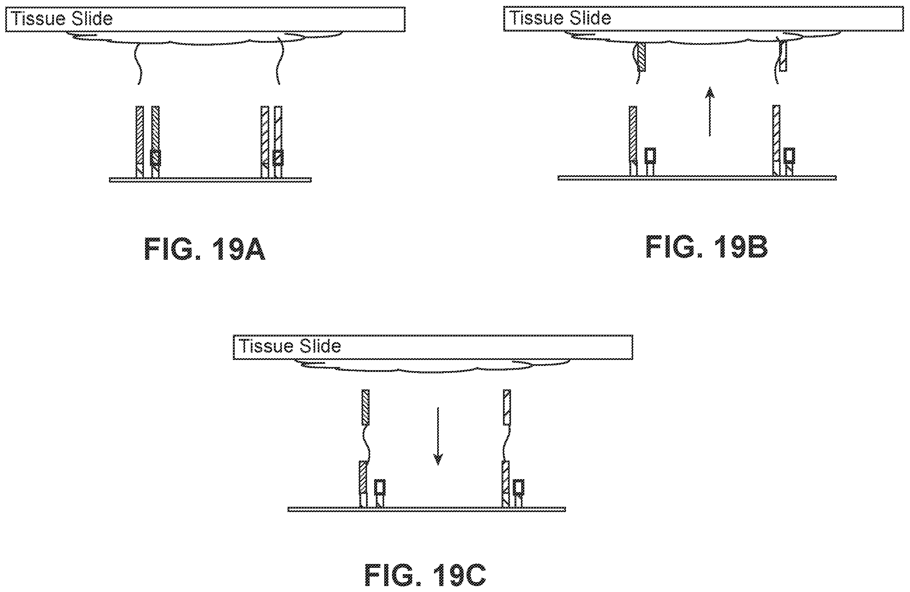

In some examples, the first plurality of capture probes is attached to the substrate and the second plurality of capture probes are released from the substrate to hybridize to the ligation products via the second capture probe binding domain. The biological sample is then permeabilized, allowing the ligation products including the second capture probe to migrate towards the array and bind to the first plurality of capture probes. In some examples, the first capture domain of the first capture probe hybridizes to the target nucleic acid. In such examples, the capture probes in the first plurality of capture probes do not include a cleavable linker. In some examples, the first plurality of capture probes is released from the substrate and the second plurality of capture probes remain immobilized on the substrate (e.g., without a cleavable linker) **(****FIGs. 19A-19C****).**

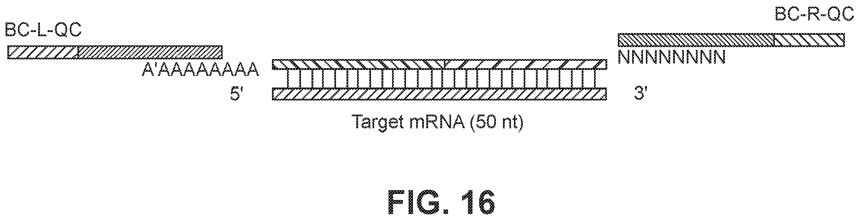

**FIG. 16** shows an exemplary embodiment where the capture probes include a quality control (QC) nucleic acid sequence. The quality control sequence can be used to identify released capture probes from one feature that appear in sequencing data from a nearby and/or adjacent sequence.

### Example 2 - Multiple Barcoding of a Nucleic Acid Analyte

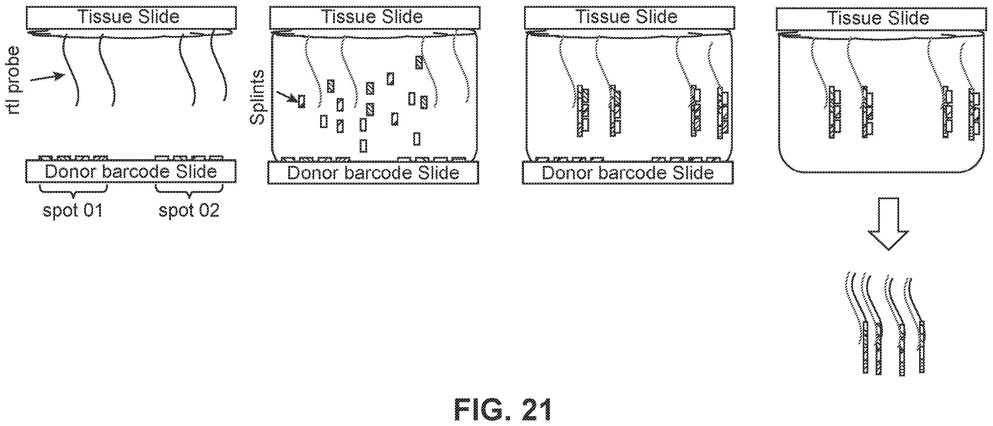

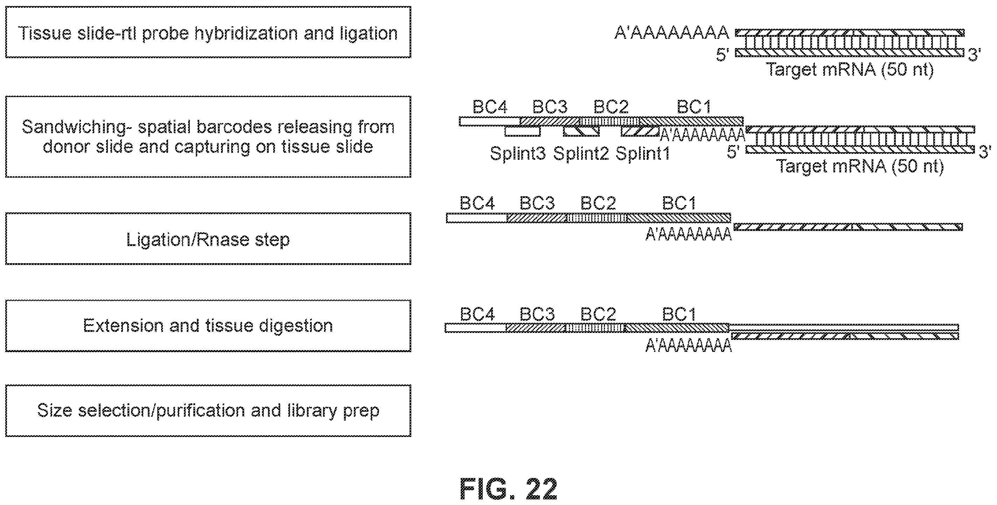

**FIG. 22** is an exemplary workflow of the methods depicted in **FIG. 21****.**

A set of probes (e.g., probe oligonucleotides) is contacted with a biological sample, e.g., a biological sample mounted on a substrate, where the probes hybridize to target nucleic acids (e.g., mRNA molecules) in the biological sample. In some examples, the probes hybridize to adjacent sequences in the target nucleic acid. In some examples, the probes hybridize to non-adjacent sequences on the target nucleic acid and a gap-filling reaction is performed to extend the first and/or second probe. The first probe and second probe are ligated to one another while hybridized to the target nucleic acid and after ligation, an endonuclease such as an RNase (e.g., any of the RNases described herein) degrades the target nucleic acid hybridized to the probes (e.g., ligation product), leaving a single-stranded DNA molecule (e.g., the ligation product).

A donor barcode slide is provided comprising pluralities of capture probes. While **FIG. 21** shows four pluralities of capture probes on each feature (e.g., spot), a feature on the spatially barcoded substrate (e.g., donor barcode slide) can include two, three, four, five, six, seven, eight or more pluralities of capture probes.

After ligation of the probe oligonucleotides in the biological sample, the pluralities of capture probes having, in some instances, a capture domain, a UMI, a spatial barcode, and a primer, are released (e.g., cleaved) from the donor barcode slide with the addition of a first reagent medium. In some examples, when the tissue slide is aligned with the donor barcode slide comprising an array of capture probes such that at least a portion of the biological sample is aligned with at least a portion of the array, the biological sample (or portion thereof) and the array are contacted with a first reagent medium. In some examples, the plurality of capture probes is released from the donor barcode slide during the contacting with the first reagent medium. Then, the released (e.g., cleaved) capture probes are free to migrate to the biological sample. In some examples, the capture probes are released sequentially (e.g., the first plurality of capture probes is released, followed by the second plurality of capture probes, followed by the third plurality of capture probes, followed by the fourth plurality of capture probes, etc.). In some examples, the pluralities of capture probes are released simultaneously.

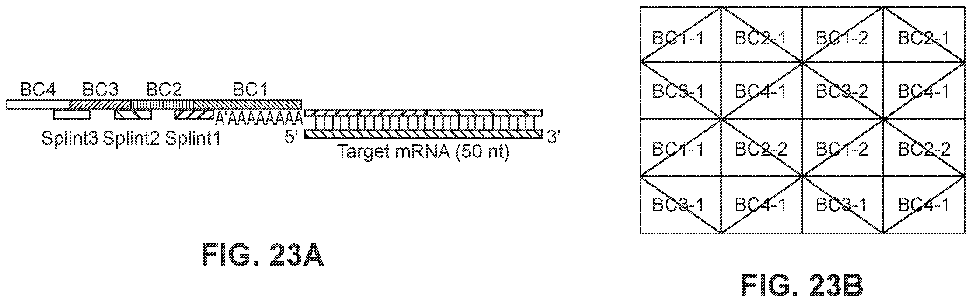

A first plurality of splint oligonucleotides is provided in the first reagent medium. The released pluralities of capture probes are coupled (e.g., ligated) with each other with the aid of the splint oligonucleotides. In some examples, the splint oligonucleotides comprise the same nucleic acid sequence. In some examples, the splint oligonucleotides comprise different sequences (e.g., a second plurality of splint oligonucleotides, a third plurality of splint oligonucleotides, a fourth plurality of splint oligonucleotides, etc.). In some examples, the capture domain (e.g., poly(T) sequence) of the first capture probe hybridizes to the ligation product in the biological sample via a first capture probe binding domain (e.g., a poly(A) sequence). The pluralities of capture probes and splint oligonucleotides hybridize one another as shown in **FIG. 23A****,** thereby spatially barcoding the nucleic acid. The sequences of the spatial barcodes or complements thereof and the sequence of the ligation product, or complement, thereof can be used to determine the location of the target nucleic acid in the biological sample. **FIG. 23B** shows an exemplary spatial barcoding pattern where each feature includes a single plurality of capture probes. As the pluralities of capture probes are released, they will diffuse in various directions and the combination of spatial barcodes associated with a target nucleic acid can be identified with a location on the substrate and thus the biological sample.

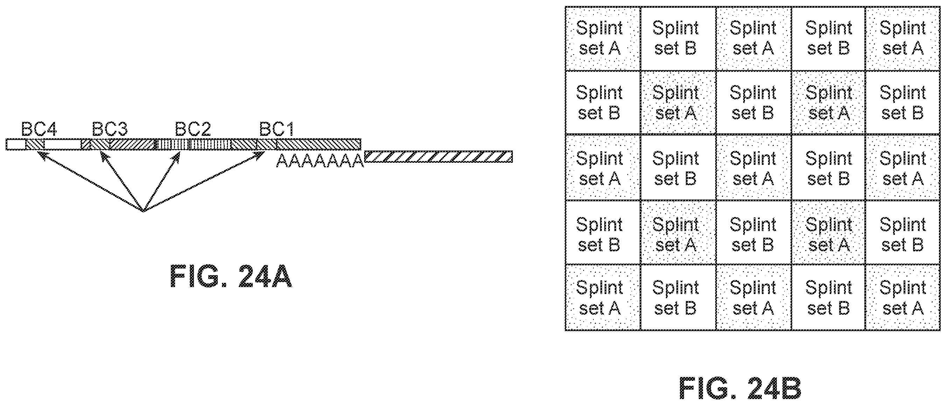

**FIG. 24A** shows an exemplary embodiment of a spatially barcoded product where the arrows indicate UMIs associated with each of the pluralities of capture probes. **FIG. 24B** shows an exemplary embodiment where different pluralities of splint oligonucleotides are used in different features to prevent mis-localization. For example, a first plurality of splint oligonucleotides (e.g., Splint set A) and a second plurality of splint oligonucleotides (e.g., Splint set B) can be delivered to different features such that only the capture probes associated with those respective features hybridize to the appropriate splint oligonucleotide.

In some examples, the biological sample is contacted with a second reagent medium, where the second reagent medium includes an RNase (e.g., any of the RNases described herein) which degrades the RNA hybridized to the DNA, thus leaving a singled-stranded DNA molecule (e.g., the ligation product) hybridized to at least the first capture probe.

In some examples, the second reagent medium can permeabilize the biological sample, thus releasing the coupled capture probe/ligation product into the second reagent medium for collection and bulk processing. Such bulk processing can include, e.g., an extension step, generating a copy of the ligation product having components of the capture probes (e.g., primer, spatial barcode, capture domain) and the ligation product. The extended product can be collected for further evaluation (e.g., sequencing).

### Example 3 - Methods for Spatial Transcriptomics Analysis in a Region of Interest

In a non-limiting example, FFPE biological tissue sections on standard slides are deparaffinized, H&E stained, and imaged. Next, the tissue samples are hematoxylin-destained. The sections are decrosslinked by incubating at 70°C for 1 hour in TE pH 9.0. TE is removed and the tissues are incubated in 1x PBS-Tween for 15 minutes.

The biological sample is permeabilized using a solution comprising e.g., pepsin or proteinase K. A non-sandwiching method can be used as a control. Following permeabilization, the capture probes interact with the ligation products in the biological sample. The ligation products are treated with RNase H to release/digest the RNA hybridized to the ligation product. An extension reaction is run, sequencing libraries are prepared and sequenced, and the results are analyzed computationally.

**FIGs. 25A-C**show an exemplary workflow of applying a mask to reduce or prevent release of capture probes outside a region of interest.

**FIG. 25A** is a schematic diagram showing an exemplary embodiment where a substrate with barcoded capture probes (e.g., any of the barcoded capture probes described herein) that include a cleavable linker (e.g., any of the photocleavable linkers described herein) and a second substrate containing a biological sample (e.g., tissue) in a sandwiching process. In some examples, the barcode is a spatial barcode as defined herein. In some examples, the capture probe includes a unique molecular identifier, one or more functional domains, and a capture domain.

**FIG. 25A** also shows a mask applied to the substrate with the barcoded capture probes. The mask is a protective covering or coating that is selectively applied or removed once region(s) of interest (ROIs) are identified within a biological sample. ROIs can be identified as described herein including through microscopy techniques and/or staining techniques to identify specific regions within the biological sample based on morphological features, cell types, disease phenotypes, etc. **FIG. 25A** shows areas of the substrate where the mask protects the substrate with the barcoded capture probes and areas where the mask has been selectively applied (or removed) from the substrate with barcoded capture probes.

**FIG. 25B** is a schematic diagram showing the substrate with the barcoded capture probes, mask, and ROIs shown in **FIG. 25A** and exposed to a light source. Exposure to light results in the selective release of the barcoded capture probes via the photocleavable linker where the mask has not been applied (or has been removed). The mask is applied to the substrate with the barcoded capture probes to areas corresponding to ROI(s) that have been identified in the biological sample (e.g., identified by any of the methods described herein). **FIG. 25B** also shows barcoded capture probes remain immobilized on the substrate in areas that remain protected by the mask.

**FIG. 25C** is a schematic diagram showing the selectively released barcoded capture probes from **FIG. 25B** hybridizing with target analytes from the biological sample. While **FIG. 25C** shows released barcoded capture probes hybridizing with an analyte, it is contemplated that such selectively released capture probes can hybridize with an analyte derived molecule, such as a ligation product or analyte capture sequence of an analyte capture agent.

The captured analytes, or analyte derived molecules, can be further processed (e.g., amplified and sequenced) to determine the location of the analyte in the biological sample.

## Claims

1. A method of spatially barcoding a target nucleic acid in a biological sample, the method comprising:
(a) providing an array comprising a first plurality of capture probes, wherein a first capture probe of the first plurality of capture probes comprises: (i) a first spatial barcode and (ii) a first capture domain, and wherein the first capture probe is attached to a substrate, and
a second plurality of capture probes, wherein a second capture probe of the second plurality of capture probes comprises: (iii) a second spatial barcode and (iv) a second capture domain, and wherein the second capture probe is attached to the substrate;
(b) hybridizing a first probe oligonucleotide and a second probe oligonucleotide to the target nucleic acid, wherein the first probe oligonucleotide and the second probe oligonucleotide each comprise a sequence that is substantially complementary to a first sequence and a second sequence of the target nucleic acid, respectively, and wherein the first probe oligonucleotide comprises a first capture probe binding domain and the second probe oligonucleotide comprises a second capture probe binding domain;
(c) coupling the first probe oligonucleotide and the second probe oligonucleotide, thereby generating a ligation product;
(d) releasing the first capture probe and/or the second capture probe from the substrate; and
(e) hybridizing (i) the first capture domain of the first capture probe to the first capture probe binding domain of the ligation product, and optionally, (ii) the second capture domain of the second capture probe to the second capture probe binding domain of the ligation product, thereby spatially barcoding the target nucleic acid in the biological sample.

2. The method of claim 1, wherein the first probe oligonucleotide and the second probe oligonucleotide hybridize to adjacent or non-adjacent sequences on the target nucleic acid.

3. The method of claim 2, wherein a gap-filling reaction extends the first probe oligonucleotide or second probe oligonucleotide.

4. The method of any one of claims 1-3, wherein the coupling of the first probe oligonucleotide and the second probe oligonucleotide comprises ligating the first probe oligonucleotide and the second probe oligonucleotide via a ligase, optionally further comprising releasing the ligation product from the target nucleic acid using a nuclease, optionally wherein the nuclease comprises an RNase, optionally, wherein the RNase is selected from RNase A, RNase C, RNase H, or RNase I.

5. The method of any one of claims 1-4, wherein the first capture probe binding domain comprises a first sequence complementary to at least a portion of the first capture domain of the first capture probe, and the second capture probe binding domain comprises a second sequence complementary to at least a portion of the second capture domain of the second capture probe, optionally wherein the first sequence complementary to at least a portion of the first capture domain of the first capture probe comprises a poly(A) sequence, optionally wherein the second sequence complementary to at least a portion of the second capture domain of the second capture probe comprises a fixed sequence.

6. The method of any one of claims 1-5, wherein the first capture probe hybridized to the first capture probe binding domain of the ligation product is extended with a polymerase, thereby generating a complement of the ligation product, optionally wherein the complement of the ligation product is ligated to the second capture probe, optionally wherein the ligation comprises use of a ligase.

7. The method of any one of claims 1-6, further comprising determining (i) all or a part of the sequence of the ligation product, or a complement thereof, and (ii) the sequence of the first spatial barcode, or a complement thereof and, optionally, the sequence of the second spatial barcode, or a complement thereof, optionally wherein the determining comprises sequencing, optionally wherein the method further comprises using the combination of the determined sequence of the first spatial barcode, or a complement thereof, and, optionally, the determined sequence of the second spatial barcode, or a complement thereof, to determine a location of the target nucleic acid in the biological sample.

8. The method of any one of claims 1-7, wherein the first capture probe, and optionally, the second capture probe further comprise a quality control (QC) sequence.

9. The method of any one of claims 1-8, wherein releasing the first capture probe and/or the second capture probe comprises cleaving the first capture probe, and optionally, the second capture probe from the substrate, optionally wherein the first capture probe comprises a first cleavable linker and the second capture probe comprises a second cleavable linker, optionally wherein the first cleavable linker and the second cleavable linker comprise a restriction enzyme site or a photocleavable linker, optionally wherein the first cleavable linker and the second cleavable linker are different or the same, optionally wherein the first cleavable linker and the second cleavable linker are cleaved at the same time or wherein the first cleavable linker is cleaved prior to the second cleavable linker being cleaved, and wherein the first capture domain hybridizes to the first capture probe binding domain of the ligation product at a first time point and the second capture domain hybridizes to the second capture probe binding domain of the ligation product at a second time point.

10. The method of claim 9, the method further comprising:
i) cleaving the first cleavable linker;
ii) permeabilizing the biological sample;
iii) hybridizing the first capture domain of the first capture probe to the first capture probe binding domain of the ligation product, thereby generating a first capture probe/ligation product complex; and
iv) hybridizing the first capture probe/ligation product complex to the second capture probe on the array via the second capture probe binding domain.

11. The method of any one of claims 1-10, wherein the target nucleic acid is DNA or RNA, , optionally wherein the RNA is mRNA.

12. The method of any one of claims 1-11, wherein the array comprises a plurality of wells, wherein a well of the plurality of wells comprises the first capture probe and the second capture probe.

13. The method of any one of claims 1-12, wherein the biological sample is disposed on the array or wherein the biological sample is disposed on a second substrate, optionally further comprising aligning the second substrate with the array, such that at least a portion of the biological sample is aligned with at least a portion of the array.

14. A method of determining a location of a target nucleic acid in a biological sample, the method comprising:
(a) providing an array comprising a first plurality of capture probes, wherein a first capture probe of the first plurality of capture probes comprises: (i) a first spatial barcode and (ii) a first capture domain, and wherein the first capture probe is attached to a substrate, and
a second plurality of capture probes, wherein a second capture probe of the second plurality of capture probes comprises: (iii) a second spatial barcode and (iv) a second capture domain, and wherein the second capture probe is attached to the substrate ;
(b) hybridizing a first probe oligonucleotide and a second probe oligonucleotide to the target nucleic acid, wherein the first probe oligonucleotide and the second probe oligonucleotide each comprise a sequence that is substantially complementary to a first sequence and a second sequence of the target nucleic acid, respectively, and wherein the first probe oligonucleotide comprises a first capture probe binding domain and the second probe oligonucleotide comprises a second capture probe binding domain;
(c) coupling the first probe oligonucleotide and the second probe oligonucleotide, thereby generating a ligation product;
(d) releasing the first capture probe and/or the second capture probe from the array;
(e) hybridizing the first capture probe to the first capture probe binding domain of the ligation product and hybridizing the second capture probe to the second capture probe binding domain of the ligation product, thereby generating a spatially barcoded product; and
(f) determining (i) all or a portion of the sequence of the ligation product of the spatially barcoded product, or a complement thereof, (ii) the sequence of the first spatial barcode, or a complement thereof, and optionally, (iii) the sequence of the second spatial barcode, or a complement thereof, and using the determined sequences of (i), (ii), and optionally, (iii) to determine the location of the target nucleic acid in the biological sample.

15. The method of any one of claims 1-14, wherein the first capture probe is attached to the substrate by its 5' end or its 3' end and wherein the second capture probe is attached to the substrate by its 5' end or its 3' end.

16. The method of any one of claims 1-15, wherein the biological sample is a tissue section, optionally wherein the tissue section is a fresh-frozen tissue section, optionally wherein the tissue section is a fixed tissue sample, optionally wherein the fixed tissue sample is a formalin-fixed paraffin-embedded (FFPE) tissue section, optionally wherein the FFPE tissue section is deparaffinized and decrosslinked prior to or after mounting on the substrate or second substrate.

## Patentansprüche

1. Verfahren zur räumlichen Barcodierung einer Zielnukleinsäure in einer biologischen Probe, wobei das Verfahren umfasst:
(a) Bereitstellen eines Arrays, umfassend eine erste Vielzahl von Erfassungssonden, wobei eine erste Erfassungssonde der Vielzahl von Erfassungssonden Folgendes umfasst: (i) einen ersten räumlichen Barcode und (ii) eine erste Erfassungsdomäne, und wobei die erste Erfassungssonde an ein Substrat gebunden ist, und
eine zweite Vielzahl von Erfassungssonden, wobei eine zweite Erfassungssonde der zweiten Vielzahl von Erfassungssonden Folgendes umfasst: (iii) einen zweiten räumlichen Barcode und (iv)eine zweite Erfassungsdomäne, und wobei die zweite Erfassungssonde an das Substrat gebunden ist;
(b) Hybridisieren eines ersten Sondenoligonukleotids und eines zweiten Sondenoligonukleotids an die Zielnukleinsäure, wobei das erste Sondenoligonukleotid und das zweite Sondenoligonukleotid jeweils eine Sequenz umfassen, die im Wesentlichen komplementär zu einer ersten Sequenz bzw. einer zweiten Sequenz der Zielnukleinsäure ist, und wobei das erste Sondenoligonukleotid eine erste Erfassungssonden-Bindungsdomäne umfasst und das zweite Sondenoligonukleotid eine zweite Erfassungssonden-Bindungsdomäne umfasst;
(c) Koppeln des ersten Sondenoligonukleotids und des zweiten Sondenoligonukleotids, wodurch ein Ligationsprodukt erzeugt wird;
(d) Freisetzen der ersten Erfassungssonde und/oder der zweiten Erfassungssonde von dem Substrat; und
(e) Hybridisieren (i) der ersten Erfassungsdomäne der ersten Erfassungssonde mit der ersten Erfassungssonden-Bindungsdomäne des Ligationsprodukts und optional (ii) der zweiten Erfassungsdomäne der zweiten Erfassungssonde mit der zweiten Erfassungssonden-Bindungsdomäne des Ligationsprodukts, wodurch die Zielnukleinsäure in der biologischen Probe mit einer räumlichen Barcodierung versehen wird.

2. Verfahren nach Anspruch 1, wobei das erste Sondenoligonukleotid und das zweite Sondenoligonukleotid an benachbarte oder nicht benachbarte Sequenzen auf der Ziel-Nukleinsäure hybridisieren.

3. Verfahren nach Anspruch 2, wobei eine Lückenfüllreaktion das erste Sondenoligonukleotid oder das zweite Sondenoligonukleotid erweitert.

4. Verfahren nach einem der Ansprüche 1 bis 3, wobei die Kopplung des ersten Sondenoligonukleotids und des zweiten Sondenoligonukleotids das Ligieren des ersten Sondenoligonukleotids und des zweiten Sondenoligonukleotids über eine Ligase umfasst, optional ferner umfassend das Freisetzen des Ligationsprodukts von der Zielnukleinsäure unter Verwendung einer Nuklease, wobei die Nuklease optional eine RNase umfasst, wobei die RNase optional ausgewählt ist aus RNase A, RNase C, RNase H oder RNase I.

5. Verfahren nach einem der Ansprüche 1 bis 4, wobei die erste Erfassungssonden-Bindungsdomäne eine erste Sequenz umfasst, die zu mindestens einem Teil der ersten Erfassungsdomäne der ersten Erfassungssonde komplementär ist, und die zweite Erfassungssonden-Bindungsdomäne eine zweite Sequenz umfasst, die zu mindestens einem Teil der zweiten Erfassungsdomäne der zweiten Erfassungssonde komplementär ist, wobei die erste Sequenz, die zu mindestens einem Teil der ersten Erfassungsdomäne der ersten Erfassungssonde komplementär ist, optional eine Poly(A)-Sequenz umfasst, wobei die zweite Sequenz, die zu mindestens einem Teil der zweiten Erfassungsdomäne der zweiten Erfassungssonde komplementär ist, optional eine fixierte Sequenz umfasst.

6. Verfahren nach einem der Ansprüche 1 bis 5, wobei die erste Erfassungssonde, die mit der ersten Erfassungssonden-Bindungsdomäne des Ligationsprodukts hybridisiert ist, mit einer Polymerase erweitert wird, wodurch ein Komplement des Ligationsprodukts erzeugt wird, wobei das Komplement des Ligationsprodukts optional mit der zweiten Erfassungssonde ligiert wird, wobei die Ligation optional die Verwendung einer Ligase umfasst.

7. Verfahren nach einem der Ansprüche 1 bis 6, ferner umfassend das Bestimmen (i) der gesamten oder eines Teils der Sequenz des Ligationsprodukts oder eines Komplements davon und (ii) der Sequenz des ersten räumlichen Barcodes oder eines Komplements davon und optional der Sequenz des zweiten räumlichen Barcodes oder eines Komplements davon, wobei das Bestimmen optional das Sequenzieren umfasst, wobei das Verfahren optional ferner die Verwendung der Kombination der bestimmten Sequenz des ersten räumlichen Barcodes oder eines Komplements davon umfasst, und optional der bestimmten Sequenz des zweiten räumlichen Barcodes oder eines Komplements davon, um eine Position der Zielnukleinsäure in der biologischen Probe zu bestimmen.

8. Verfahren nach einem der Ansprüche 1 bis 7, wobei die erste Erfassungssonde und optional die zweite Erfassungssonde ferner eine Sequenz zur Qualitätskontrolle (QC) umfasst.

9. Verfahren nach einem der Ansprüche 1-8, wobei das Freisetzen der ersten Erfassungssonde und/oder der zweiten Erfassungssonde das Spalten der ersten Erfassungssonde umfasst, und optional der zweiten Erfassungssonde von dem Substrat, wobei die erste Erfassungssonde optional einen ersten spaltbaren Linker umfasst und die zweite Erfassungssonde optional einen zweiten spaltbaren Linker umfasst, wobei der erste spaltbare Linker und der zweite spaltbare Linker optional eine Restriktionsenzymstelle oder einen photospaltbaren Linker umfassen, wobei der erste spaltbare Linker und der zweite spaltbare Linker optional verschieden oder gleich sind, wobei der erste spaltbare Linker und der zweite spaltbare Linker optional gleichzeitig gespalten werden oder wobei der erste spaltbare Linker gespalten wird, bevor der zweite spaltbare Linker gespalten wird, und wobei die erste Erfassungsdomäne mit der ersten Erfassungssonden-Bindungsdomäne des Ligationsprodukts zu einem ersten Zeitpunkt hybridisiert und die zweite Erfassungsdomäne mit der zweiten Erfassungssonden-Bindungsdomäne des Ligationsprodukts zu einem zweiten Zeitpunkt hybridisiert.

10. Verfahren nach Anspruch 9, das Verfahren ferner umfassend:
i) Spalten des ersten spaltbaren Linkers;
ii) Permeabilisieren der biologischen Probe;
iii) Hybridisieren der ersten Erfassungsdomäne der ersten Erfassungssonde mit der ersten Erfassungssonden-Bindungsdomäne des Ligationsprodukts, wodurch ein erster Erfassungssonden-/Ligationsproduktkomplex erzeugt wird; und
iv) Hybridisieren des ersten Erfassungssonden-/ Ligationsproduktkomplexes mit der zweiten Erfassungssonde auf dem Array über die zweite Erfassungssonden-Bindungsdomäne.

11. Verfahren nach einem der Ansprüche 1-10, wobei die Zielnukleinsäure DNA oder RNA ist, wobei die RNA optional mRNA ist.

12. Verfahren nach einem der Ansprüche 1 bis 11, wobei das Array eine Vielzahl von Wells umfasst, wobei ein Well der Vielzahl von Wells die erste Erfassungssonde und die zweite Erfassungssonde umfasst.

13. Verfahren nach einem der Ansprüche 1 bis 12, wobei die biologische Probe auf dem Array angeordnet ist oder wobei die biologische Probe auf einem zweiten Substrat angeordnet ist, optional ferner umfassend das Ausrichten des zweiten Substrats auf das Array, sodass mindestens ein Teil der biologischen Probe auf mindestens einen Teil des Arrays ausgerichtet ist.

14. Verfahren zur Bestimmung eines Orts einer Zielnukleinsäure in einer biologischen Probe, wobei das Verfahren umfasst:
(a) Bereitstellen eines ersten Substrats, umfassend eine erste Vielzahl von Erfassungssonden, wobei eine erste Erfassungssonde der ersten Vielzahl von Erfassungssonden (i) einen ersten räumlichen Barcode und (ii) eine erste Erfassungsdomäne umfasst, und wobei die erste Erfassungssonde an ein Substrat gebunden ist, und
eine zweite Vielzahl von Erfassungssonden, wobei eine zweite Erfassungssonde der zweiten Vielzahl von Erfassungssonden (iii) einen zweiten räumlichen Barcode und (iv) eine zweite Erfassungsdomäne umfasst, und wobei die zweite Erfassungssonde an das Substrat gebunden ist;
(b) Hybridisieren eines ersten Sondenoligonukleotids und eines zweiten Sondenoligonukleotids an die Zielnukleinsäure, wobei das erste Sondenoligonukleotid und das zweite Sondenoligonukleotid jeweils eine Sequenz umfassen, die im Wesentlichen komplementär zu einer ersten Sequenz bzw. einer zweiten Sequenz der Zielnukleinsäure ist, und wobei das erste Sondenoligonukleotid eine erste Erfassungssonden-Bindungsdomäne umfasst und das zweite Sondenoligonukleotid eine zweite Erfassungssonden-Bindungsdomäne umfasst;
(c) Koppeln des ersten Sondenoligonukleotids und des zweiten Sondenoligonukleotids, wodurch ein Ligationsprodukt erzeugt wird;
(d) Freisetzen der ersten Erfassungssonde und/oder der zweiten Erfassungssonde von dem Array;
(e) Hybridisieren der ersten Erfassungssonde mit der ersten Erfassungssonden-Bindungsdomäne des Ligationsprodukts und Hybridisieren der zweiten Erfassungssonde mit der zweiten Erfassungssonden-Bindungsdomäne des Ligationsprodukts, wodurch ein Produkt mit einem räumlichen Barcode erzeugt wird; und
(f) Bestimmen (i) der gesamten oder eines Teils der Sequenz des Ligationsprodukts des Produkts mit einem räumlichen Barcode oder eines Komplements davon, (ii) der Sequenz des ersten räumlichen Barcodes oder eines Komplements davon und optional (iii) der Sequenz des zweiten räumlichen Barcodes oder eines Komplements davon, und Verwenden der bestimmten Sequenzen aus (i), (ii) und optional (iii) zum Bestimmen des Orts der Zielnukleinsäure in der biologischen Probe.

15. Verfahren nach einem der Ansprüche 1 bis 14, wobei die erste Erfassungssonde mit ihrem 5'-Ende oder ihrem 3'-Ende an das Substrat gebunden ist, und wobei die zweite Erfassungssonde mit ihrem 5'-Ende oder ihrem 3'-Ende an das Substrat gebunden ist.

16. Verfahren nach einem der Ansprüche 1 bis 15, wobei die biologische Probe ein Gewebeschnitt ist, wobei der Gewebeschnitt optional ein frisch gefrorener Gewebeschnitt ist, wobei der Gewebeschnitt optional eine fixierte Gewebeprobe ist, wobei die fixierte Gewebeprobe optional ein Formalin-fixierter Paraffin-eingebetteter (FFPE) Gewebeschnitt ist, wobei der FFPE-Gewebeschnitt optional vor oder nach dem Anbringen auf dem Substrat entparaffiniert und entnetzt wird.

## Revendications

1. Procédé de codage à barres spatialement d'un acide nucléique cible dans un échantillon biologique, le procédé comprenant :
(a) la fourniture d'un réseau comprenant une première pluralité de sondes de capture, dans lequel une première sonde de capture de la pluralité de premières sondes de capture comprend : (i) un premier code à barres spatial et (ii) un premier domaine de capture , et dans lequel la première sonde de capture est fixée à un substrat, et
une deuxième pluralité de sondes de capture, dans lequel une deuxième sonde de capture de la deuxième pluralité de sondes de capture comprend : (iii) un deuxième code à barres spatial et (iv) un deuxième domaine de capture, et dans lequel la deuxième sonde de capture est fixée au substrat ;
(b) l'hybridation d'un premier oligonucléotide sonde et d'un deuxième oligonucléotide sonde à l'acide nucléique cible, dans lequel le premier oligonucléotide sonde et le deuxième oligonucléotide sonde comprennent chacun une séquence qui est sensiblement complémentaire d'une première séquence et d'une deuxième séquence de l'acide nucléique cible, respectivement, et dans lequel le premier oligonucléotide sonde comprend un premier domaine de liaison de sonde de capture et le deuxième oligonucléotide sonde comprend un deuxième domaine de liaison de sonde de capture ;
(c) le couplage du premier oligonucléotide sonde et du deuxième oligonucléotide sonde, générant ainsi un produit de ligature ;
(d) la libération de la première sonde de capture et/ou de la deuxième sonde de capture du substrat ; et
(e) l'hybridation (i) du premier domaine de capture de la première sonde de capture au premier domaine de liaison de sonde de capture du produit de ligature, et facultativement, (ii) du deuxième domaine de capture de la deuxième sonde de capture au deuxième domaine de liaison de sonde de capture du produit de ligature, codant ainsi à barres spatialement l'acide nucléique cible dans l'échantillon biologique.

2. Procédé selon la revendication 1, dans lequel le premier oligonucléotide sonde et le deuxième oligonucléotide sonde s'hybrident à des séquences adjacentes ou non-adjacentes sur l'acide nucléique cible.

3. Procédé selon la revendication 2, dans lequel une réaction de remplissage d'espace prolonge le premier oligonucléotide sonde ou le deuxième oligonucléotide sonde.

4. Procédé selon l'une quelconque des revendications 1 à 3, dans lequel le couplage du premier oligonucléotide sonde et du deuxième oligonucléotide sonde comprend la ligature du premier oligonucléotide sonde et du deuxième oligonucléotide sonde par l'intermédiaire d'une ligase, comprenant en outre facultativement la libération du produit de ligature à partir de l'acide nucléique cible en utilisant une nucléase, facultativement dans lequel la nucléase comprend une RNase, facultativement, dans lequel la RNase est choisie parmi RNase A, RNase C, RNase H ou RNase I.

5. Procédé selon l'une quelconque des revendications 1 à 4, dans lequel le premier domaine de liaison de sonde de capture comprend une première séquence complémentaire d'au moins une portion du premier domaine de capture de la première sonde de capture, et le deuxième domaine de liaison de sonde de capture comprend une deuxième séquence complémentaire d'au moins une portion du deuxième domaine de capture de la deuxième sonde de capture, facultativement dans lequel la première séquence complémentaire d'au moins une portion du premier domaine de capture de la première sonde de capture comprend une séquence poly(A), facultativement dans lequel la deuxième séquence complémentaire d'au moins une portion du deuxième domaine de capture de la deuxième sonde de capture comprend une séquence fixe.

6. Procédé selon l'une quelconque des revendications 1 à 5, dans lequel la première sonde de capture hybridée au premier domaine de liaison de sonde de capture du produit de ligature est étendue avec une polymérase, générant ainsi un complément du produit de ligature, facultativement dans lequel le complément du produit de ligature est ligaturé à la deuxième sonde de capture, facultativement dans lequel la ligature comprend l'utilisation d'une ligase.

7. Procédé selon l'une quelconque des revendications 1 à 6, comprenant en outre la détermination (i) de la totalité ou d'une partie de la séquence du produit de ligature, ou d'un complément de celui-ci, et (ii) de la séquence du premier code à barres spatial, ou d'un complément de celui-ci et, facultativement, de la séquence du deuxième code-barres spatial, ou d'un complément de celui-ci, facultativement dans lequel la détermination le séquençage, facultativement dans lequel le procédé comprend en outre l'utilisation de la combinaison de la séquence déterminée du premier code à barres spatial, ou d'un complément de celui-ci, et, facultativement, de la séquence déterminée du deuxième code à barres spatial, ou d'un complément de celui-ci, pour déterminer un emplacement de l'acide nucléique cible dans l'échantillon biologique.

8. Procédé selon l'une quelconque des revendications 1 à 7, dans lequel la première sonde de capture, et facultativement, la deuxième sonde de capture comprennent en outre une séquence de contrôle qualité (QC).

9. Procédé selon l'une quelconque des revendications 1 à 8, dans lequel la libération de la première sonde de capture et/ou de la deuxième sonde de capture comprend le clivage de la première sonde de capture, et facultativement, de la deuxième sonde de capture à partir du substrat, facultativement dans lequel la première sonde de capture comprend un premier lieur clivable et la deuxième sonde de capture comprend un deuxième lieur clivable, facultativement dans lequel le premier lieur clivable et le deuxième lieur clivable comprennent un site d'enzyme de restriction ou un lieur photoclivable, facultativement dans lequel le premier lieur clivable et le deuxième lieur clivable sont différents ou identiques, facultativement dans lequel le premier lieur clivable et le deuxième lieur clivable sont clivés en même temps ou dans lequel le premier lieur clivable est clivé avant que le deuxième lieur clivable soit clivé et dans lequel le premier domaine de capture s'hybride au premier domaine de liaison de sonde de capture du produit de ligature clivable à un premier point temporel et le deuxième domaine de capture s'hybride au deuxième domaine de liaison de sonde de capture du produit de ligature à un deuxième point temporel.

10. Procédé selon la revendication 9, le procédé comprenant en outre :
i) le clivage du premier lieur clivable ;
ii) la perméabilisation de l'échantillon biologique ;
iii) l'hybridation du premier domaine de capture de la première sonde de capture au premier domaine de liaison de sonde de capture du produit de ligature, générant ainsi un complexe première sonde de capture/produit de ligature ; et
iv) l'hybridation du complexe première sonde de capture/produit de ligature à la deuxième sonde de capture sur le réseau par l'intermédiaire du deuxième domaine de liaison de sonde de capture.

11. Procédé selon l'une quelconque des revendications 1 à 10, dans lequel l'acide nucléique cible est un ADN ou ARN, facultativement dans lequel l'ARN est un ARNm.

12. Procédé selon l'une quelconque des revendications 1 à 11, dans lequel le réseau comprend une pluralité de puits, dans lequel un puits de la pluralité de puits comprend la première sonde de capture et la deuxième sonde de capture.

13. Procédé selon l'une quelconque des revendications 1 à 12, dans lequel l'échantillon biologique est disposé sur le réseau ou dans lequel l'échantillon biologique est disposé sur un deuxième substrat, comprenant en outre facultativement l'alignement du deuxième substrat avec le réseau, de sorte qu'au moins une portion de l'échantillon biologique est alignée avec au moins une portion du réseau.

14. Procédé de détermination d'un emplacement d'un acide nucléique cible dans un échantillon biologique, le procédé comprenant :
(a) la fourniture d'un réseau comprenant une première pluralité de sondes de capture, dans lequel une première sonde de capture de la pluralité de premières sondes de capture comprend : (i) un premier code à barres spatial et (ii) un premier domaine de capture , et dans lequel la première sonde de capture est fixée à un substrat, et
une deuxième pluralité de sondes de capture, dans lequel une deuxième sonde de capture de la deuxième pluralité de sondes de capture comprend : (iii) un deuxième code à barres spatial et (iv) un deuxième domaine de capture, et dans lequel la deuxième sonde de capture est fixée au substrat ;
(b) l'hybridation d'un premier oligonucléotide sonde et d'un deuxième oligonucléotide sonde à l'acide nucléique cible, dans lequel le premier oligonucléotide sonde et le deuxième oligonucléotide sonde comprennent chacun une séquence qui est sensiblement complémentaire d'une première séquence et d'une deuxième séquence de l'acide nucléique cible, respectivement, et dans lequel le premier oligonucléotide sonde comprend un premier domaine de liaison de sonde de capture et le deuxième oligonucléotide sonde comprend un deuxième domaine de liaison de sonde de capture ;
(c) le couplage du premier oligonucléotide sonde et du deuxième oligonucléotide sonde, générant ainsi un produit de ligature ;
(d) la libération de la première sonde de capture et/ou de la deuxième sonde de capture du réseau ;
(e) l'hybridation de la première sonde de capture au premier domaine de liaison de sonde de capture du produit de ligature et l'hybridation de la deuxième sonde de capture au deuxième domaine de liaison de sonde de capture du produit de ligature, générant ainsi un produit codé spatialement par code à barres ; et
(f) la détermination (i) de la totalité ou d'une portion de la séquence du produit de ligature du produit codé spatialement par code à barres, ou d'un complément de celui-ci, (ii) de la séquence du premier code à barres spatial, ou d'un complément de celui-ci, et facultativement, (iii) de la séquence du deuxième code à barres spatial, ou d'un complément de celui-ci, et l'utilisation des séquences déterminées de (i), (ii), et facultativement, (iii) pour déterminer l'emplacement de l'acide nucléique cible dans l'échantillon biologique.

15. Procédé selon l'une quelconque des revendications 1 à 14, dans lequel la première sonde de capture est fixée au substrat par son extrémité 5' ou son extrémité 3' et dans lequel la deuxième sonde de capture est fixée au substrat par son extrémité 5' ou son extrémité 3'.

16. Procédé selon l'une quelconque des revendications 1 à 15, dans lequel l'échantillon biologique est une section de tissu, facultativement dans lequel la section de tissu est une section de tissu congelé frais, facultativement dans lequel la section de tissu est un échantillon de tissu fixé, facultativement dans lequel l'échantillon de tissu fixé est une section de tissu fixé au formol et inclus dans la paraffine (FFPE), facultativement dans lequel la section de tissu FFPE est déparaffinée et déréticulée avant ou après le montage sur le substrat ou le deuxième substrat.
